# EUROPEAN PATENT SPECIFICATION

(11) **EP 1 789 580 B1**
(45) Date of publication and mention of the grant of the patent: **12.03.2014**
(21) Application number: 05757406.3
(22) Date of filing: 04.07.2005
(51) Int. Cl.: C12Q 1/68

(54) **GENETIC ANALYSIS IN RACING ANIMALS**
GENETISCHE ANALYSE IN BEI RENNEN EINGESETZTEN TIEREN
ANALYSE GÉNÉTIQUE D'ANIMAUX DE COURSE

(30) Priority: 02.07.2004 GB 0414965; 25.08.2004 GB 0418986
(43) Date of publication of application: 30.05.2007
(73) Proprietor: Thoroughbred Genetics Ltd, Kent CT4 7DT (GB)
(72) Inventor: HARRISON, Stephen Paul, Thoroughbred Genetics Ltd, Kent ME9 8AX (GB)
(86) International application number: PCT/GB2005/002623
(87) International publication number: WO 2006/003436

(56) References cited:
- WO-A-02/092851
- WO-A-03/046203
- XU XIUFENG ET AL: "The complete mitochondrial DNA sequence of the horse, Equus caballus: Extensive heteroplasmy of the control region" GENE (AMSTERDAM), vol. 148, no. 2, 1994, pages 357-362, XP002348743 ISSN: 0378-1119 cited in the application -& DATABASE EMBL [Online] 22 November 1994 (1994-11-22), "Equus caballus mitochondrial DNA complete sequence." XP002348887 retrieved from EBI accession no. EM_PRO:MIECCOMP Database accession no. X79547
- RUPERT JIM L: "The search for genotypes that underlie human performance phenotypes." COMPARATIVE BIOCHEMISTRY AND PHYSIOLOGY. PART A, MOLECULAR & INTEGRATIVE PHYSIOLOGY. SEP 2003, vol. 136, no. 1, September 2003 (2003-09), pages 191-203, XP002348837 ISSN: 1095-6433 cited in the application
- HILL E W ET AL: "History and integrity of thoroughbred dam lines revealed in equine mtDNA variation" ANIMAL GENETICS, vol. 33, no. 4, August 2002 (2002-08), pages 287-294, XP002348836 ISSN: 0268-9146 cited in the application
- PARK H B ET AL: "Molecular characterization and mutational screening of the PRKAG3 gene in the horse." CYTOGENETIC AND GENOME RESEARCH, vol. 102, no. 1-4, 2003, pages 211-216, XP008053914 ISSN: 1424-8581

## Description

### Field of the Invention

The present invention relates to processes for determining and predicting racing ability and aptitude in animals through the study of nucleotide sequence variants, genetic types or profiles and in particular the study of mitochondrial haplotype. Also provided are nucleotide sequence variants and their use in genetic typing analysis and classification. The invention is especially useful in breeding and training of thoroughbred horses for example in selecting performers for purchase or breeding, the study and design of breeding lines and determining preferred race distance and age of optimum racing ability. The invention is also particularly useful in the gambling industry as a valuable additional tool in the settling of suitable odds and classification of performers and their preferred/optimum racing distances. Also provided are kits for use in or with said analyses and methods.

### Background to the invention

Horse racing is a multimillion pound industry and a sport enjoyed by millions of followers around the world. The 'Classic' races such as the Derby and Oaks generate huge amounts of public interest and income for the industry every year. However the breeding and training of successful racing thoroughbreds remains a very expensive and high-risk business and methods for improved prediction of racing ability and improved breeding and training methods applicable to racing are much needed.

A particular problem is that biologically and historically, thoroughbred racehorses have not lent themselves to conventional genetic studies relating racing ability, that is actual performance on the track, to genotype. The assessment of performance genotype ratios in their progeny is difficult. They are mono ovulating animals with an average gestation period of 11 months, which restricts mares to the production of one foal per year. Furthermore, in order to assess performance phenotypes it is necessary to wait until horses have attained at least the minimum racing age of 2 years old.

No attention has been given to associations between true racing performance (as directly related to actual racing potential, ability and aptitude) and genetic factors. The application of new genetic advances and DNA technologies in this industry has been very limited and centred on veterinary areas. WO 03/046203 [Equigene] refers to a 'shot gun' approach to identify genetic markers for individual traits in horses using partial sequencing of the genome, polymorphism identification and whole-genome linkage and analysis, and dismisses candidate gene approaches. Nevertheless, no named genes, relevant to performance are identified.

The potential multifactorial nature of the inheritance of racing ability is another, significant problem for the study of simple Mendelian mechanisms, as are other variable factors that impact performance such as environmental and commercial factors. There is no formal or established method of anatomical or pre-competitive performance assessment for thoroughbreds as there is for other sporting horse breeds such as showjumpers. Nor are there any precise genetic criteria applied in the selection of these horses for breeding. Information about genetic inheritance of performance traits and potential breeding value is based primarily on unquantified and frequently anecdotal anatomical observations about the progeny of stallions and mares or, naturally, on the racecourse performances of their descendants over a number of generations.

Another complicating feature for this industry is that there are strict rules against conception of thoroughbreds by any other means than natural cover which means that artificial insemination is banned, as is embryo transfer. Similarly there would be no acceptance of animals produced by DNA manipulation or cloning in racing, even if such an approach was practicable given that it is likely that a multitude of genes are involved. Even with cheaper and more readily available agricultural or laboratory animals such studies remain highly problematic.

The pedigree records kept for the thoroughbred are unparalleled in detail in comparison with any other breed or domestic species with the current population being derived from three males. In theory all thoroughbreds can be traced back to three original males lines. The population is genetically finite and a restricted stud book has been employed since 1791 (Weatherby and Sons, 1791). Consequently, assessment of pedigree data has served as the basis for a simple form of genetic quantification over the years. This has frequently been informally applied through associations made between the occurrence of particular ancestors and the inheritance of performance traits and perceived anatomical characteristics in their descendants. Numerous pedigree theories have been evolved to explain these relationships and some are more scientifically viable than others.

A recurrent theme relates to the hierarchical breeding and racing value attached to different maternal lines found in the Thoroughbred breed. In breeding and pedigree terminology these are also referred to as families, dam lines or tail-female lines. Efforts were made to categorise maternal lines in order of importance defined by the quality of horses descended from them. Lowe (1898) and Bobinski (1958) traced the origin of the maternal families to the founder mares of the stud book. Today, their family comparisons are considered less relevant and their main role is in providing a convenient form of pedigree classification. However, recent female family performance history, incorporating female line relatives within 3-4 generations, is still used as a subjective means of assessing the value of horses for racing and breeding.

Origins of the domestic horse have been studied though the mtDNA (Vila et al, 2001; Jansen et al, 2002). Also, mtDNA can be used to study the diversity and variation of female lines in horse breeds like Lipizzan (Kavar et al, 1999), Arabian (Bowling et al, 2000) or Cheju (Yang et al, 2002). However, none of these works have studied any relationship between genetic type and performance.

Studies of mtDNA variation in thoroughbreds have shown variation within the breed but these studies were restricted by relatively small samples and by examinations restricted to D-loop SSCP and sequence variation. These studies only used a hyper variable fragment of the control region of the mtDNA (Ishida et al, 1994; Marklund et al, 1995; Hill et al., 2002). Classification studies of mitochondrial phylogenetics based on the mitochondrial control region (d loop) can miss variation located in other parts of the mitochondria (Parsons and Clobe, 2001) and make phylogenetic studies quite complicated (Ingman, 2000). In addition, methods using sequencing as a sole main tool can give rise to incorrect identification of genotypes (Foster, 2003). Thus, experiments, which are based on d loop also increase the number of erroneous sequences recorded.

In humans, variation in 7 mitochondrial genes has been implicated in influencing fitness and performance characteristics (Dionne et al., 1993; Perusse et al., 2001). Of these, two are constituents of the cytochrome oxidase complex IV, one is cytochrome b and four are members of the NADH dehydrogenase complex I. Specifically, variants of genes coding for NADH hydrogenase subunits ND2 and ND4 affect initial fitness and responses to training. However equine genes do not always have an equivalent role as their human counterpart and similar phenotypic expression may not be controlled by exactly the same genes. For example, there is variation between the groups in the multiallelic systems that control blood groupings.

Another reason why the study of horse genetics might be different and (possibly) more complex is that thoroughbreds, in particular, have been deliberately selected for racing ability in many instances. This means that, unlike in humans, genetic biases will have been introduced and it may be difficult to assign importance to certain genes because preferential variants may have already been 'fixed' in this preselected population.

While useful commercial testing services for certain thoroughbred characteristics (for example, inbreeding analysis and prepotency analysis) have recently become available (Wood, 2002; Hickman 2003) the scientific and technical basis for these has not been published.

According to the present invention it has now been determined that functional mitochondrial gene sequence variation in the current thoroughbred population exists and that it is possible to investigate associations, between individual gene variants and combined gene variation, with racing ability or performance. In particular, it has been shown that variation between horses in the protein-encoding mitochondrially-transmitted genes is associated with and affects racing aptitudes and racing ability characteristics. Furthermore, from analysis of enzyme-encoding genes, rRNA-encoding genes and D-loop, it has now been shown that mitochondrial haplotypes identified from an analysis of 1,000 horses vary significantly in their effects on racecourse performance capabilities and stamina optima. The invention therefore provides an important link between mitochondrial gene variation and racing performance variability.

The present invention is the first reported to employ specific examination of candidate genes for the study of genetic associations with racing ability and aptitudes as outlined by racecourse success. Although thoroughbred horses are selected for racing ability, there have not been any previous reported associations between genotypes and racecourse performance nor any reliable predictive techniques.

In thoroughbred breeding, importance is traditionally attached to the variable racing endurance and racing performance abilities of different female breeding lines. From the invention it is now possible to show that this is due, at least in part, to the maternal mode of inheritance, and variation in their mitochondrial DNA (mtDNA) constitution.

### Summary of the Invention

Accordingly, in a first aspect, the present invention provides a process for determining or predicting sporting performance, ability or aptitude of horses, comprising analysis of the presence, distribution or expression of one or more nucleotide sequence variants having an association with sporting performance, ability or performance aptitude, said variants comprising mitochondrial nucleotide sequence variants and/or chromosomal nucleotide sequence variants which are able to complement said mitochondrial nucleotide sequence variants.

Animal athletes or performance animals according to the invention include sporting breeds such as horses, dogs and birds. Sporting horses include thoroughbred and non-thoroughbred horses, Arab endurance horses, quarter horses and standardbreds. Non equids include greyhounds, racing pigeons, hawks, owls and camels.

The invention is particularly useful to the sport of racing, particularly horse racing. Uses include gambling, training, breeding, veterinary or nutritional fields. Preferably the performance aptitude is for speed, stamina, optimum racing distance or precocity of age for racing. Preferably the performance horses are selected from thoroughbred, standardbreds and trotting horses.

As used herein, 'racing ability/aptitude/performance' refers to athletic qualities such as speed and stamina, but does not include disease attributes.

In a further aspect the present invention provides a nucleotide sequence variant for use in the process of the invention. Suitably the variant comprises a nucleotide sequence selected from
a) the sequences listed in Example 1
b) gene specific nucleotides based on the Example 1 sequences,
c) complimentary strands thereof or nucleotide sequences which hybridise under stringent conditions to Example 1 sequences,
d) degenerate nucleotide sequences of any of a) b) or c)
e) fragments of any of a) - d) above.

As used herein 'gene-specific nucleotides' is intended to include nucleotides unique to the relevant gene, such as fragments of the gene that are unique to said gene. As used herein 'hybridisation' relates to sequences that hybridise to said nucleotides. Conditions which affect hybridisation include temperature and salt concentration. In the present context, stringent hybridisation conditions refers to temperatures of greater than 30 degrees centigrade, typically greater than 40 and usually about 55 degrees. Salt conditions are typically 1M, suitably less than 0.5 M. The skilled person would appreciate that the consideration of the combinations of salt and temperature conditions are more important than either condition on its own.

In a further aspect the present invention provides a protein encoded by any of the variants of the invention or antibodies thereto.

In a further aspect the present invention provides a set of variant nucleotide sequences comprising one or more dloop mitochondrial gene sequences, plus one or more respiratory enzyme gene sequences and optionally one or more rRNA gene sequences for use in haplotype analysis or classification in animals.

Preferably said mitochondrial nucleotide sequence variants are selected from respiratory enzyme and rRNA nucleotide sequences. Preferably the respiratory enzyme sequences are selected from NADH dehydrogenase 2, NADH dehydrogenase 4, NADH dehydrogenase 4L, NADH dehydrogenase 5, Cytochrome Oxidase I, Cytochrome Oxidase II, ATPase 6, and Cytochrome B. Preferably the rRNA sequences are selected from rRNA 16S-1, rRNA 16S-2 and rRNA 12S.

Preferably the variants comprise NADH dehydrogenase 2, NADH dehydrogenase 4, NADH dehydrogenase 4L, NADH dehydrogenase 5, Cytochrome Oxidase I, Cytochrome Oxidase II, ATPase 6, Cytochrome B, rRNA 16S-1, rRNA 16S-2, rRNA 12S and dloop variants.

In a further aspect the present invention provides a set of variants according to the invention, which represent a haplotype for use in a process of the invention. In a preferred set the respiratory enzyme sequences are selected from NADH dehydrogenase 2, NADH dehydrogenase 4, NADH dehydrogenase 4L, NADH dehydrogenase 5, cytochrome oxidase I, cytochrome oxidase II, ATPase 6, and cytochrome B. Other preferred sets include rRNA sequences which are selected from rRNA 16S-1, rRNA 16S-2 and rRNA 12S.

A further preferred set comprises NADH dehydrogenase 2, NADH dehydrogenase 4, NADH dehydrogenase 4L, NADH dehydrogenase 5, cytochrome oxidase I, cytochrome oxidase II, ATPase 6, cytochrome B, rRNA 16S-1, rRNA 16S-2, rRNA 12S and dloop variants.
According to the present invention there are now defined new indicator haplotypes, 17 preferred haplotypes are shown as types 1- XVII listed in Table 1.

In a further aspect of the invention there is provided a kit for use in the process of the invention comprising one or more nucleotide sequences, or a protein or antibody according to the invention and supporting material and optionally probes, labels or instructions for use. Kits suitable for home or field testing are particularly useful.

Preferably the kit comprises a microarray. As used herein, a 'microarray' comprises sets of chemical reaction areas where different probes are provided on a suitable carrier such as a microchip, slide , bead or the like, to test nucleotide or other fragments.

In a further aspect the present invention provides a process of classification, identification, sampling or description of genetic types of performance animals using analysis of nucleotide sequence variants as defined in any preceding claim, optionally with pedigree data or a combination thereof.

The present invention includes sequences between the primers as defined below, the primers, primer sites within the amplified regions and sequences between the start and the stop of the gene. Also provided for are cDNA, mRNA, polypeptide and antisense sequences, which can be extrapolated from the sequences detailed in Example 1 below.

In a further aspect the present invention provides a kit for use in a mitochondrial type analysis comprising a DNA sequence or a set of sequences as defined above on a suitable medium. The present invention further provides a method and a kit for determination of haplotype based on polymorphism in mitochondrial DNA which is simple to use, yet precise and suitable for use under 'home'/field test conditions. Kits suitable for multiple/bulk testing are also provided.

Preferably the sequence(s) is attached to a support material. Suitably the support material is nylons, nitrocellulose, plastic film or slides. Suitably the kit is a microarray. Suitable methods are described in Sambrook et al., 1989; Schaefer, 1997; Andrade, 2003; Dunham, 2003.

In a further aspect the present invention provides a process for identifying variants for use in a process of the invention comprising, selecting candidate nucleotide sequences for comparison, amplifying whole or partial nucleotide sequences using one or more primers if required , analysing samples for candidate sequence variants and correlation of variant types and performance or performance qualities to identify variants with performance implications.

In a further aspect the present invention provides a process for identifying or detecting in a sample, one or more variants of the invention, comprising amplifying nucleic acid sequence or parts of sequence in the sample using one or more primers if required, followed by testing with or using a suitable analytical technique. Useful analytical techniques include SSCP or SSCP plus nucleotide sequencing, or hybridisation to one or more probes relevant to said variant or variants. In a further aspect the present invention provides primers for use in processes according to the invention.

In a further aspect the present invention provides the use or analysis of one or more variants of the invention, or one or more variants identified according to the invention or one or more haplotypes according to the invention in the horse racing business.

Particular uses of the invention include the organisation or reorganisation of stud books or pedigree databases or in the verification of pedigree, providing typing systems for equine maternally-inherited extra-chromosomal DNA, the study of horse physiology, the study of complementation between respiratory complex genes carried on the chromosomes and those carried on mtDNA and other genetic interactions so as to identify positive outcomes resulting in or contributing to, superior energy release or other characteristics which affect sporting performance or ability.

Importantly, it has now been found that by analysis of mitochondrial typing it is possible to rank subjects in relation to racing ability and aptitude criteria. For the first time, the present invention permits a classification of the population of thoroughbreds for prediction of breeding, training and race suitability and for detailed forensic studies of thoroughbred lines and pedigree. Moreover, for particular races, contestants can be ranked based on suitable age and/or distance by analysis of genetic profile or on a deduced profile based on pedigree and profiles of known family lines.

In a further aspect the present invention provides the use for ranking the genetic profile of a subject, group, or predicting the relative outcome of racing involving several subjects, comprising measuring or estimating the haplotype and optionally measuring or estimating the presence, distribution or differential expression of one or more nucleotide sequence variants, and applying the resulting analysis so as to rank the subjects according to preferred racing category or distance.

According to the invention, an index can be calculated for use in the process of the invention, for example using the percentage of winners of the relevant type related to the frequency of that type in the relevant population. Such an analytical and predictive approach has not hitherto been possible in any of the above mentioned racing spheres, whether breeding, training, betting or other relevant applications.

In a further aspect the present invention provides the use of the invention for determining suitable odds for gambling optionally taking into account information on other race specific factors. Usefully the information can be used to prepare a database, literature or a test system. Such databases are a further aspect of the invention and their use in consultancy or advice businesses. Clearly the invention has application to all types of racing systems, UK, non-UK and amateur as well as professional /top class racing. Non UK racing systems include systems such as that in the USA, Canada, Ireland, Australia, New Zealand, Japan, Singapore, Hong Kong, South Africa, South American Countries China, Dubai, Saudi Arabia, Kenya, Norway, Sweden, Denmark, Germany and France.

The present invention is very useful in the betting industry. For example the information provided can supplement traditional information (going, recent form etc.) in assisting settling of odds for any particular race. Furthermore more accurate information can be assembled on horses appearing during any one season. Similarly, private and commercial breeders and owners can make considerable use of the invention in breeding and training schedules, regimes, strategies and customisation.

In many animal species, members of the same respiratory enzyme gene complex appear both on mitochondria and the chromosomes. The present invention provides a practical means of testing and measuring for complementation or other interactions between mitochondrial and chromosomal variants of the same or different complexes. Other studies, which are possible with the present invention, include the study of heteroplasmy and incompatibility of co-existing d loops in the same individual, which may explain a number of phenomena such as surprisingly good or bad mating outcomes. For example, it can be shown that horses carrying two forms of mtDNA, as determined by functional gene differences, are likely to be subject to variable phenotype expression based on the gene differences present. This may adversely affect racing/breeding ability. The present invention also allows the identification of horses, which are heteroplasmic in terms of their mtDNA status, and the possible effects and implications of this phenomena on performance.

The information provided by the present invention is particularly useful in correlation of the information gathered with physiology or phenotypes. The present invention also allows analysis of relationships between variants and muscle type determination (fast/slow twitch). Determination of performance also includes stamina prediction and absolute race winning or losing ability, family trends etc.

One useful aspect of the present invention is the use of pedigree theories or databases that determine sire/dam mating based on matching of mitochondria DNA types. The invention also allows the use of the information system to identify physiological attributes. The system of the invention is particularly useful in conjunction with other systems or pedigree information. A large number of new products are possible according to the results of the present invention. A wide number of technical applications of this type would be possible and appreciated by the ordinary skilled person and are also encompassed within the present invention.

The invention is particularly useful because it improves the welfare of horses. For example by identifying animal types which are susceptible to breakdown or other failures in training and so allow planning of 'appropriate course of training. The present invention also reduces the risk of a horse with a genetic predisposition to poor stamina or *vice versa* from being trained in areas for which is it unsuitable.The present invention can also be applied to bodybuilding and other applications of dietary manipulation so as to affect, for example muscle mass, the effect of high lysine foods and muscle mass versus age.

### Detailed description of the invention

It is possible to carry out the method of the invention using any suitable sample from the subject. Suitably the sample is blood, but it can be any tissue sample such as a nasal swab, cheek cell sample, semen, urine, hair cell, lymph fluid, serum etc. If samples are not available but details of pedigree and results from related animals are known or can be obtained then it is still possible to determine an animal's performance genotype.

Blood samples can be obtained fresh or taken as samples, which are mixed with EDTA anticoagulant. EDTA tubes (obtainable from Greiner Ltd.) are suitable. ACD or lithium chloride tubes can also be used. Samples taken on solid matrices, for instance FTA paper (Whatman Ltd.) is also suitable. Blood samples are prepared by cell lysis. For example, red cell lysis using freeze/thaw and addition of water, white blood cell lysis is with Proteinase K and SDS. DNA deproteinisation (ammonium persulphate) is followed by DNA precipitation using ethanol. Suitable DNA from blood preparation kits are Perfect gDNA (Eppendorf) and Nucleon (Amersham).

Suitably the assessment of mitochondrial type is determined using SSCP and/or sequence analysis. Other suitable methods include SNPs (Single Nucleotide Polymorphisms), RFLPs (Restriction Fragment Length Polymorphisms), AFLPs (Amplified Fragment Length Polymorphisms), VNTRs (Variable Number Tandoem Repeats), and TRS (Tandem repeats) . Others will be readily apparent to the skilled person. A combination of methods can be used. Automated methods (such as real-time PCR and use of micro-arrays) are particularly convenient. Details of these, and other suitable methods can be found in Sambrook et al., 1989; Schaefer, 1997; Andrade, 2003; Dunham, 2003.

Variants, haplotypes and their sequences are detailed in Example 1 below. Haplotype composition is illustrated in Table 1. The present invention provides and identifies 17 new variants, useful in haplotype analysis. Examples of correlation between haplotype and performance are given in Examples 2-5.

The present invention has the potential for application in a number of formats. These may include: use of simple lab-based and conventional DNA polymorphism tests such as measurements of Single Nucleotide Polymorphisms (SNP), Restriction Fragment Length Polymorphisms (RFLP), Variable Number Tandem Repeats (VNTR), Amplified Fragment Length Polymorphisms (AFLP), Tandem Repeats (TR) and other polymorphism variation; Automated tests involving use of DNA sequencers or genotypers, real-time PCR machines or thermocyclers to detect polymorphisms as previously described; use of tagged primers, oligonucleotide and other biochemical molecules; use of combinations of the previously cited application methods; Denaturing High Performance Liquid Chromatography (DHPLC) based systems, Chromatography based systems; use of fixed matrix detection systems based on anchored nucleotides, polynucleotides or polypeptides, such as micro-array systems or chip-based systems; use of non-fixed or anchored detection systems; use of immunological detection kits; use of polypeptide, nucleotide, polynucleotide or enzymatic detection kits; use of colourimetrically based kits; incorporation into database products providing information relating to breeding, training, veterinary or gambling aspects of horse racing. Details of these, and other suitable methods can be found in Sambrook et al., 1989; Schaefer, 1997; Andrade, 2003; Dunham, 2003.

In particular, kits might be considered in two forms: Those that identify gene variants carried by a horse or a genetic profile; and those that measure the levels of performance-affecting enzymes, polypeptides, polynucleotide or hormones in body tissues or fluids. Other suitable alternatives will be apparent to the skilled person.

As an example, the detection of the gene products covered in this invention in body tissues can be achieved in two ways. One way involves tests based on a colourimetric change resulting from presence in body tissue and brought about by conversion of one biochemical into another with a subsequent release of, for example, amino acids which can be detected *in vitro.* The other method is to use tests based on immunological reaction of antibodies to the presence of allelic variants of gene products in body tissue.

### APPLICATIONS

From an examination of the DNA of a bank of 1,000 horses, the existence of variants of 11 different genes or loci with performance implications has been identified enabling thoroughbred horses to be assigned one of 17 different 'types' based on their combination of variants. Using pedigree data, it is possible to extrapolate this information to allow the winners of all UK Group races of the last 50-100 years to be grouped into the various genetic types.

The results are striking. Some genetic types are more effective in sporting terms than others and in particular, their success is related to stamina capabilities. There are clear differences in the success of the various types in different races. The system has also been applied in an examination of the Raceform ratings of over 3,000 horses and these observations are clearly supported.

Not only will the ability to predict inheritance and possession of these components of stamina allow breeders to co-ordinate breeding more efficiently but it will also enable owners and trainers to purchase horses and target training programmes and race entries more effectively. Importantly, the ability to assign genetic types to potential runners in Group races offers a unique and exclusive aid to betting and laying, particularly in ante-post markets. The information generated by this invention is extremely useful in the breeding, gambling and training aspects of the racing business.

In essence, at the production and racing level, the Thoroughbred population can be practically subdivided into three sub groups: breeding stock; young, unraced, stock and horses in training. Generally, the people dealing with each sub-group differ and specialisation occurs. Similarly, the types of organisation or processes involved in managing these sub-groups are different. With regard to products and information arising from this invention, each one of these groups will have varying requirements and there will be different reasons for their application. In essence there are three markets within a market.

*Breeding stock:* Owners, of both mares and stallions, would benefit from information which can tell them about the variants of important performance-related genes carried by these horses and whether choices of stallion/mare matings based on haplotype is appropriate.

*Young, unraced, stock:* Owners or potential purchasers of these animals will require information which will help them to decide whether to buy or sell unraced animals, i.e. whether an animal has received the desired genetic variants which will enhance its likelihood of success. Breeders may also wish to know whether a particular breeding strategy has resulted in a foal receiving desirable gene versions from its parents which are co-ordinated to achieving success over specific race distances.

*Horses in training:* The principal people involved with this group are owners and trainers. They would be primarily interested in using products which give an indication of a horse's genetic performance profile e.g. tests or database/pedigree information which indicate potential stamina range. Unlike the other groups, they would also be interested in biochemical testing kits that can measure the levels of hormones or enzymes or polypeptides shown to affect performance and fitness that are present in a horse at any one time.

Therefore there is potential application of products resulting from this invention in test, kit or database format at all levels of Thoroughbred management and production and in all establishments, i.e. stud farms, training facilities, sales facilities, veterinary centres etc. Some details of uses follow.

### Use in breeding or breeding related applications

In the breeding sphere, it is commonplace for breeders to employ pedigree advice and software to determine their mating strategies for their mares. It is possible to use the data generated according to the invention to provide a confirmatory test of the genetic variants carried by horses and/or to provide information which will help direct breeding policies more accurately to achieve enhance racing success. It is possible to modify the tests for inclusion into automated sequencer/genotyper, real time PCR, micro-array or chip based system based on SNP or other DNA polymorphism variation within the mitochondrial genes.

This also makes possible the concept of an interactive 'suite' of tests to provide a complete overview of a horse's genetic profile with respect to racing ability and stamina potential. Ultimately, this test is likely to be used in conjunction with other tests. It is feasible to envisage the development of microarray/chip-based systems for all of these components that can be applied quickly using an eventual portable system at, for instance, horse sales. For example, a complementary test might include a microarray-based test for identification of chromosomally determined proteins of the mitochondrial respiratory complexes.

There is also potential to combine the mitochondrial data into a database system (literature or IT based) that more accurately reflects the correct genetic pedigrees and racing ability/aptitude of horses. A common feature of breeding systems is to base mating plans on the female family line origins. This system can be used to provide more accurate pedigree and information to breeders.

One particularly useful aspect of the present invention is the use of pedigree theories or databases that determine sire/dam mating based on matching of mitochondrial DNA types as defined by variation dictated by the protein encoding genes. Incorporation of information derived from this invention into database (literature or IT based) for aiding in the purchase of horses at horse sales is an option.

### Use in training or training related applications

The demonstration that mtDNA types, as defined by variation dictated by the protein encoding genes, affect a horse's stamina or racing performance capabilities make this invention useful for trainers of racehorses because it will allow them to target the training regimes of horses to their genetic potential. Similarly, it may also aid trainers involved in the purchase of horses for clients in their decision-making processes. Muscle composition is affected by training (Hodgson et al., 1986). In future, it may be possible to link the haplotype and variant variability with particular physiological conditions or processes, which may need particular handling with regard to training approaches. Similarly, it is possible that lab based tests, such as measurement of heart rates or VO2 Max, etc., employing mechanical apparatus (e.g. treadmills etc.), may be correlated with the possession of mtDNA haplotype or gene variants for the assessment of physical and racing/breeding capabilities.

Modes of application of this information would be similar to that detailed for the breeding aspects above. In particular, database products detailing the expected racing performance of genetic types or biochemical and biological tests measuring the levels or variants of gene products present in horses at any one time would be valuable.

The invention is also particularly useful because it improves the welfare of animals and horses in training. For example by identifying types which are susceptible to breakdown or other failures in training and also reduces the risk of a horse with a genetic predisposition to distance races from being trained in areas for which is it unsuitable.

### Use in veterinary and animal husbandry areas

Various veterinary, lab-based, tests are popular. For instance, around 50% of Thoroughbred horses in training are regularly tested for white blood cell counts to estimate 'immunological' health for racing.

In database, kit and test form the information provided by the current invention offers many veterinary uses. The ability to predict or determine horses which carry specific variants or mtDNA haplotypes may help to select horse which are more physically capable of surviving specific training regimes. A very important use will be where horses varying in their mtDNA can be shown to respond differently to certain drugs, hormones or manual treatment. Identification of these predispositions through test or database will enhance the effects of these processes or treatments. Planning of reproductive or rehabilitation processes following injury or trauma may also be aided by programme targeting through haplotype definition.

Similarly, horses of particular haplotype may respond differently to specific feeding or nutritional regimes. It is likely that they will vary with regard to such features as live weight gain or increases in muscle mass. For example the present invention can be applied to body building and other applications of dietary manipulation so as to affect, for example muscle mass, the effect of high lysine foods and muscle mass versus age. Other uses include use studying the interation of diet, such as responses to the use of probiotics, neutraceuticals and other compounds.

As in the case with training applications, it is also possible that lab based tests, such as measurement of heart rates or VO2 Max, etc., employing mechanical apparatus (e.g. treadmills etc.), may be correlated with the possession of mtDNA haplotype or gene variants for the assessment of veterinary treatments, processes or use of pharmaceuticals.

### Use in Gambling

The present invention is very useful in gambling and offers a system that is the first of its kind and provides a fresh angle or approach on the betting process. The systems according to the invention have applications to both the laying and placing of bets and offers useful tools for bookmaking organisation in providing useful information when deciding odds. The system of the invention enables the development of databases that can provide pre-race genetic data which can be used as supplementary information for the compiling of odds and laying of bets. The possibility also exists for the provision of database/internet/media products for punters, betting data companies or racing journals and publications. Clearly the invention has application all types of racing systems, UK non-UK and amateur as well as professional /top class racing.

The invention is further illustrated by the accompanying drawing in which:

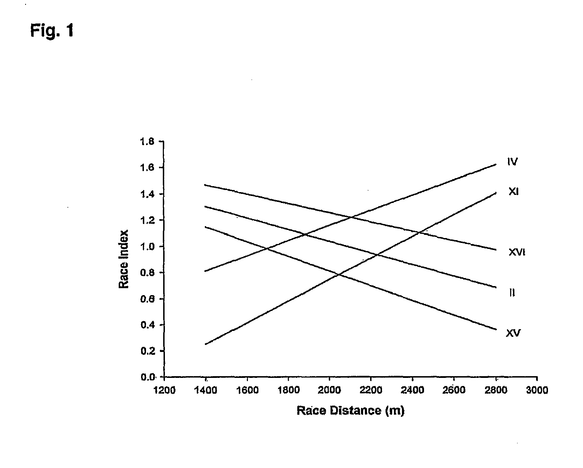

Fig. 1 shows regression lines describing the relationship of RI vs race distance for 5 haplotypes. Each regression is significant (II, P<0.05; IV, P<0.001; XI, P<0.05; XV, P<0.05; XVI, P<0.05) but also clearly illustrates the shift in their order of performance merit at different distances.

The invention will now be described in more detail, with reference to and illustrated by the following examples, which are designed to assist a person of ordinary skill in the art in carrying out the invention. The examples are not intended to limit the scope of the invention. Methods are standard methods known in the art unless specifically indicated otherwise.

### EXAMPLES

### General techniques

Unless otherwise indicated the methods required for practising the invention are conventional techniques known to the ordinary skilled person including texts incorporated by reference, see for example, *Sambrook et al.,* 1989 and other references listed herein.

### EXAMPLE 1. MITOCHONDRIAL DNA VARIANT AND HAPLOTYPE ANALYSIS

### Sampline

DNA samples from a random group of 1,000 thoroughbred horses were selected for study. DNA was taken from horse blood using the Perfect gDNA Blood Mini Isolation Kit from Eppendorf. 1000 samples were analysed representing 29 thoroughbred female families from different parts of the world. Reference to the thoroughbred General Stud Book (Weatherby & Sons, 1791) showed that the majority of currently existing European female lines, traceable to original stud book members, were represented within this selection, thereby heightening the chances of achieving coverage of all available mtDNA variation within the breed.

### Primer design

DNA regions from 18 loci were amplified in order to characterise the mtDNA of the thoroughbred horses studied. Groups of Polymerase Chain Reaction (PCR) DNA oligonucleotide primers were designed for each of the protein-encoding genes of the equine mtDNA with reference to a mtDNA genome sequence previously outlined (Xu & Arnason, 1994) for the horse. In addition to these regions, primers were also designed to provide amplification of the 16S and 12S ribosomal DNA genes and the D loop region. Primers were designed to achieve the highest degree of PCR coverage for each gene. Eventually, single primer pairs were selected and used to amplify the most variable gene regions from genomic DNA from all of the horses in our sample.

The primers used with their localisation in the mitochondrial DNA are: Nd1/1 (2772-2791 bp), Nd1/2 (3727-3709 bp); Nd2/1 (3944-3964 bp), Nd2/2 (4936-4916 bp); Nd3/1 (9498-9519 bp), Nd3/2 (9822-9805 bp); Nd4/1 (10345-10364 bp), Nd4/2 (11344-11326 bp); Nd4L/1 (9934-9954 bp), Nd4L/2 (10210-10191 bp); Nd5/1 (12193-12210 bp), Nd5/2 (13192-13174 bp); Nd6/1. (14048-14029 bp), Nd6/2 (13587-13605 bp); CcO1/1 (5402-5419 bp), CcO1/2 (6401-6382 bp); CcO2/1 (7057-7074 bp), CcO2/2 (7724-7705 bp); CcO3/1 (8651-8668 bp), CcO3/2 (9424-9403 bp); ATP6/1 (7973-7991 bp), ATP6/2 (8643-8622 bp); ATP8/1 (7804-7823 bp), ATP8/2 (7996-7977 bp); CytB1 (14196-14213 bp), CytB2 (15306-15289 bp); d1 (15860-15838), d2(15420-15442); dloop2/1 (15826-15846), dloop2/2 (16658-16641); 12S/1 (108-129), 12s/2 (1039-1019); 16S-1/1 (1114-1125), 16S-1/2 (1939-1921); 16S-2/1 (1868-1885), 16S-2/2 (2679-2658) (see SEQ ID NOS: 1 - 75).

From 5' to 3' end the sequences of the primers are:

| | |
|---|---|
| Nd1/1 | TGTTCATAATTAACGTCCTC |
| Nd1/2 | CTATGTTTGTGGTGGGATG |
| | |
| Nd2/1 | CCCTTATCTTCACAACTATTC |
| Nd2/2 | GGGAGGATATAACAATTAACG |
| | |
| Nd3/1 | ATAAACCTCATACTGACACTCC |
| Nd3/2 | TTTGGGTTCATTCGTAGG |
| | |
| Nd4/1 | CAATAGCCTAAACTTCTCAC |
| Nd4/2 | GAATAGCTCTCCAATTAGG |
| | |
| Nd4L/1 | ATATCTTCCTAGCATTCACAG |
| Nd4L/2 | TAGCATTGGAGGAGGTTAAG |
| | |
| Nd5/1 | TTTCCAACTGTTCATCGG |
| Nd5/2 | GTTGGAGATGAAGAATCCG |
| | |
| Nd6/1 | AAACCTTCACCTATTTATGG |
| Nd6/2 | TTAATCTCCACGAGTAACC |
| | |
| CcO1/1 | ACATCGGCACTCTGTACC |
| CcO1/2 | AAGAAGATGAAGCCTAGAGC |
| | |
| CcO2/1 | CCCTTCCAACTAGGATTC |
| CcO2/2 | ATTGATGCAGATCATTCTTC |
| | |
| CcO3/1 | CACCAAACCCACGCTTAC |
| Cc03/2 | TCCTCATCAATAAATAGAGACG |
| | |
| ATP6/1 | AAATCTATTCGCCTCTTTC |
| ATP6/2 | AGGTGTTGTCGTGTAAGTAAAG |
| | |
| ATP8/1 | ATGCCACAGTTGGATACATC |
| ATP8/2 | GTAGCGAAAGAGGCGAATAG |
| | |
| 16S-1/1 | CTAAAGCTAGCCCAAACAATAC |
| 16S-1/2 | GTTTGTGTTTGCCGAGTTC |
| | |
| 16S-2/1 | TGTTAACCCAACACAGGC |
| 16S-2/2 | GGCGGTAGAAGTTATAAATTAG |
| | |
| 12S/1 | AGAATTACACATGCAAGTATCC |
| 12S/2 | CAAGTACACCTTCCGGTATAC |
| | |
| D1 | GCTCCACCATCAACACCCAAAG |
| D2 | TGAAGAAAGAACCAGATGCCAG |
| | |
| D2/1 | AATGAAACTATACCTGGCATC |
| D2/2 | GGGAAGAAGGGTTGACAG |
| | |
| CYT B 1 | CATCCGGAAATCTCACCC |
| CYT B 2 | TTCGATGGTGCTTGCGAG |

### Amplification Methods

The PCR reaction were carried out using 1×buffer (10mM Tris-HCL, pH 8.0, 50mM KCL, 3.75 mM MgCl), 0.2 mM dNTPs, 0.5 pmol/*µ*l of each primer, 0.025 U/*µ*l Taq polymerase and 5 ng/*µ*l DNA template. The PCR thermal cycles were: 1 cycle at 94°C for 1 minute; 30 cycles at 94°C (denaturation) for 30 seconds, 30 seconds (annealing) at 47.5°C for Nd1 or 48.5°C for Nd2 and CcO2 or 50.5°C for 16S-2 and Nd3 or 46.5°C for Nd4 or 49°C for Nd4L and ATP6 or 51°C for Nd5 or 47°C for Nd6 or 50°C for CcO1 or 52°C for D loop 2, 16S-2, 12S and CcO3 and D loop or 55°C for Cytochrome b, 72°C (extension) for 3 minutes for Nd1, Nd2, Nd4, Nd5, CcO1, CytB, D loop2, 12S, 16S-1 and 16S-2 or for 2 minutes and a half for CcO2, Cc03 and ATP6 or for 2 minutes for D loop or for 1 minute and a half for Nd3, Nd4L and ATP8.

### SSCP analysis

To genetically characterise these amplicons, SSCP analysis was carried out by denaturing PCR products through heating at 95°C for 10 mins followed by immediate placement on ice. The PCR products were assessed for polymorphisms by running the denatured DNA on 10 % polyacrylamide, 5 % glycerol, 0.5×TBE gels (Kukita et al, 1997). The gels were run at 0.6 V/h×100bp and stained by silver staining (Caetano-Anolles & Gresshoff, 1994). PCR products shown to represent variants were DNA sequenced.

Representatives of variants of each gene (excluding D loop) were selected for DNA sequencing. The DNA of these loci from representatives of all known thoroughbred female lines was also sequenced. Sequencing was performed using an Applied Biosystems ABI PRISM 377 sequencer/genotyper. Amplification products using the relevant primers sequences detailed above were cleaned for sequencing using ChargeSwitch PCR clean-up kits (DNA Research Instruments). 2ul of PCR product was added to 18ul of water and sequencing of the regions was carried out using the forward and reverse primers in both directions.

Sequences were analysed using "Bioedit "programme (Hall, 1999).

18 mtDNA loci were analysed by Polymerase Chain Reaction-Single Strand Conformational Polymorphism (PCR-SSCP). NADH dehydrogenase 2, NADH dehydrogenase 4, NADH dehydrogenase 4L, NADH dehydrogenase 5, cytochrome oxidase I, cytochrome oxidase II, ATPase 6, cytochrome b, rRNA 16S-1, rRNA 16S-2, rRNA 12S and dloop presented variation in the horse samples tested.

The genes coding for NADH hydrogenase 1, 3 and 6, ATP synthase 8 and cytochrome oxidase 3 showed no allelic variation.

Variants sequences (5' to 3') are as follows:

| **LOCUS** | **12S rRNA** | **931bp** |
|---|---|---|
| Variant ORIGIN | A | |
| 1 CATAG GCTTG GTCCT AGCCT TTTTA TTAGT TATTA ATAGA ATTAC ACATG 51 CAAGT ATCCG CACCC CAGTG AGAAT GCCCT CTAAA TCACG TCTCT ACGAT 101 TAAAA GGAGC AGGTA TCAAG CACAC TAGAA AGTAG CTCAT AACAC CTTGC 151 TCAGC CACAC CCCCA CGGGA CACAG CAGTG ATAAA AATTT AGCTA TGAAC 201 GAAAG TTCGG CTAAG TCATT TTAAA TAAGG GTTGG TAAAT TTCGT GCCAG 251 CCCCC GCGGT CATAC GATTA ACCCA AATTA ATAAA CCTCC GGCGT AAAGC 301 GTGTC AAAGA CTAAT ACCAA AATAA AGTTA AAACC CAGTT AAGCC GTAAA 351 AAGCT ACAAC CAAAG TAAAA TAGAC TACGA AAGTG ACTTT AATAC CTCTG 401 ACTAC ACGAT AGCTA AGACC CAAAC TGGGA TTAGA TACCC CACTA TGCTT 451 AGCCC TAAAC TAAAA TAGCT TACCA CAACA AAGCT ATTCG CCAGA GTACT 501 ACTAG CAACA GCCTA AAACT CAAAG GACTT GGCGG TGCTT TACAT CCCTC 551 TAGAG GAGCC TGTTC CATAA TCGAT AAACC CCGAT AAACC CCACC ATCCC 601 TTGCT AATTC AGCCT ATATA CCGCC ATCTT CAGCA AACCC TAAAC AAGGT 651 ACCGA AGTAA GCACA AATAT CCAAC ATAAA AACGT TAGGT CAAGG TGTAG 701 CCCAT GGGAT GGAGA GAAAT GGGCT ACATT TTCTA CCCTA AGAAC AAGAA 751 CTTTA ACCCG GACGA AAGTC TCCAT GAAAC TGGAG ACTAA AGGAG GATTT 801 AGCAG TAAAT TAAGA ATAGA GAGCT TAATT GAATC AGGCC ATGAA GCGCG 851 CACAC ACCGC CCGTC ACCCT CCTTA AATAT CACAA ATCAC AACAT AACAT | | |
| 901 AAAAC CGTGA CCCAA ACATG TGAAA GGAGA CAAGT CGTAA CAAGG TAAGT 951 ATACC GGAAG GTGTA CTTGG ATAAC | | |

| Variant ORIGIN | B | |
|---|---|---|
| 1 CATAG GCTTG GTCCT AGCCT TTTTA TTAGT TATTA ATAGA ATTAC ACATG 51 CAAGT ATCCG CACCC CAGTG AGAAT GCCCT CTAAA TCACG TCTCT ACGAT 101 TAAAA GGAGC AGGTA TCAAG CACAC TAGAA AGTAG CTCAT AACAC CTTGC 151 TCAGC CACAC CCCCA CGGGA CACAG CAGTG ATAAA AATTA AGCTA TGAAC 201 GAAAG TTCGA CTAAG TCAAT TTAAA TAAGG GTTGG TAAAT TTCGT GCCAG 251 CCACC GCGGT CATAC GATTA ACCCA AATTA ATAAA CCTCC GGCGT CAAGT 301 GTGTC AAAGA CTAAT ACCAA AATAA AGTTA AAACC CAGTT AAGCC GTAAA 351 AAGCT ACAAC CAAAG TAAAA TAGAC TACGA AAGTG ACTTT AATAC CTCTG 401 ACTAC ACGAT AGCTA AGACC CAAAC TGGGA TTAGA TACCC CACTA TGCTT 451 AGCCC TAAAC TAAAA TAGCT TACCA CAACA AAGCT ATTCG CCAGA GTACT 501 ACTAG CAACA GCCTA AAACT CAAAG GACTT GGCGG TGCTT TACAT CCCTC 551 TAGAG GAGCC TGTTC CATAA TCGAT AAACC CCGAT AAACC CCACC ATCCC 601 TTGCT AATTC AGCCT ATATA CCGCC ATCTT CAGCA AACCC TAAAC AAGGT 651 ACCGA AGTAA GCACA AATAT CCAAC ATAAA AACGT TAGGT CAAGG TGTAG 701 CCCAT GGGAT GGAGA GAAAT GGGCT ACATT TTCTA CCCTA AGAAC AAGAA 751 CTTTA ACCCG GACGA AAGTC TCCAT GAAAC TGGAG ACCAA AGGAG GATTT 801 AGCAG TAAAT TAAGA ATAGA GAGCT TAATT GAATC AGGCC ATGAA GCGCG 851 CACAC ACCGC CCGTC ACCCT CCTTA AATAT CACAA ATCAT AACAT AACAT 901 AAAAC CGTGA CCCAA ACATA TGAAA GGAGA CAAGT CGTAA CAAGG TAAGT 951 ATACC GGAAG GTGTA CTTGG ATAAC | | |

| Variant ORIGIN | C | |
|---|---|---|
| 1 CATAG GCTTG GTCCT AGCCT TTTTA TTAGT TATTA ATAGA ATTAC ACATG 51 CAAGT ATCCG CACCC CAGTG AGAAT GCCCT CTAAA TCACG TCTCT ACGAT 101 TAAAA GGAGC AGGTA TCAAG CACAC TAGAA AGTAG CTCAT AACAC CTTGC 151 TCAGC CACAC CCCCA CGGGA CACAG CAGTG ATAAA AATTA AGCTT TGACC 201 GAAAG TTCGG CTAAG TCATT TTAAA TAAGG GTTGG TAAAT TTGGT GCCAG 251 CCACC GCGGT CATAC GATTT ACCCA AATTA ATAAA CCTCC GGCGT CAAGC 301 GTGTC AAAGA CTAAT GCCAA AATAA AGTTA AAACC CAGTT AAGCT GTAAA 351 AAGCT ACAAC CAAAG TAAAA TAGAC TACGA AAGTG ACTTT AATAC CTCTG 401 ACTAC ACGAT AGCTA AGACC CAAAC TGGGA TTAGA TACCC CACTA TGCTT 451 AGCCC TAAAC TAAAA TAGCT TACCA CAACA AAGCT ATTCG CCAGA GTACT 501 ACTAG CAACA GCCTA AAACT CAAAG GACTT GGCGG TGCTT TACAT CCCTC 551 TAGAG GAGCC TGTTC CATAA TCGAT AAACC CCGAT AAACC CCACC ATCCC 601 TTGCT AATTC AGCCT ATATA CCGCC ATCTT CAGCA AACCC TAAAC AAGGT 651 ACCGA AGTAA GCACA AATAT CCAAC ATAAA AACGT TAGGT CAAGG TGTAG 701 CCCAT GGGAT GGAGA GAAAT GGGCT ACATT TTCTA CCCTA AGAAC AAGAA 751 CTTTA ACCCG GACGA AAGTC TCCAT GAAAC TGGAG ACTAA AGGAG GATTT 801 AGCAG TAAAT TAAGA ATAGA GAGCT TAATT GAATC AGGCC ATGAA GCGCG 851 CACAC ACCGC CCGTC ACCCT CCTTA AATAT CACAA ATCAT AACAT AACAT 901 AAAAC CGTGA CCCAA ACATA TGAAA GGAGA CAAGT CGTAA CAAGG TAAGT 951 ATACC GGAAG GTGTA CTTGG ATAAC | | |

| LOCUS | **16S-1** | **825bp** |
|---|---|---|
| Variant ORIGIN | A | |
| 1 CTAAA GCTAG CCCAA ACAAT ACCTA ATTCA ATTAC CCTAA TTCAC TTAAC | | |
|  51 TAAAA CATTC ACCAA ACCAT TAAAG TATAG GAGAT AGAAA TTTTA ACTTG 101 GCGCT ATAGA GAAAG TACCG TAGAG GAAAC GATGA AAGAT GCATT AAAAG 151 TACTA AACAG CAAAG CTTAC CCCTT TTACC TTTTG CATAA TGATT TAACT 201 AGAAT AAACT TAGCA AAGAG AACTT AAGCT AAGCA CCCCG AAACC AGACG 251 AGCTA CCTAT GAACA GTTAC AAATG AACCA ACTCA TCTAT GTCGC AAAAT 301 AGTGA GAAGA TTCGT AGGTA GAGGT GAAAA GCCCA ACGAG CCTGG TGATA 351 GCTGG TTGTC CAGAA ACAGA ATTTC AGTTC AAATT TAAAT TTACC TAAAA 401 ACTAC TCAAT TCTAA TGTAA ATTTA AATTA TAGTC TAAAA AGGTA CAGCT 451 TTTTA GATAC AGGTT ACAAC CTTTA TTAGA GAGTA AGAAC AAGAT AAACC 501 CATAG TTGGC TTAAA AGCAG CCATC AATTA AGAAA GCGTT CAAGC TCAAC 551 GACAC ATCTA TCTTA ATCCC AACAA TCAAC CCAAA CTAAC TCCTA ATCTC 601 ATACT GGACT ATTCT ATCAA CACAT AGAAG CAATA ATGTT AATAT GAGTA 651 ACAAG AATTA TTTCT CCTTG CATAA GCCTA TATCA GAACG AATAC TCACT 701 GATAG TTAAC AACAA GATAG GGATA ATCCA AAAAC TAATC ATCTA TTTAA 751 ACCAT TGTTA ACCCA ACACA GGCTT GCTTC TTTAA GGAAA GATTA AAAGA 801 AGTAA AAGGA ACTCG GCAAC CCCAA AC | | |

| Variant ORIGIN | B | |
|---|---|---|
| 1 CTAAA GCTAG CCCAA ACAAT ACCTA ATTCA ATTAC CCGTA TTCAC TTAAC 51 TAAAA CATTC ACCAA ACCAT TAAAG TATAG GAGAT AGAAA TTTTA ACTTG 101 GCGCT ATAGA GAAAG TACCG TAGAG GGAAC GATGA AAGAT GCATT AAAAG 151 TACTA AACAG CAAAG CTTAC CCCTT TTACC TTTTG CATAA TGATT TAACT 201 AGAAT AAACT TAGCA AAGAG AACTT AAGCT AAGCA CCCCG AAACC AGACG 251 AGCTA CCTAT GAACA GTTAC AAATG AACCA ACTCA TCTAT GTCGC AAAAT 301 AGTGA GAAGA TTCGT AGGTA GAGGT GAAAA GCCCA ACGAG CCTGG TGATA 351 GCTGG TTGTC CAGAA ACAGA ATTTC AGTTC AAATT TAAAT TTACC TAAAA 401 ACTAC TCAAT TCTAA TGTAA ATTTA AATTA TAATC TAAAA AGGTA CAGCT 451 TTTTA GATAC AGGTT ACAAC CTTCA TTAGA GAGTA AGAAC AAGAT AAACC 501 CATAG TTGGC TTAAA AGCAG CCATC AATTA AGAAA GCGTT CAAGC TCAAC 551 GACAT ATCTA TCTTA ATCCC AACAA TCAAC CCAAA CTAAC TCCTA ATCTC 601 ATACT GGACT ATTCT ATCAA CACAT AGAAG CAATA ATGTT AATAT GAGTA 651 ACAAG AATTA TTTCT CCTTG CATAA GCTTA TATCA GAACG AATAC TCACT 701 GATAG TTAAC AACAA GATAG GGATA ATCCA AAAAC TAATC ATCTA TTTAA 751 ACCAT TGTTA ACCCA ACACA GGCTT GCTTC TTTAA GGAAA GATTA AAAGA 801 AGTAA AAGGA ACTCG GCAAC CCCAA AC | | |

| **LOCUS** | **16S-2** | **811bp** |
|---|---|---|
| Variant ORIGIN | A | |
| 1 TGTTA ACCCA ACACA GGCTT GCTTC TTTAA GGAAA GATTA AAAGA AGTAA 51 AAGGA ACTCG GCAAC CCCAA ACCCC GCCTG TTTAC CAAAA ACATC ACCTC 101 TAGCA TTTCC AGTAT TAGAG GCACT GCCTG CCCAG TGACA TCTGT TTAAA 151 CGGCC GCGGT ATCCT AACCG TGCAA AGGTA GCATA ATCAC TTGTT CCCTA 201 AATAG GGACT TGTAT GAATG GCCAC ACGAG GGTTT TACTG TCTCT TACTT 251 CCAAT CAGTG AAATT GACCT TCCCG TGAAG AGGCG GGAAT GACTA AATAA 301 GACGA GAAGA CCCTA TGGAG CTTTA ATTAA CTGAT TCACA AAAAA CAACA 351 CACAA ACCTT AACCT TCAGG GACAA CAAAA CTTTT GATTG AATCA GCAAT 401 TTCGG TTGGG GTGAC CTCGG AGAAC AAAAC AACCT CCGAG TGATT TAAAT 451 CCAGA CTAAC CAGTC AAAAT ATATA ATCAC TTATT GATCC AAACC ATTGA 501 TCAAC GGAAC AAGTT ACCCT AGGGA TAACA GCGCA ATCCT ATTCC AGAGT 551 CCATA TCGAC AATTA GGGTT TACGA CCTCG ATGTT GGATC AAGAC ATCCT 601 AATGG TGCAA CCGCT ATTAA GGGTT CGTTT GTTCA ACGAT TAAAG TCTTA 651 CGTGA TCTGA GTTCA GACCG GAGTA ATCCA GGTCG GTTTC TATCT ATTCT | | |
| 701 ATACT TTTCC CAGTA CGAAA GGACA AGAAA AGTAG GGCCC ACTTT ACAAG 751 AAGCG CCCTC AAACT AATAG ATGAC ATAAT CTAAA TCTAA CTAAT TTATA 801 ACTTC TACCG CC | | |

| Variant ORIGIN | B | |
|---|---|---|
| 1 TGTTA ACCCA ACACA GGCTT GCTTC TTTAA GGAAA GATTA AAAGA AGTAA 51 AAGGA ACTCG GCAAC CCCAA ACCCC GGCTG TTTAC CAAAA ATATC ACCTC 101 TAGCA TTTGC AGTAT TAGAG GGACT GCCCG CCCAG TGACA TCTTT TTAAA 151 CGGCC GCGGT ATCCT AACCG TGCAA AGGTA GCATA ATCAC TTGTT CCCTA 201 AATAG GGACT TGTAT GAATG GCCAC ACGAG GGTTT TACTG TCTCT TACTT 251 CCAAT CAGTG AAATT GACCT TCCCG TGAAG AGGCG GGAAT GACTA AATAA 301 GACGA GAAGA CCCTA TGGAG CTTTA ATTAA CTGAT TCACA AAAAA CAACA 351 CACAA ACCTT AACCT TCAGG GACAA CAAAA CTTTT GATTG AATCA GCAAT 401 TTCGG TTGGG GTGAC CTCGG AGAAC AAAAC AACCT CCGAG TGATT TAAAT 451 CCAGA CTAAC CAGTC AAAAT ATATA ATCAC TTATT GATCC AAACC ATTGA 501 TCAAC GGAAC AAGTT ACCCT AGGGA TAACA GCGCA ATCCT ATTCC AGAGT 551 CCATA TCGAC AATTA GGGTT TACGA CCTCG ATGTT GGATC AAGAC ATCCT 601 AATGG TGCAA CCGCT ATTAA GGGTT CGTTT GTTCA ACGAT TAAAG TCTTA 651 CGTGA TCTGA GTTCA GACCG GAGTA ATCCA GGTCG GTTTC TATCT ATTCT 701 ATACT TTTCC CAGTA CGAAA GGACA AGAAA AGTAG GGCCC ACTTT ACAAG 751 AAGCG CCCTC AAACT AATAG ATGAC ATAAT CTAAA TCTAA CTAAT TTATA 801 ACTTC TACCG CC | | |

| **LOCUS** | **ATP6** | **671 bp** |
|---|---|---|
| Variant ORIGIN | A | |
| 1 AAATC TATTC GCCTC TTTCG CTACC CCAAC AATAG TAGGC CTCCC TATTG 51 TAATT CTGAT CAACA TATTT CCcAA CATCC TATtG CCCTC ACCCA ACCGA 101 CTAAT TAACA ATCGC CTAAT CTCAA TTCAA CAATG GCTAG TCCAA CTTAC 151 ATCAA AACAA ATAAT AGCTA TCCAT AACAA CAAAG GACAA ACCTG AACTC 201 TTATG CTCAT ATCAC TGATC CTATT CATTG GCTCA ACAAA CTTAT TAGGC 251 CTACT ACCTC ACTCA TTCAC ACCAA CAACA CAACT ATCAA TAAAC CTAGG 301 CATAG CTATC CCCCT ATGGG CAGGG ACAGT ATTCA TAGGC TTTCG TCACA 351 AAACA AAAGC AGCCC TAGCC CACTT TCTAC CTCAA GGAAC GCCCA TTTTC 401 CTCAT CCCCA TACTA GTAAT TATCG AGACT ATCAG CCTAT TTATT CAACC 451 TGTAG CCCTA GCCGT GCGGC TAACC GCTAA CATTA CCGCC GGACA CCTCC 501 TAATA CACCT CATCG GAGGG GCAAC ACTAG CCCTC ATAAG CATCA GCCCC 551 TCAAC AGCCC TTATT ACGTT TATCA TCCTA ATTCT TCTAA CTATT CTCGA 601 ATTCG CAGTA GCTAT AATCC AAGCC TACGT ATTCA CTCTC CTGGT AAGCC 651 TTTAC TTACA CGACA ACACC T | | |

| Variant ORIGIN | B | |
|---|---|---|
| 1 AAATC TATTC GCCTC TTTCG CTACC CCAAC AATAG TAGGC CTCCC TATTG 51 TAATT CTGAT cATCA TATTT CCCAA CATCC TATtG CCTTC ACCCA ACCGA 101 CTAAT TAACA ATCGC CTAAT CTCAA TTCAA CAATG GCTAG TCCAA CTTAC 151 ATCAA AACAA ATAAT AGCTA TCCAT AACAG CAAAG GACAA ACCTG AACTC 201 TTATA CTCAT ATCAC TGATC CTATT CATTG GCTCA ACAAA CTTAT TAGGC 251 CTACT ACCTC ACTCA TTTAC ACCAA CAACA CAACT ATCAA TAAAC CTAGG 301 CATAG CTATT CCCCT ATGGG CAGGG ACAGT GTTCA TGGGC TTTCG TCATA 351 AAACA AAAGC AGCCC TAGCC CACTT TCTAC CTCAA GGAAC GCCCA TTTTC 401 CTCAT CCCCA TACTA GTAAT TATCG AGACT ATCAG CCTAT TTATT CAACC 451 TGTAG CCCTA GCCGT GCGGC TAACC GCTAA CATTA CCGCC GGACA CCTCC | | |
| 501 TAATA CACCT CATCG GAGGG GCAAC ACTAG CCCTC ATAAG CATCA GCCCC 551 TCAAC AGCCC TTATT ACGTT TATCA TCCTA ATTCT ACTAA CTATT CTCGA 601 ATTCG CAGTA GCTAT AATCC AAGCC TACGT ATTCA CTCTC CTGGT AAGCC 651 TTTAC TTACA CGACA ACACC T | | |

| Variant ORIGIN | C | |
|---|---|---|
| 1 AAATC TATTC GCCTC TTTCG CTACC CCAAC AATAG TAGGC CTCCC TATTG 51 TAATT CTGAT cAACA TATTC CCCAA CATCC TATTC CCTTC ACCCA ACCGA 101 CTAAT CAACA ATCGC CTAAT CTCAA TTCAA CAATG GCTAG TCCAA CTTAC 151 ATCAA AACAA ATAAT AGCTA TCCAT AACAG CAAAG GACAA ACCTG AACTC 201 TTATA CTCAT ATCAC TGATC CTATT CATTG GCTCA ACAAA CTTAT TAGGC 251 CTACT ACCTC ACTCA TTTAC ACCAA CAACA CAACT ATCAA TAAAC CTAGG 301 CATAG CTATT CCCCT ATGGG CAGGG ACAGT GTTCA TAGGC TTTCG TCATA 351 AAACA AAAGC AGCCC TAGCC CACTT TCTAC CTCAA GGAAC GCCCA TTTTC 401 CTCAT CCCCA TACTA GTAAT TATCG AGACT ATCAG CCTAT TTATT CAACC 451 TGTAG CCCTA GCCGT GCGGC TAACC GCTAA CATTA CCGCC GGACA CCTCC 501 TAATA CACCT CATCG GAGGG GCAAC ACTAG CCCTC ATAAG CATCA GCCCC 551 TCAAC AGCCC TTATT ACGTT TATCA TCCTA ATTCT ACTAA CTATT CTCGA 601 ATTCG CAGTA GCTAT AATCC AAGCC TACGT ATTCA CTCTC CTGGT AAGCC 651 TTTAC TTACA CGACA ACACC T | | |

| Variant ORIGIN | D | |
|---|---|---|
| 1 AAATC TATTC GCCTC TTTCG CTACC CCAAC AATAG TAGGC CTCCC TATTG 51 TAATT CTGAT CATCA TATTC CCCAG CATCC TATTC CCCTC ACCCA ACCGA 101 CTAAT CAACA ATCGC CTAAT CTCAA TTCAA CAATG GCTAG TCCAA CTTAC 151 ATCAA AACAA ATAAT AGCTA TCCAT AACAG CAAAG GACAA ACCTG AACTC 201 TTATA CTCAT ATCAC TGATC CTATT CATTG GCTCA ACAAA CTTAT TAGGC 251 CTACT ACCTC ACTCA TTTAC ACCAA CAACA CAACT ATCAA TAAAC CTAGG 301 CATAG CTATT CCCCT ATGGG CAGGG ACAGT ATTCA TAGGC TTTCG TCACA 351 AAACA AAAGC AGCCC TAGCC CACTT TCTAC CTCAA GGGAC GCCCA TTTTC 401 CTCAT CCCCA TACTA GTAAT TATCG AGACT ATCAG CCTAT TTATT CAACC 451 TGTAG CCCTA GCCGT GCGGC TAACC GCTAA CATTA CCGCC GGACA CCTCC 501 TAATA CACCT TATCG GAGAG GCAAC ACTAG CCCTC ATAAG CATCA GCCCC 551 TCAAC AGCCC TTATT ACGTT TATCA TCCTA ATTCT ACTAA CTATC CTCGA 601 ATTCG CAGTA GCTAT AATCC AAGCC TACGT ATTCA CTCTC CTGGT AAGCC 651 TTTAC TTACA CGACA ACACC T | | |

| Variant ORIGIN | E | |
|---|---|---|
| 1 AAATC TATTC GCCTC TTTCG CTACC CCAAC AATAG TAGGC CTCCC TATTG 51 TAATT CTGAT CATCA TATTT CCCAG CATCC TATTC CCCTC ACCCA ACCGA 101 CTAAT TAACA ATCGC CTAAT CTCAA TTCAA CAATG GCTAG TCCAA CTTAC 151 ATCAA AACAA ATAAT AGCTA TCCAT AACAG CAAAG GACAA ACCTG AACTC 201 TTATA CTCAT ATCAC TGATC CTATT CATTG GCTCA ACAAA CTTAT TAGGC 251 CTACT ACCTC ACTCA TTTAC ACCAA CAACA CAACT ATCAA TAAAC CTAGG 301 CATAG CTATT CCCCT ATGGG CAGGG ACAGT ATTCA TAGGC TTTCG TCACA 351 AAACA AAAGC AGCCC TAGCC CACTT TCTAC CTCAA GGGAC GCCCA TTTTC 401 CTCAT CCCCA TACTA GTAAT TATCG AGACT ATCAG CCTAT TTATT CAACC 451 TGTAG CCCTA GCCGT GCGGC TAACC GCTAA CATTA CCGCC GGACA CCTCC 501 TAATA CACCT CATCG GAGGG GCAAC ACTAG CCCTC ATAAG CATCA GCCCC 551 TCAAC AGCCC TTATT ACGTT TATCA TCCTA ATTCT TCTAA CTATC CTCGA 601 ATTCG CAGTA GCTAT AATCC AAGCC TACGT ATTCA CTCTC CTGGT AAGCC | | |
| 651 TTTAC TTACA CGACA ACACC T | | |
| **LOCUS** | **CCO1** | **1000 bp** |

| Variant ORIGIN | A | |
|---|---|---|
| 1 ACATC GGCAC TCTGT ACCTC CTATT CGGCG CTTGA GCTGG AATAG TAGGA 51 ACTGC CCTAA GCCTC CTAAT CCGTG CTGAA TTAGG CCAAC CTGGG ACCCT 101 ACTAG GAGAT GATCA GATCT ACAAT GTCAT TGTAA CCGAC CATGC ATTCG 151 TAATA ATTTT CTGTA TGGGC ATACC CATAA TAATC GGCGG ATTCA GAAAC 201 TGATT AGTCC CCCTG ATAAT TGGTG CACCT GAAAT AGCTT GCCCC CGAAT 251 AAAAA ACATA AGCTG CTGAT TACTT CCCCC ATCAT TCCTA CTTCT TCTCG 301 CTTCC TCAAT AATTG AAGCA GGTGC CGGAA CAGGC TGAAC CGTAT ATCCT 351 CCTCT AGCTG GAAAT CTGGC GCATG CAGGA GCCTC TGTTG ACTTA ACCAT 401 TTTCT CTCTC CACCT GGCTG GGGTG TCCTC GATTT TAGGT GCCAT CAACT 451 TTATT ACCAC AATCA TTAAC ATAAA ACCAC CAGCT CTATC CCAAT ATCAA 501 ACCCC CCTAT TCGTT TGATC TGTCC TTATT ACGGC AGTAC TCCTT CTCCT 551 AGCCC TCCCG GTCCT AGCAG CAGGC ATTAC CATGC TTCTC ACAGA CCGTA 601 ACCTA AACAC TACTT TCTTC GACCC CGCAG GAGGA GGGGA TCCAA TCCTT 651 TATCA ACACC TATTC TGATT CTTCG GACAC CCCGA AGTCT ATATT CTTAT 701 CCTAC CAGGC TTCGG TATAA TCTCA CACAT CGTCA CATCC AACTC AGGTA 751 AGAAG GTACG TTTCG GCTAC ATGGG AAAAG TGTGA GCTAC AATAT CCATC 801 GGCGT TGTAG GCTTC ATCGA ATGGG CGCGC CTCAT GTTTA CAGTA GGGAT 851 AGACG TTGAC ACACG CGCAT GCTTC GCATC AGCTA CCATA ATCAT CGCTA 901 TCCCT ACTGG TGTAA AAGTA TTCAG CTGAC TAGCC ACCCT GCACG GAGGA 951 AATAT CAAAT GATCT CCAGC TATAC TCTGA GCTCT AGGCT TCATC TTCTT | | |

| Variant ORIGIN | B | |
|---|---|---|
| 1 ACATC GGCAC TCTGT ACCTC CTATT CGGCG CTTGA GCTGG AATAG TAGGA 51 ACTGC CCTAA GCCTC CTAAT CCGTG CTGAA TTAGG CCAAC CTGGG ACCCT 101 ACTAG GAGAT GATCA GATCT ACAAT GTCAT GGTAC CCGCG CATGC ATTCG 151 TAATA ATATT CTTTA TGGAC ATACC CATAA TAATC GGCGG ATTCA GAAAC 201 TGATT AGTAC CCCTG ATAAC TGGTG CACCC GTAAT AGCTT TCTCC CAAAT 251 GAACA ACATA AGCTG CTGAT TACTT CCACC ATCAT TCCTA CTTCT TCTCG 301 CTTCC TCAAT AATTG AAGCA GGTGC CGGAA CAGGC TGAAC CGTAT ATCCT 351 CCTCT AGCTG GAAAT CTGGC GCATG CAGGA GCCTC TGTTG ACTTA ACCAT 401 TTTCT CTCTC CACCT AGCTG GGGTG TCCTC GATTT TAGGT GCCAT CAACT 451 TTATT ACCAC AATCA TTAAC ATAAA ACCAC CAGCT CTATC CCAAT ATCAA 501 ACCCC CCTAT TCGTT TGATC TGTCC TTATT ACGGC AGGAC TCCTT CTCCT 551 AGCCC TCCCG GTCCT AGCAG CAGGC ATTAC CATGC TTCTC ACAGA CCGTA 601 ACCTA AACAC TACTT TCTTC GACCC CGCAG GAGGA GGGGA TCCAA TCCTT 651 TATCA ACACC TATTC TGATT CTTCG GACAC CCCGA AGTCT ATATT CTTAT 701 CCTAC CAGGC TTCGG TATAA TATCA CACAT CGTCA CAACC ACATC AGGTA 751 TAAAG GTGCC TTTCG GCTAC AAGGG CACAG CGTGA GCTAA AATAT CCATT 801 GGCTT TGTAG GCTTC ATCGA ATGGG CGCGC CTCAT GTTTA CAGTA GGGAT 851 AGACG TTGAC ACACG CGCAT ACTTA GCATC AGCTA CCATA ATCAT CGCTA 901 TCCCT ACTGG TGTAA AAGTA TTCAG CTGAC TAGCC ACCCT GCACG GAGGA 951 AATAT CAAAT GATCT CCAGC TATAC TCTGA GCTCT AGGCT TCATC TTCTT | | |

| Variant ORIGIN | C | |
|---|---|---|
| | | |
| 1 ACATC GGCAC TCTGT ACCTC CTATT CGGCG CTTGA GCTGG AATAG TAGGA 51 ACTGC CCTAA GCCTC CTAAT CCGTG CTGAA TTAGG CCAAC CTGGG ACCCT 101 ACTAG GAGAT GATCA GATCT ACAAT GTCAT TGTAA CCGCC CATGC ATTCG 151 TAATA ATTTT CTTTA TGGTC ATACC CATTA TAATC GGAGG ATTCG GAAAC 201 TGATT AGTCC CCCTG ATAAT TGGAG CACCT GATAT AGCTT TCCCC CGAAT 251 AAACA ACATA AGCTT CTGAT TACTT CCCCC ATCAT TCCTA CTTCT TCTCG 301 CTTCC TCAAT AATTG AAGCA GGTGC CGGAA CAGGC TGAAC CGTAT ATCCT 351 CCTCT AGCTG GAAAT CTGGC GCATG CAGGA GCCTC TGTTG ACTTA ACCAT 401 TTTCT CTCTC CACCT AGCTG GGGTG TCCTC GATTT TAGGT GCCAT CAACT 451 TTATT aCCaC AATCA TTAAC ATAAA ACCAC CAGCC CTATC CCAAT ATCAA 501 ACCCC CCTAT TCGTT TGATC TGTCC TTATT ACGGC AGTAC TCCTT CTCCT 551 AGCCC TCCCG GTCCT AGCAG CAGGC ATTAC CATGC TTCTC ACAGA CCGTA 601 ACCTG AACAC TACTT TCTTC GACCC CGCAG GAGGA GGGGA TCCAA TCCTT 651 TATCA ACACC TATTC TGATT CTTCG GACAC CCCGA AGTCT ATATT CTTAT 701 CCTAC CAGGC TTCGG TATAA TCTCA CACAT CGTCA CATAC TACTC AGGTA 751 AAAAG GAACC TTTTG GCTAC ATGGG TATAG TGTGA GCTAT AATAT CCATT 801 GGCTT TCTAG GCTTC ATCGT ATGGG CTCAC CACAT GTTTA CAGTA GGGAT 851 AGACG TTGAC ACACG AGCAT ACTTC ACATC AGCTA CCATA ATCAT CGCTA 901 TCCCT ACTGG TGTAA AAGTA TTCAG CTGAC TAGCC ACCCT GCACG GAGGA 951 AATAT CAAAT GATCT CCAGC TATAC TCTGA GCTCT AGGCT TCATC TTCTT | | |

| Variant ORIGIN | D | |
|---|---|---|
| 1 ACATC GGCAC TCTGT ACCTC CTATT CGGCG CTTGA GCTGG AATAG TAGGA 51 ACTGC CCTGA GCCTG CTAAT ACGTG CTGAA TTAGG CCAAC CTGGG ACCCT 101 ACTAG GAGAT GATCA GATCT ACAAT GTTAT GGTAA CCGCC CATGC ATTCG 151 TAATA ATTTT CTTTA TGGGC ATACC CATTA TAATC GGAGG ATTCG GAAAC 201 TGATT AGTCC CCCTG ATAAT TGGAG CACCT GAAAT AGCTT TCCCC CGAAT 251 AAACA ACATA AGCTT CTGAT TACTT CCCCC ATCAT TCCTA CTTCT TCTCG 301 CTTCC TCAAT AATTG AAGCA GGTGC CGGAA CAGGC TGAAC CGTAT ATCCT 351 CCTCT AGCTG GAAAT CTGGC GCATG CAGGA GCCTC TGTTG ACTTA ACCAT 401 TTTCT CTCTC CACCT AGCTG GGGTG TCTTC GATTT TAGGT GCCAT CAACT 451 TTATT ACCAC AATCA TTAAC ATAAA ACCAC CAGCT CTATC CCAAT ATCAA 501 ACCCC CCTAT TCGTT TGATC TGTCC TTATT ACGGC AGTAC TCCTT CTCCT 551 AGCCC TCCCG GTCCT AGCAG CAGGC ATTAC CATGC TTCTC ACAGA CCGTA 601 ACCTA AACAC TACTT TCTTC GACCC CGCAG GAGGA GGGGA TCCAA TCCTT 651 TATCA ACACC TATTC TGATT CTTCG GGCAC CCCGA AGTCT ATATT CTTAT 701 CCTAC CAGGC TTCGG TATAA TCTCA CACAT CGTCA CATAC TACTC AGGTA 751 AAAAG GAACC TTTTG GCTAC ATGGG TATAG TGTGA GCTAT AATAT CCATT 801 GGCTT TCTAG GCTTC ATCGT ATGGG CTCAC CACAT GTTTA CAGTA GGGAT 851 AGACG TTGAC ACACG AGCAT ACTTC GCATC AGCTA CCATA ATCAT CGCTA 901 TCCCT ACCGG TGTAA AAGTA TTCAG ATGTC TAGCC ACCCT GCACG GAGGA 951 AATAT CAAAT GATCT CCAGC TATAC TCTGA GCTCT AGGCT TCATC TTCTT | | |

| **LOCUS** | **CCO2** | **668 bp** |
|---|---|---|
| Variant ORIGIN | A | |
| 1 CCCTT CCAAC TAGGA TTCCA AGACG CAACA TCCCC TATTA TAGAA GAACT 51 CCTAC ACTTC CACGA CCACA CACTA ATAAT CGTAT TCCTA ATTAG CTCTC 101 TAGTA TTATA TATTA TCTCA TCAAT ACTAA CAACT AAATT AACCC ATACC 151 AGCAC CATAG ATGCT CAAGA AGTAG AGACA ATTTG AACGA TTTTA CCAGC 201 CATCA TCCTT ATTCT AATCG CCCTC CCATC CCTAC GAATT CTATA TATAA 251 TAGAT GAAAT CAATA ATCCG TCCCT CACAG TCAAA ACAAT AGGCC ACCAA | | |
| 301 TGATA CTGAA GCTAC GAGTA TACCG ATTAC GAAGA CTTGA CCTTT GACTC 351 CTACA TGATC CCCAC ATCAG ACCTA AAGCC AGGAG AATTA CGTCT TCTAG 401 AAGTC GACAA TCGAG TGGTT CTCCC CATAG AAATA ACCAT CCGAA TGCTA 451 ATTTC ATCCG AAGAC GTCCT ACACT CATGA GCTGT GCCCT CCCTA GGCCT 501 AAAAA CAGAC GCTAT CCCTG GGCGC CTAAA TCAGA CAACT CTCGT GGCCT 551 CTCGA CCAGG ACTTT ACTAC GGTCA ATGCT CAGAG ATCTG CGGAT CAAAC 601 CACAG CTTTA TGCCA ATTGT CCTTG AACTA GTTCC ACTGA AACAC TTCGA 651 AGAAT GATCT GCATC AAT | | |

| Variant ORIGIN | B | |
|---|---|---|
| 1 CCCTT CCAAC TAGGA TTCCA AGACG CAACA TCCCC TATTA TAGAA GAACT 51 CCTAC ACTTC CACGA CCACA CACTA ATAAT CGTAT TCCTA ATTAG CTCTC 101 TAGTA TTATA TATTA TCTCA TCAAT ACTAA CAACT AAATT AACCC ATACC 151 AGCAC CATAG ATGCT CAAGA AGTAG AGACA ATTTG AACGA TTTTA CCAGC 201 CATCA TCCTT ATTCT AATCG CCCTC CCATC CCTAC GAATT CTATA TATAA 251 TAGAT GAAAT CAATA ATCCG TCCCT CACAG TCAAA ACAAT AGGCC ACCAA 301 TGATA CTGAA GCTAC GAGTA TACCG ATTAC GAAGA CTTGA CCTTT GACTC 351 CTACA TGATC CCCAC ATCAG ACCTA AAACC AGGAG AATTA CGTCT TCTAG 401 AAGTC GACAA TCGAG TGGTT CTCCC CATAG AAATA ACCAT CCGAA TGCTA 451 ATTTC ATCCG AGGAC GTCCT ACACT CATGA GCTGT GCCCT CCCTA GGCCT 501 AAAAA CAGAC GCTAT CCCTG GGCGC CTAAA TCAGA CAACT CTCGT GGCCT 551 CTCGA CCAGG ACTTT ACTAC GGTCA ATGCT CAGAG ATCTG CGGAT CAAAC 601 CACAG CTTTA TGCCA ATTGT CCTTG AACTA GTTCC ACTGA AACAC TTCGA 651 AGAAT GATCT GCATC AAT | | |

| Variant ORIGIN | C | |
|---|---|---|
| 1 CCCTT CCAAC TAGGA TTCCA AGACG CAACA TCCCC TATTA TAGAA GAACT 51 CCTAC ACTTC CACGA CCACA CACTA ATAAT CGTAT TCCTA ATTAG CTCTC 101 TAGTA TTATA TATTA TCTCA TCAAT ACTAA CAACT AAATT AACCC ATACC 151 AGCAC CATAG ATGCT CAAGA AGTAG AGACA ATTTG AACGA TTTTA CCAGC 201 CATCA TCCTT ATTCT AATCG CCCTC CCATC CCTAC GAATT CTATA TATAA 251 TAGAT GAAAT CAATA ATCCG TCCCT CACAG TCAAA ACAAT AGGCC ACCAA 301 TGATA CTGAA GCTAC GAGTA TACCG ATTAC GAAGA CTTGA CCTTT GACTC 351 CTACA TGATC CCCAC ATCAG ACCTA AAACC AGGAG AATTA CGTCT TCTAG 401 AAGTC GACAA TCGAG TGGTT CTCCC CATAG AAATA ACCAT CCGAA TGCTA 451 ATTTC ATCCG AAGAC GTCCT ACACT CATGA GCTGT GCCCT CCCTA GGCCT 501 AAAAA CAGAC GCTAT CCCTG GGCGC CTAAA TCAGA CAACT CTCGT GGCCT 551 CTCGA CCAGG ACTTT ACTAC GGTCA ATGCT CAGAG ATCTG CGGAT CAAAC 601 CACAG CTTTA TACCA ATTGT CCTTG AACTA GTTCC ACTGA AACAC TTCGA 651 AGAAT GATCT GCATC AAT | | |

| Variant ORIGIN | D | |
|---|---|---|
| 1 CCCTT CCAAC TAGGA TTCCA AGACG CAACA TCCCC TATTA TAGAA GAACT 51 CCTAC ACTTC CACGA CCACA CACTA ATAAT CGTAT TCCTA ATTAG CTCTC 101 TAGTA TTATA TATTA TCTCA TCAAT ACTAA CAACT AAATT AACCC ATACC 151 AGCGC CATAG ATGCT CAAGA AGTAG AGACA ATTTG AACGA TTTTA CCAGC 201 CATCA TCCTT ATTCT AATCG CCCTC CCATC CCTAC GAATT CTATA TATAA 251 TAGAT GAAAT CAATA ATCCG TCCCT CACAG TCAAA ACAAT AGGCC ACCAA 301 TGATA CTGAA GCTAC GAGTA TACCG ATTAC GAAGA CTTGA CCTTT GACTC 351 CTACA TGATC CCCAC ATCAG ACCTA AAGCC AGGAG AATTA CGTCT TCTAG | | |
| 401 AAGTC GACAA TCGAG TGGTT CTCCC CATAG AAATA ACCAT CCGAA TGCTA 451 ATTTC ATCCG AAGAC GTCCT ACACT CATGA GCTGT GCCCT CCCTA GGCCT 501 AAAAA CAGAC GCTAT CCCTG GGCGC CTAAA TCAGA CAACT CTCGT GGCCT 551 CTCGA CCAGG ACTTT ACTAC GGTCA ATGCT CAGAG ATCTG CGGAT CAAAC 601 CACAG CTTTA TGCCA ATTGT CCTTG AACTA GTTCC ACTGA AACAC TTCGA 651 AGAAT GATCT GCATC AAT | | |

| **LOCUS** | **CYT B** | **1110 bp** |
|---|---|---|
| Variant ORIGIN | A | |
| 1 CATCC GGAAA TCTCA CCCAC TAATT AAAAT CATCA ATCAC TCTTT TATTG 51 ACCTA CCAGC CCCCT CAAAC ATTTC ATCAT GATGA AACTT CGGCT CCCTC 101 CTAGG AATCT GCCTA ATCCT CCAAA TCTTA ACAGG CCTAT TCCTA GCCAT 151 ACACT ACACA TCAGA CACGA CAACT GCCTT CTCAT CCGTC ACTCA CATCT 201 GCCGA GACGT TAACT ACGGA TGAAT TATTC GCTAC CTCCA TGCCA ACGGA 251 GCATC AATAT TTTTT ATCTG CCTCT TCATT CACGT AGGAC GCGGC CTCTA 301 CTACG GCTCT TACAC ATTCC TAGAG ACATG AAACA TTGGA ATCAT CCTAC 351 TTTTC ACAGT CATAG CTACA GCATT CATGG GCTAT GTCCT ACCAT GAGGC 401 CAAAT ATCCT TTTGA GGAGC AACAG TCATC ACGAA CCTCC TATCA GCAAT 451 TCCCT ACATC GGTAC TACCC TCGTC GAGTG AATCT GAGGT GGATT CTCAG 501 TAGAC AAAGC CACCC TTACC CGATT TTTTG CTTTC CACTT CATCC TACCC 551 TTCAT CATCA CAGCC CTGGT AGTCG TACAT TTACT ATTTC TTCAC GAAAC 601 AGGAT CTAAT AACCC CTCAG GAATC CCATC CGATA TGGAC AAAAT CCCAT 651 TCCAC CCATA TTATA CAATT AAAGA CATCC TAGGA CTCCT CCTCC TGATC 701 TTGCT CCTAC TAACT CTAGT ATTAT TCTCC CCCGA CCTCC TAGGA GACCC 751 AGACA ACTAC ACCCC AGCTA ACCCT CTCAG CACTC CCCCT CATAT TAAAC 801 CAGAA TGGTA CTTCC TGTTT GCCTA CGCCA TCCTA CGCTC CATTC CCAAC 851 AAACT AGGCG GCGTA TTAGC CCTAA TCCTC TCCAT CCTGA TCCTA GCACT 901 CATCC CCACC CTCCA CATAT CAAAA CAACG AAGCA TAATA TTTCG GCCTC 951 TCAGC CAATG CGTAT TCTGA CTCTT AGTGG CAGAC TTACT GACAC TAACA 1001 TGAAT CGGCG GACAG CCAGT GGAAC ACCCA TACGT AATTA TCGGC CAACT 1051 GGCCT CAATC CTCTA CTTCT CCCTA ATTCT CATTT TTATA CCACT CGCAA 1101 GCACC ATCGA A | | |

| Variant ORIGIN | B | |
|---|---|---|
| 1 CATCC GGAAA TCTCA CCCAC TAATT AAAAT CATCA ATCAC TCTTT TATTG 51 ACCTA CCAGC CCCCT CAAAC ATTTC ATCAT GATGA AACTT CGGCT CCCTC 101 CTAGG AATCT GCCTA ATCCT CCAAA TCTTA ACAGG CCTAT TCCTA GCCAT 151 ACACT ACACA TCAGA CACGA CAACT GCCTT CTCAT CCGTC ACTCA CATCT 201 GCCGA GACGT TAACT ACGGA TGAAT TATTC GCTAC CTCCA TGCCA ACGGA 251 GCATC AATAT TTTTT ATCTG CCTCT TCATT CACGT AGGAC GCGGC CTCTA 301 CTACG GCTCT TACAC ATTCC TAGAG ACATG AAACA TTGGA ATCAT CCTAC 351 TTTTC ACAGT TATAG CTACA GCATT CATGG GCTAT GTCCT ACCAT GAGGC 401 CAAAT ATCCT TTTGA GGAGC AACAG TCATC ACGAA CCTCC TATCA GCAAT 451 TCCCT ACATC GGTAC TACCC TCGTC GAGTG AATCT GAGGT GGATT CTCAG 501 TAGAC AAAGC CACCC TTACC CGATT TTTTG CTTTC CACTT CATCC TACCC 551 TTCAT CATCA CAGCC CTGGT AGTCG TACAT TTACT ATTTC TTCAC GAAAC 601 AGGAT CTAAC AACCC CTCAG GAATC CCATC CGATA TGGAC AAAAT CCCAT 651 TCCAC CCATA TTATA CAATT AAAGA CATCC TAGGA CTCCT CCTCC TGATC 701 TTGCT CCTAC TAACT CTAGT ATTAT TCTCC CCCGA CCTCC TAGGA GACCC 751 AGACA ACTAC ACCCC AGCTA ACCCT CTCAG CACTC CCCCT CATAT TAAAC 801 CAGAA TGGTA CTTCC TGTTT GCCTA CGCCA TCCTA CGCTC CATTC CCAAC 851 AAACT AGGCG GCGTA TTAGC CCTAA TCCTC TCCAT CCTGA TCCTA GCACT 901 CATCC CCACC CTCCA CATAT CAAAA CAACG AAGCA TAATA TTCCG GCCTC | | |
| 951 TCAGC CAATG CGTAT TCTGA CTCTT AGTGG CAGAC TTACT GACAC TAACA 1001 TGAAT CGGCG GACAG CCAGT GGAAC ACCCA TACGT AATTA TCGGC CAACT 1051 GGCCT CAATC CTCTA CTTCT CCCTA ATTCT CATTT TTATA CCACT CGCAA 1101 GCACC ATCGA A | | |

| Variant ORIGIN | C | |
|---|---|---|
| 1 CATCC GGAAA TCTCA CCCAC TAATT AAAAT CATCA ATCAC TCTTT TATTG 51 ACCTA CCAGC CCCCT CAAAC ATTTC ATCAT GATGA AACTT CGGCT CCCTC 101 CTAGG AATCT GCCTA ATCCT CCAAA TCTTA ACAGG CCTAT TTCTA GCCAT 151 ACACT ACACA TCAGA CACGA CAACT GCCTT CTCAT CCGTC ACTCA CATCT 201 GCCGA GACGT TAACT ACGGA TGAAT TATTC GCTAC CTCCA TGCCA ACGGA 251 GCATC AATAT TTTTT ATCTG CCTCT TCATT CACGT AGGAC GCGGC CTCTA 301 CTACG GCTCT TACAC ATTCC TAGAG ACATG AAACA TTGGA ATCAT CCTAC 351 TTTTC ACAGT TATAG CTACA GCATT CATGG GCTAT GTCCT ACCAT GAGGC 401 CAAAT ATCCT TTTGA GGAGC AACAG TCATC ACGAA CCTCC TATCA GCAAT 451 TCCCT ACATC GGTAC TACCC TCGTC GAGTG AATCT GAGGT GGATT CTCAG 501 TAGAC AAAGC CACCC TTACC CGATT TTTTG CTTTC AACTT CATCC TACCC 551 TTCAT TATCA CAGCC CTGGT AGTCG TACAT TTACT ATTTC TTCAC GAAAC 601 AGGAT CTAAT AACCC CTCAG GAATC CCATC CGATA TGGAC AAAAT CCCAT 651 TCCAC CCATA TTATA CAATT AAAGA CATCC TAGGA CTCCT CCTCC TGATC 701 TTGCT CCTAC TAACT CTAGT ATTAT TCTCC CCCGA CCTCC TAGGA GACCC 751 AGACA ACTAC ACCCC AGCTA ACCCT CTCAG CACTC CCCCT CATAT TAAAC 801 CAGAA TGGTA TTTCC TGTTT GCCTA CGCCA TCCTA CGCTC CATTC CCAAC 851 AAACT AGGCG GCGTA TTAGC CCTAA TCCTC TCCAT CCTGA TCCTA GCACT 901 CATCC CCACC CTCCA CATAT CAAAA CAACG AAGCA TAATA TTCCG GCCTC 951 TCAGC CAATG CGTAT TCTGA CTCTT AGTGG CAGAC TTACT GACAC TAACA 1001 TGAAT CGGCG GACAG CCAGT GGAAC ACCCA TACGT AATTA TCGGC CAACT 1051 GGCCT CAATC CTCTA CTTCT CCCTA ATTCT CATTT TTATA CCACT CGCAA 1101 GCACC ATCGA A | | |

| **LOCUS** | **ND4** | **1001 bp** |
|---|---|---|
| Variant ORIGIN | A | |
| 1 CAATA GCCTA AACTT CTCAC TAATA TTCTT CTCCG ATCCC CTATC AGCCC 51 CACTT CTGGT GCTGG CAACA TGACT ACTGC CACTA ATACT CATAG CCAGC 101 CAACA TCATC TATCT AAAGA ACCAC TAATC CGAAA AAAAC TCTAC ATCAC 151 CATGC TAACC ATACT TCAAA CTTTT CTAAT CATGA CTTTT ACCGC CACAG 201 AACTA ATCTC CTTCT ACATC CTATT TGAAG CCACA TTAGT TCCAA CACTA 251 ATTAT CATCA CCCGC TGAGG CAACC AAACA GAACG CCTGA ACGCA GGCCT 301 CTATT TCCTA TTCTA CACAC TAATA GGTTC CCTCC CACTC TTAGT TGCAC 351 TAATC TCTAT CCAAA ACCTA ACAGG CTCAC TAAAC TTCCT ATTAA TTCAA 401 TACTG AAACC AAGCA CTACC CGACT CTTGA TCCAA TATTT TCCTA TGACT 451 AGCAT GTATA ATAGC ATTCA TAGTC AAAAT ACCCC TATAT GGTCT TCACC 501 TCTGA CTCCC AAAAG CTCAT GTAGA AGCCC CAATT GCCGG ATCCA TAGTG 551 CTAGC AGCCA TTCTA CTAAA ACTAG GAGGC TACGG AATAC TACGA ATTAC 601 AACAA TACTA AACCC CCAAA CTAGC TTTAT AGCCT ACCCC TTCCT CATAC 651 TATCC CTGTG AGGAA TAATC ATAAC TAGTT CCATC TGCTT GCGAC AAACC 701 GATCT AAAAT CA-TT ATTGC AGACT CCTCT GTCAG CCACA TAGCC CTAGT 751 AATCG TAGCC GTCCT CATCC AAACA CCATG AAGTT ATATA GGAGC TACAG 801 CCCTA ATAAT CGCTC ACGGC CTTAC ATCAT CAATC CTATT CTGCC TGGCA 851 AACTC AAATT ACGAA TGTAC CCATA GCCGA TATAT AATCT TAGCC CGTGA | | |
|  901 GGCTT CAAAC ACTTC TTCCC CTTAT AGCAG CTTGA TGACT ATTAG CCAGC 951 CTAAC CAACC TGGCC CTCCC TCCCA GCATT AACCT AATTG GAGAG CTATT 1001 C | | |

| Variant ORIGIN | B | |
|---|---|---|
| 1 CAATA GCCTA AACTT CTCAC TAATA TTCTT CTCCG ATCCC CTATC AGCCC 51 CACTT CTGGT GTTGA CAACA TGACT ACTAC CACTA ATACT CATAG CCAGC 101 CAACA CCATC TATCT AAGGA ACCAC TAATC CGAAA AAAAC TCTAC ATCAC 151 CATGC TAACC ATACT TCAAA CTTTC CTAAT CATGA CTTTT ACCGC CACAG 201 AACTA ATCTC CTTCT ACATC CTATT TGAAG CCACA TTAGT TCCAA CACTA 251 ATTAT CATCA CCCGC TGAGG TAACC AAACA GAACG CCTGA ACGCA GGCCT 301 CTACT TCCTA TTCTA CACAC TAATA GGTTC CCTCC CACTC TTAGT TGCAC 351 TAATC TCTAT CCAAA ACCTA ACAGG CTCAC TAAAC TTCCT ATTAA TTCAA 401 TACTG AAACC AAGCA CTACC CGACT CTTGA TCCAA TATTT TCCTA TGACT 451 AGCAT GTATA ATAGC ATTCA TAGTC AAAAT ACCCC TATAT GGTCT TCACC 501 TCTGA CTCCC AAAAG CCCAT GTAGA AGCCC CAATT GCCGG ATCCA TAGTG 551 CTAGC AGCCA TTCTA CTAAA ACTAG GAGGC TACGG AATAC TACGA ATTAC 601 AACAA TACTA AACCC CCAAA CTAGC TTTAT AGCCT ACCCC TTCCT CATAC 651 TATCC CTGTG AGGAA TAATC ATAAC TAGTT CCATC TGCTT GCGAC AAACC 701 GATCT AAAAT CA-TT ATTGC ATACT CCTCT GTCAG CCACA TAGCC CTAGT 751 AATCG TAGCC GTCCT CATCC AAACA CCATG AAGTT ATATA GGAGC TACAG 801 CCCTA ATAAT CGCTC ACGGC CTTAC ATCAT CAATA CTATT CTGCC TGGCA 851 AACTC AAATT ACGAA CGTAC CCATA GCCGA ACTAT AATCC TAGCC CGTGA 901 GGCTT CAAAC ACTTC TTCCC CTTAT AGCAG CCTGA TGACT ATTAG CCAGC 951 CTAAC CAACC TGGCC CTCCC TCCCA GCATT AACCT AATTG GAGAG CTATT 1001 C | | |

| Variant ORIGIN | C | |
|---|---|---|
| 1 CAATA GCCTA AACTT CTCAC TAATA TTCTT CTCCG ATCCC CTATC AGCCC 51 CACTT CTGGA GTTGG CAACA TGACT ACTGC CACTA ATACT CATAG CCAGC 101 CAACA CCATC TATCT AAGGA ACCAC TAATC CGAAA AAAAC TCTGC ATCGC 151 CATGC TAACC ATACT TCAAA CTTTC CTAAT CATGA CTTTT ACCGC CACAG 201 AACTA ATCTC CTTCT ACATC CTATT TGAAG CCACA TTAGT TCCAA CACTA 251 ATTAT CATCA CCCGC TGAGG CAACC AAACA GAACG CCTGA ACGCA GGCCT 301 CTACT TCCTA TTCTA CACAC TAATA GGTTC CCTCC CACTC TTAGT TGCAC 351 TAATC TCTAT CCAAA ACCTA ACAGG CTCAC TAAAC TTCCT ATTAA TTCAA 401 TACTG AAACC AAGCA CTACC CGACT CTTGA TCCAA TATTT TCCTA TGACT 451 AGCAT GTATA ATAGC ATTCA TAGTC AAAAT ACCCC TATAT GGTCT TCACC 501 TCTGA CTCCC AAAAG CCCAT GTAGA AGCCC CAATT GCCGG ATCCA TAGTG 551 CTAGC AGCCA TTCTA CTAAA ATTAG GAGGC TACGG AATAC TACGA ATTAC 601 AACAA TACTA AACCC CCAAA CTAGC TTTAT AGCCT ACCCC TTCCT CATAC 651 TATCC CTGTG AGGAA TAATC ATAAC TAGTT CCATC TGCTT GCGAC AAACC 701 GATCT AAAAT CA-TT ATTGC AGACT CCTCT GTCAG CCACA TAGCC CTAGT 751 AATCG TAGCC GTCCT CATCC AAACG CCATG AAGTT ATATA GGAGC GACAG 801 CCCTA ATAAT CGCTC AGGGC CTTAC ATCAT CAATC CTATT AAGCT GGGCA 851 AACTC AAATT GCGAA TGTAC CCATA GCCGA TATAT AATCT TAGTC CGTGA 901 GGCTT CAAAC ACTTC TTCCC CTTAT AGCAG CTTGA TGACT ATTAG CCAGC 951 CTAAC CAACC TGGCC CTCCC TCCCA GCATT AACCT AATTG GAGAG CTATT 1001 C | | |

| Variant ORIGIN | D | |
|---|---|---|
| | | |
| 1 CAATA GCCTA AACTT CTCAC TAATA TTCTT CTCCG ATCCC CTATC AGCCC 51 CACTT CTGGT GTTGA CAACA TGACT ACTGC CACTA ATACT CATAG CCAGC 101 CAACA TCATC TATCT AAAGA ACCAC TAATC CGAAA AAAAC TCTAC ATCAC 151 CATGC TAACC ATACT TCAAA CTTTC CTAAT CATGA CTTTT ACCGC CACAG 201 AACTA ATCTC CTTCT ACATC CTATT TGAAG CCACA TTAGT TCCAA CACTA 251 ATTAT CATCA CCCGC TGAGG CAACC AAACA GAACG CCTGA ACGCA GGCCT 301 CTATT TCCTA TTCTA CACAC TAATA GGTTC CCTCC CACTC TTAGT TGCAC 351 TAATC TCTAT CCAAA ACCTA ACAGG CTCAC TAAAC TTCCT ATTAA TTCAA 401 TACTG AAACC AAGCA CTACC CGACT CTTGA TCCAA TATTT TCCTA TGACT 451 AGCAT GTATA ATAGC ATTCA TAGTC AAAAT ACCCC TATAT GGTCT TCACC 501 TCTGA CTCCC AAAAG CTCAT GTAGA AGCCC CAATT GCCGG ATCCA TAGTG 551 CTAGC AGCCA TTCTA CTAAA ACTAG GAGGC TACGG AATAC TACGA ATTAC 601 AACAA TACTA AACCC CCAAA CTAGC TTTAT AGCCT ACCCC TTCCT CATAC 651 TATCC CTGTG AGGAA TAATC ATAAC TAGTT CCATC TGCTT GCGAC AAACC 701 GATCT AAAAT CA-TT ATTGC ATGCT CCTCT GTCAG CCACA TAGCC CTAGT 751 AATCG TAGCC GTCCT CATCC AAACG CCATG AAGTT ATATA GGAGC TACAG 801 CCCTA ATAAT CGCTC ACGGC CTTAC ATCAT CAATA CTATT ATGCC TGGCA 851 AACTC AAATT ACGAA TGTAC CCATA GCCGA ACTAT AATCC TAGCC CGTGA 901 GGCTT CAAAC ACTTC TTCCC CTTAT AGCAG CCTGA TGACT ATTAG CCAGC 951 CTAAC CAACC TGGCC CTCCC TCCCA GCATT AACCT AATTG GAGAG CTATT 1001 C | | |

| Variant ORIGIN | E | |
|---|---|---|
| 1 CAATA GCCTA AACTT CTCAC TAATA TTCTT CTCCG ATCCC CTATC AGCCC 51 CACTT CTGGT GTTGA CAACA TGACT ACTGC CACTA ATACT CATGG CCAGC 101 CAACA CCATC TATCT AAGGA ACCAC TAATC CGAAA AAAAC TCTAC ATCAC 151 CATGC TAACC ATACT TCAAA CTTTC CTAAT CATGA CTTTT ACCGC CACAG 201 AACTA ATCTC CTTCT ACATC CTATT TGAAG CCACA TTAGT TCCGA CACTA 251 ATTAT CATCA CCCGC TGAGG CAACC AAACA GAACG CCTGA ACGCA GGCCT 301 CTACT TCCTA TTCTA CACAC TAATA GGTTC CCTCC CACTC TTAGT TGCAC 351 TAATC TCTAT CCAAA ACCTA ACAGG CTCAC TAAAC TTCCT ATTAA TTCAA 401 TACTG AAACC AAGCA CTACC CGACT CTTGA TCCAA TATTT TCCTA TGACT 451 AGCAT GTATA ATAGC ATTCA TAGTC AAAAT ACCCC TATAT GGTCT TCACC 501 TCTGA CTCCC AAAAG CCCAT GTAGA AGCCC CAATT GCCGG ATCCA TAGTG 551 CTAGC AGCCA TTCTA CTAAA ACTAG GAGGC TACGG AATAC TACGA ATTAC 601 AACAA TACTA AACCC CCAAA CTAGC TTTAT AGCCT ACCCC TTCCT CATAC 651 TATCC CTGTG AGGAA TAATC ATAAC TAGTT CCATC TGCTT GCGAC AAACC 701 GATCT AAAAT CACTT ATTGC ATACT CCTCT GTCAG CCACA TAGCC CTAGT 751 AATCG TAGCC GTCCT CATCC AAACA CCATG AAGTT ATATA GGAGC TACAG 801 CCCTA ATAAT CGCTC ACGGC CTTAC ATCAT CAATA TTATT CTGCC TGGCA 851 AACTC AAATT ACGAA CGTAC CCATA GCCGA ACTAT AATCC TAGAC CGCG- 901 GGCTT CAAAC ACTTC TTCCC CTTAT AGCAG CCTGA TGACT ATTAG CCAGC 951 CTAAC CAACC TGGCC CTCCC TCCCA GCATT AACCT AATTG GAGAG CTATT 1001 C | | |

| **LOCUS** | **ND5** | **1000 bp** |
|---|---|---|
| Variant ORIGIN | A | |
| 1 TTTCC AACTG TTCAT CGGAT GGGAG GGAGT AGGCA TCATG TCATT CTTAC 51 TAATC GGATG ATGAT ACGGC CGAAC AGATG CCAAC ACCGC GGCCC TTCAA 101 GCAAT CCTTT ATAAC CGCAT CGGGG ATATC GGCTT CATCA TGGCC ATAGC 151 CTGAT TCCTA TTCAA CACCA ACACA TGAGA CCTCC AACAA ATCTT CATAC 201 TCGAC CCCAA CCTTA CCAAC CTCCC GCTCC TAGGC CTCCT CCTAG CCGCA | | |
| 251 ACTGG CAAAT CCGCT CAATT TGGAC TCCAC CCATG ACTTC CTTCA GCCAT 301 AGAGG GCCCT ACACC AGTCT CAGCC CTACT CCACT CCAGC ACAAT AGTTG 351 TAGCA GGCGT CTTCC TGCTA ATCCG CTTCC ATCCA CTAAT AGAAA ACAAC 401 AAAAC AATCC AGTCA CTTAC CCTAT GCCTA GGAGC CATCA CCACA CTATT 451 CACAG CAATC TGCGC ACTCA CTCAA AACGA TATCA AAAAA ATCAT TGCTT 501 TCTCC ACCTC CAGCC AACTA GGCCT GATAA TCGTA ACCAT CGGTA TCAAT 551 CAACC CTACC TAGCA TTCCT CCACA TCTGC ACTCA CGCAT TCTTC AAAGC 601 TATAC TATTT ATATG TTCCG GATCC ATTAT CCACA GCCTA AATGA CGAGC 651 AAGAT ATCCG AAAAA TAGGC GGACT ATTTA ATGCA ATACC CTTCA CCACC 701 ACATC CCTAA TTATT GGCAG CCTTG CACTC ACCGG AATTC CTTTC CTCAC 751 AGGCT TCTAT TCCAA AGACC TCATC ATCGA AACCG CCAAC ACATC GTACA 801 CCAAC GCCTG AGCCC TACTA ATAAC TCTCA TTGCC ACATC CCTCA CAGCT 851 GTCTA CAGCA CCCGA ATCAT CTTCT TTGCA CTCCT AGGGC AACCC CGCTT 901 CCTCC CTCTG ACCTC AATCA ACGAA AATAA CCCCT TTCTA ATTAA CTCCA 951 TCAAA CGCCT CTTAA TTGGC AGCAT TTTTG CCGGA TTCTT CATCT CCAAC | | |

| Variant ORIGIN | B | |
|---|---|---|
| 1 TTTCC AACTG TTCAT CGGAT GGGAG GGAGT AGGCA TCATG TCATT CTTAC 51 TAATC GGATG ATGAC ACGGC CGAAC AGATG CCAAC ACCGC GGCCC TTCAA 101 GCAAT CCTTT ATAAC CGCAT CGGGG ATATC GGCTT CATCA TGGCC ATAGC 151 CTGAT TCCTA TTCAA CACCA ACACA TGAGA CCTCC AACAA ATCTT CATAC 201 TCGAC CCCAA CCTCA CCAAC CTCCC GCTCC TAGGC CTCCT CCTAG CCGCA 251 ACCGG CAAAT CCGCT CAATT TGGAC TCCAC CCATG ACTTC CTTCA GCCAT 301 AGAGG GCCCT ACACC AGTCT CAGCC CTACT CCACT CCAGC ACAAT AGTTG 351 TAGCA GGCGT CTTCC TGCTA ATCCG CTTCC ATCCA CTAAT AGAAA ACAAC 401 AAAAC AATCC AGTCA CTTAC CCTAT GCCTA GGAGC CATCA CCACA CTATT 451 CACAG CAATC TGCGC ACTCA CTCAA AACGA TATCA AAAAA ATTAT TGCTT 501 TCTCC ACCTC CAGCC AACTA GGCCT GATAA TCGTA ACCAT CGGTA TCAAT 551 CAACC CTACC TAGCA TTCCT CCACA TCTGC ACTCA CGCAT TCTTC AAAGC 601 TATAC TATTT ATATG TTCCG GATCC ATTAT CCACA GCCTA AATGA CGAGC 651 AAGAT ATCCG AAAAA TAGGC GGACT ATTTA ATGCA ATACC CTTCA CCACC 701 ACATC TCTAA TTATT GGCAG CCTTG CACTC ACCGG AATTC CTTTC CTCAC 751 AGGCT TCTAC TCCAA AGACC TCATC ATCGA AACCG CCAAC ACATC GTACA 801 CCAAC GCCTG AGCCC TACTA ATAAC TCTCA TTGCC ACATC CCTCA CAGCT 851 GTCTA CAGCA CCCGA ATCAT CTTCT TTGCA CTCCT AGGGC AACCC CGCTT 901 CCTCC CTGTG ACCTC AATCA ACGAA AAAAA CCCCT TTCTA ATTAA CTCCA 951 TCAAA CGCCT CTTAA TTGGC AGCAT TTTTG CCGGA TTCTT CATCT CCAAC | | |

| Variant ORIGIN | C | |
|---|---|---|
| 1 TTTCC AACTG TTCAT CGGAT GGGAG GGAGT AGGCA TCATG TCATT CTTAC 51 TAATC GGATG ATGAT ACGGC CGAAC AGATG CCAAC ACCGC GGCAC TTCAA 101 GCAAT CCTTT ATAAC CGCAT CGGGG ATATC GGCTT CATCA TGGCC ATAGC 151 CTGAT TCCTA TTCAA CACCA ACACA TGAGA CCTCC AACAA ATCTT CATAC 201 TCGAC CCCAA CCTTA CCAAC CTCCC GCTCC TAGGC CTCCT CCTAG CCGCA 251 ACTGG CAAAT CCGCT CAATT TGGAC TCCAC CCATG ACTTC CTTCA GCCAT 301 AGAGG GCCCT ACACC AGTCT CAGCC CTACT CCACT CCAGC ACAAT AGTTG 351 TAGCA GGCGT CTTCC TGCTA ATCCG CTTCC ATCCA CTAAT AGAAA ACAAC 401 AAAAC AATCC AGTCA CTTAC CCTAT GCCTA GGAGC CATCA CCACA CTATT 451 CACAG CAATC TGCGC ACTCA CTCAA AACGA TATCA AAAAA ATCAT TGCTT 501 TCTCC ACCTC CAGCC AACTA GGCCT GATAA TCGTA ACCAT GGGTA TCAAT 551 CAACC CTACC TAGCA TTCCT CCACA TTTGC ACTCA CGCAT TCTTC AAAGC 601 TATAC TATTT ATATG TTCCG GATCC ATTAT CCACA GCCTA AATGA CGAGC 651 AAGAT ATCCG AAAAA TAGGC GGACT ATTTA ATGCA ATACC CTTCA CCACC 701 ACATC TCTAA TTATT GGCAG CCTTG CACTC ACCGG AATTC CTTTC CTCAC 751 AGGCT TCTAC TCCAA AGACC TCATC ATCGA AACCG CCAAC ACATC GTACA | | |
| 801 CCAAC GCCTG AGCCC TACTA ATAAC TCTCA TTGCC ACATC CCTCA CAGCT 851 GTCTA CAGTA CCCGA ATCAT CTTCT TTGCA CTCCT AGGGC AACCC CGCTT 901 CCTCC CTCTG ACCTC AATCA ACGAA AATAG CCCCT TTCTA ATTAA CTCCA 951 TCAAA CGCCT CTTAA TTGGC AGCAT TTTTG CCGGA TTCTT CATCT CCAAC | | |

| VARIANT ORIGIN | D | |
|---|---|---|
| 1 TTTCC AACTG TTCAT CGGAT GGGAG GGAGT AGGCA TCATG TCATT CTTAC 51 TAATC GGATG ATGAT ACGGC CGAAC AGATG CCAAC ACCGC GGCCC TTCAA 101 GCAAT CCTTT ATAAC CGCAT CGGGG ATATC GGCTT CATCA TGGCC ATAGC 151 CTGAT TCCTA TTCAA CACCA ACACA TGAGA CCTCC AACAA ATCTT CATAC 201 TCGAC CCCAA CCTTA CCAAC CTCCC GCTCC TAGGC CTCCT CCTAG CCGCA 251 ACTGG CAAAT CCGCT CAATT TGGAC TCCAC CCATG ACTTC CTTCA GCCAT 301 AGAGG GCCCT ACACC AGTCT CAGCC CTACT CCACT CCAGC ACAAT AGTTG 351 TAGCA GGCGT CTTCC TGCTA ATCCG CTTCC ATCCA CTAAT AGAAA ACAAC 401 AAAAC AATCC AGTCA CTTAC CCTAT GCCTA GGAGC CATCA CCACA CTATT 451 CACAG CAATC TGCGC ACTCA CTCAA AACGA TATCA AAAAA ATCAT TGCTT 501 TCTCC ACCTC CAGCC AACTA GGCCT GATAA TCGTA ACCAT CGGTA TCAAT 551 CAACC CTACC TAGCA TTCCT CCACA TCTGC ACTCA CGCAT TCTTC AAAGC 601 TATAC TATTT ATATG TTCCG GATCC ATTAT CCACA GCCTA AATGA CGAGC 651 AAGAT ATCCG AAAAA TAGGC GGACT ATTTA ATGCA ATACC CTTCA CCACC 701 ACATC CCTAA TTATT GGCAG CCTTG CACTC ACCGG AATTC CTTTC CTCAC 751 AGGCT TCTAT TCCAA AGACC TCATC ATCGA AACCG CCAAC ACATC GTACA 801 CCAAC GCCTG AGCCC TACTA ATAAC TCTCA TTGCC ACATC CCTCA CAGCT 851 GTCTA CAGCA CCCGA ATCAT CTTCT TTGCA CTCCT AGGGC AACAC CGCTT 901 CCTCC CTCTG ACCTC AATCA ACGAA AATAA CCCCT TTCTA ATTAA CTCCA 951 TCAAA CGCCT CTTAA TTGGC AGCAT TTTTG CCGGA TTCTT CATCT CCAAC | | |

| Variant ORIGIN | E | |
|---|---|---|
| 1 TTTCC AACTG TTCAT CGGAT GGGAG GGAGT AGGCA TCATG TCATT CTTAC 51 TAATC GGATG ATGAT ACGGC CGAAC AGATG CCAAC ACCGC GGCAC TTCAA 101 GCAAT CCTTT ATAAC CGCAT CGGGG ATATC GGCTT CATCA TGGCC ATAGC 151 CTGAT TGCTA TTCAA CACCA ACACA TGAGA CCTCC AACAA ATCTT CATAC 201 TCGAC CCCAA CCTTT CCAAC CTCCC GCTCC TAGGC CTCCT CCTAG CCGCA 251 ACTGG CAAAT CCGCT CAATT TGGAC TCCAC CCATG ACTTC CTTCA GCCAT 301 AGAGG GCCCT ACACC AGTCT CAGCC CTACT CCACT CCAGC ACAAT AGTTG 351 TAGCA GGCGT CTTCC TGCTA ATCCG CTTCC ATCCA CTAAT AGAAA ACAAC 401 AAAAC AATCC AGTCG CTTAC CCTAT GCCTA GGAGC CTTCA CCACA CTATT 451 CACAG CAATC TGCGC ACTCA CTCAA AACGA TATCA AAAAA ATCAT TGCTT 501 TCTCC ACCTC CAGCC AACTA GGCCT GATAA TCGTA ACCAT GGGTA TCAAT 551 CAACC CTACC TAGCA TTCCT CCACA TTTGC ACTCA CGCAT TCTTC AAAGC 601 TATAC TATTT ATATG TTCCG GATCC ATTAT CCACA GCCTA AATGA CGAGC 651 AAGAT ATCCG AAAAA TAGGC GGACT ATTTA ATGCA ATACC CTTCA CCCCC 701 ACATC TCTAA TTATT GGCAG CCTTG CACTC ACCGG AATTC CTTTC CTCAC 751 AGGCT TCTAC TCCAA AGACC TCATC ATCGA AACCG CCAAC ACATC GTACA 801 CCAAC GCCTG AGCCC TACTA ATAAC TCTCA TTGCC ACATC CCTCA CAGCT 851 GTCTA CAGTA CGCGA ATCAT CTTCT TTGCA CTCCT AGGGG AACCC CGCTT 901 CCTCC CTCTA ACCTC AATCA ACGAA AATAG CCCCT TTCTA ATTAA CTCCA 951 TCAAA CGCCT CTTAA TTGGC AGCAT TTTTG CCGGA TTCTT CATCT CCAAC | | |

| Variant ORIGIN | F | |
|---|---|---|
| 1 TTTCC AACTG TTCAT CGGAT GGGAG GGAGT AGGCA TCATG TCATT CTTAC | | |
| 51 TAATC GGATG ATGAT ACGGC CGAAC AGATG CCAAC ACCGC GGCCC TTCAA 101 GCAAT CCTTT ATAAC CGCAT CGGGG ATATC GGCTT CATCA TGGCC ATAGC 151 CTGAT TCCTA TTCAA CACCA ACACA TGAGA CCTCC AACAA ATCTT CATAC 201 TCGAC CCCAA CCTTA CCAAC CTCCC GCTCC TAGGC CTCCT CCTAG CCGCA 251 ACTGG CAAAT CCGCT CAATT TGGAC TCCAC CCATG ACTTC CTTCA GCCAT 301 AGAGG GCCCT ACACC AGTCT CAGCC CTACT CCACT CCAGC ACAAT AGTTG 351 TAGCA GGCGT CTTCC TGCTA ATCCG CTTCC ATCCA CTAAT AGAAA ACAAC 401 AAAAC AATCC AGTCA CTTAC CCTAT GCCTA GGAGC CATCA CCACA CTATT 451 CACAG CAATC TGCGC ACTCA CTCAA AACGA TATTA AAAAA ATCAT TGCTT 501 TCTCC ACCTC CAGCC AACTA GGCCT GATAA TCGTA ACCAT CGGTA TCAAT 551 CAACC CTACC TAGCA TTCCT CCACA TCTGC ACTCA CGCAT TCTTC AAAGC 601 TATAC TATTT ATATG TTCCG GATCC ATTAT CCACA GCCTA AATGA CGAGC 651 AAGAT ATCCG AAAAA TAGGC GGACT ATTTA ATGCA ATACC CTTCA CCACC 701 ACATC TCTAA TTATT GGCAG CCTTG CACTC ACCGG AATTC CTTTC CTCAC 751 AGGCT TCTAC TCCAA AGACC TCATC ATCGA AACCG CCAAC ACATC GTACA 801 CCAAC GCCTG AGCCC TACTA ATAAC TCTCA TTGCC ACATC CCTCA CAGCT 851 GTCTA CAGCA CCCGA ATCAT CTTCT TTGCA CTCCT AGGGC AACCC CGCTT 901 CCTCC CTCTA ACCTC AATCA ACGAA AATAA CCCCT GTCTA ATTAA CTCCA 951 TCAAA CGCCT CTTAA TTGGC AGCAT TTTTG CCGGA TTCTT CATCT CCAAC | | |

| **LOCUS** | **ND2** | **993 bp** |
|---|---|---|
| Variant ORIGIN | A | |
| 1 CCCTT ATCTT CACAA CTATT CTAAT AACAG TTCTT CTAGG AACTA TAATC 51 GTTAT AATAA GCTCA CACTG ACTAA TGATC TGAAT CGGAT TTGAA ATAAA 101 TCTAC TAGCC ATTAT CCCCA TCCTA ATAAA AAAGT ACAAT CCCCG AACCA 151 TAGAA GCCTC CACCA AATAT TTTCT AACCC AAGCC ACCGC ATCAA TACTC 201 CTCAT AATAG CGATC ATCAT TAACC TCATA CACTC AGGCC AATGA ACAAT 251 CACAA AAGTC TTCAA CCCCA CAGCG TCCAT CATTA TAACT TCAGC TCTCG 301 CCATA AAACT TGGAC TCACA CCATT CCACT TCTGA GTACC CGAAG TCACA 351 CAGGG CATCT CATTA ACATC AGGTC TCATC CTACT TACAT GACAA AAACT 401 AGCCC CAATA TCAAT CCTAT ATCAA ATCTC ACCCT CAATT AACCT AAATA 451 TCTTA TTAAC TATAG CCGTA CTGTC AATCC TAGTA GGAGG CTGAG GCGGT 501 CTCAA CCAAA CCCAA CTACG AAAAA TCATA GCATA CTCGT CAATC GCGCA 551 TATAG GATGA ATAAC AGCTG TCCTA GTATA TAACC CAACA CTGAC AATAC 601 TAAAC ATATT AATTT ACATT ATAAT AACAC TCACA ATATT CATAC TATTT 651 ATCCA CAGCT CCTCT ACTAC AACAC TATCA CTCTC CCACA CATGA AACAA 701 AACAC CTCTA ACCAC TACAC TAATC CTAAT TACCT TACTA TCCAT AGGAG 751 GCCTC CCCCC ACTAT CAGGA TTCAT ACCCA AATGA ATAAT CATTC AAGAG 801 CTCAC CAAAA ATAGC AGCAT CATCC TCCCC ACACT AATAG CCATT ATAGC 851 ACTAC TCAAC CTCTA CTTCT ACATA CGACT AACCT ATTCC ACCTC ACTGA 901 CCATA TTCCC ATCCA CAAAC AACAT AAAAA TAAAA TGACA ATTCG AAACC 951 AAACG AATTA CTCTC TTACC CCCGT TAATT GTTAT ATCCT CCC | | |

| Variant ORIGIN | B | |
|---|---|---|
| 1 CCCTT ATCTT CACAA CTATT CTAAT AACAG TTCTT CTAGG AACTA TAATC 51 GTTAT AATAA GCTCA CACTG ACTAA TAATC TGAAT CGGAT TTGAA ATAAA 101 TCTAC TAGCC ATTAT CCCCA TCCTA ATAAA AAAGT ACAAT CCCCG AACCA 151 TAGAA GCCTC CACCA AATAT TTTCT AACCC AAGCC ACCGC ATCAA TACTC 201 CTCAT AATAG CGATC ATCAT TAACC TCATA CACTC AGGTC AATGA ACAAT 251 CACAA AAGTC TTCAA CCCCA CAGCG TCCAT CATTA TAACT TCAGC TCTCG 301 CCATA AAACT TGGAC TCACA CCATT CCACT TCTGA GTACC CGAAG TCACA 351 CAGGG CATCT CATTA ACATC AGGTC TCATC CTACT TACAT GACAA AAACT | | |
| 401 AGCCC CAATA TCAAT CCTAT ATCAA ATCTC ACCCT CAATT AACCT AAATA 451 TCTTA TTAAC TATAG CCGTA CTGTC AATCC TAGTA GGAGG CTGAG GCGGT 501 CTCAA CCAAA CCCAA CTACG AAAAA TCATA GCATA CTCGT CAATC GCGCA 551 TATAG GATGA ATAAC AGCTG TCCTA GTATA TAACC CAACA CTAAC AATAC 601 TAAAC ATATT AATTT ACATT ATAAT AACAC TCACA ATATT CATAC TATTT 651 ATCCA CAGCT CCTCT ACTAC AACAC TATCA CTCTC CCACA CATGA AACAA 701 AACAC CTCTA ACCAC TACAC TAATC CTAAT TACCT TACTA TCCAT AGGAG 751 GCCTC CCCCC ACTAT CAGGA TTCAT ACCCA AATGA ATAAT CATTC AAGAG 801 CTCAC CAAAA ATAGC AGCAT CATCC TCCCC ACACT AATAG CCATT ATAGC 851 ACTAC TCAAC CTCTA CTTCT ACATA CGACT AACCT ACTCC ACCTC ACTGA 901 CCATA TTCCC ATCCA CAAAC AACAT AAAAA TAAAA TGACA ATTCG AAACC 951 AAACG AATTA CTCTC TTACC CCCGT TAATT GTTAT ATCCT CCC | | |

| Variant ORIGIN | C | |
|---|---|---|
| 1 CCCTT ATCTT CACAA CTATT CTAAT AACAG TTCTT CTAGG AACTA TAATC 51 GTTAT AATAA GCTCA CACTG ACTAA TAATC TGAAT CGGAT TTGAA ATAAA 101 TCTAC TAGCC ATTAT CCCCA TCCTA ATAAA AAAGT ACAAT CCCCG AACCA 151 TAGAA GCCTC CACCA AATAT TTTCT AACCC AAGCC ACCGC ATCAA TACTC 201 CTCAT AATAG CGATC ATCAT TAACC TCATA CACTC AGGCC AATGA ACAAT 251 CACAA AAGTC TTCAA CCCCA CAGCG TCCAT CATTA TAACT TCAGC TCTCG 301 CCATA AAACT TGGAC TCACA CCATT CCACT TCTGA GTACC CGAAG TCACA 351 CAGGG CATCT CATTA ACATC AGGTC TCATC CTACT TACAT GACAA AAACT 401 AGCCC CAATA TCAAT CCTAT ATCAA ATCTC ACCCT CAATT AACCT AAATA 451 TCTTA TTAAC TATAG CCGTA CTGTC AATCC TAGTG GGAGG CTGAG GCGGT 501 CTCAA CCAAA CCCAA CTACG AAAAA TCATA GCATA CTCGT CAATC GCGCA 551 TATAG GATGA ATAAC AGCTG TCCTA GTATA TAACC CAACA CTGAC AATAC 601 TAAAC ATATT AATTT ACATT ATAAT AACAC TCACA ATATT CATAC TATTT 651 ATCCA CAGCT CTTCT ACTAC AACAC TATCA CTCTC CCACA CATGA AACAA 701 AACAC CTCTA ACCAC TACAC TAATC CTAAT TACCT TACTA TCCAT AGGAG 751 GCCTC CCCCC ACTAT CAGGA TTCAT ACCCA AATGA ATAAT CATTC AAGAG 801 CTCAC CAAAA ATAGC AGCAT CATCC TCCCC ACACT AATAG CCATT ATAGC 851 ACTAC TCAAC CTCTA CTTCT ACATA CGACT AACCT ATTCC ACCTC ACTGA 901 CCATA TTCCC ATCCA CAAAC AACAT AAAAA TAAAA TGACA ATTCG AAACC 951 AAACG AATTA CTCTC TTACC CCCGT TAATT GTTAT ATCCT CCC | | |

| **LOCUS** | **ND4L** | **277bp** |
|---|---|---|
| Variant ORIGIN | A | |
| 1 ATATC TTCCT AGCAT TCACA GTATC CCTCG TAGGC CTACT AATGT ACCGA 51 TCCCA CCTAA TATCC TCACT CCTAT GCCTA GAAGG AATAA TACTA TCACT 101 ATTCG TCATA GCAAC CATAA TAGTC CTAAA CACCC ACTTC ACACT AGCTA 151 GTATA GTACC TATCA TCTTA CTAGT ATTCG CTGCC TGCGA AGCAG CTCTA 201 GGATT ATCCC TACTA GTCAT AGTCT CCAAT ACTTA TGGAG TAGAC CACGT 251 ACAAA ACCTT AACCT CCTCC AATGC TA | | |

| Variant ORIGIN | B | |
|---|---|---|
| 1 ATATC TTCCT AGCAT TCACA GTATC CCTCG TAGGC CTGCT AGTGG ACCGA 51 TTCCA CCTAT ATTCT TCACT CTTAT GCCTA GAAGG AATAA TACTA TCACT 101 ATTCG TCATA GCAAC CATAA TAGTC CTAAA CACCC ACTTC ACACT AGCTA 151 GCATA ATACC TATCA TCTTA CTAGT ATTTG CTGCC TGCGA AGCAG CTCTA | | |
| 201 GGATT ATCCC TACTA GTCAT AGTCT CCAAT ACTTA TGGAG TAGAC CACGT 251 ACAAA ACCTT AACCT CCTCC AATGC TA | | |

### Mitochondrial type assignation

Based on the combination of the variation of the 13 gene loci, it was possible to assign 17 mitochondrial types as summarised in Table 1.

In common with previous studies (Marklund *et al.,* 1995; Hill *et al.,* 2002), D-loop variation was also extensive, however, while the present study identified a total of 17 genetic types, there were only 14 D-loop variants found. In these previous studies this specific amplicon of the D-loop and other nested zones within the same region have been used as a purported definitive means of assessing mtDNA variability. Importantly, however, we have found that its isolated use is unreliable in assessing mtDNA variability, making it unsuitable as a basis for assessing potential performance correlations or clarification of female family origins. Variation in the protein encoding genes is not fully described by that of the, supposedly more hypervariable, D-loop. For example possession of the same D-loop variant by horses from differing female origins did not guarantee that they did not vary at other loci. In particular, as can be seen from the present results, the Types II, III and XV, carrying the same D-loop variant B, could be distinguished through variation at the *ATPase* 6 and 16S *rRNA genes.* Similarly, horses carrying the same functional gene variants sometimes possessed different D-loop versions. This included types VII and VIII and types XV and XVII respectively.

The relative abundance of genetic types was a result of the occurrence of between 2 and 6 variants at each variable locus. The thoroughbred breed has been selected for over 200 years for racecourse performance and these observations support a reduced likelihood of there having been coincidental selection of any specific mtDNA type or gene variant. However, different horses perform to varying degrees of competence over a variety of distances and age groups that may require specific physiological attributes. Additionally, the occurrence of handicap races where poorer quality horses carry less weight is likely to impinge upon strict selection and culling procedures. Commercial aspects of breeding and racing also dictate selection policies in that breeders are just as likely to breed from poor or better mares if they have already invested financially in their racing careers.

As 5 of the genes examined showed no variation, it was possible that their consistency had been due to continuing subconscious selection. To test this, analyses were also carried out on a range of non-thoroughbred horses. These horses were chosen to represent breeds that have not been selected for racing performance. They could be categorised into two groups: old breeds that existed prior to the formal instigation of the thoroughbred which consisted of 21 Polish Tarpans, 23 Mongolian riding horses and 22 Peruvian Paso horses; and 56 Irish draught horses, a relatively new breed arising as a result of cross breeding with the thoroughbred. Using this comparison it is possible to gain an indication of whether specific selection for mtDNA types or gene variants has occurred for the thoroughbred in relation to other breeds.

The genes that did not exhibit variation in the thoroughbred did not show any variation in the other breeds tested, indicating absence of selection for racing performance at these loci. However, there was considerable variability at the loci also found to be variable in the thoroughbred and in the frequency of occurrence of genetic types. Most of the types found in these breeds were also observed for the thoroughbred, although minorities were peculiar to specific breeds. Although the differences in type distribution between the thoroughbred and the other breeds are significant, it is not possible to state whether these are as a result of selection or due to restriction of maternal line variation in the founder general populations.

From analysis of the pedigrees of the thoroughbreds in this sample, 29 different female lines were categorised. An indicator of the power of the analysis possible according to the invention is shown by the fact that it was possible to ascertain, through use of the greater discerning power of combined loci typing, that past inaccuracies in the recording of thoroughbred stud book registration are far more widespread than indicated through use of D-loop analysis alone (Hill, et al., 2002). In the lines analysed, 26 incorrect sub-branches were identified by virtue of anomalous mtDNA inheritance and these were spread over 17 of the 29 lines.

By virtue of the comprehensive sample size used in the present study, it has been possible to provide near saturation coverage of all correct and incorrect female branches in existence. In particular, this has also given the valuable ability to extrapolate from pedigree data the mtDNA type of 98.9% and 97.25% of the current thoroughbred populations of the United Kingdom (UK) and United States Of America USA), respectively and to make corrections when necessary.

### EXAMPLES 2 - 5

To illustrate the analysis enabled by the present invention, mtDNA type assignment provided a mechanism enabling investigation of the relative performance merits of the genetic types and to study their potential selection over time, for different stamina / distance requirements and for performance optima at different racing ages. Using a combination of the General Stud Book and the American Stud Book the pedigrees of randomly selected batches of 1,000 thoroughbred horses known to have been racing or breeding in the years 1903, 1953 and 2003 in the UK and USA were identified. Where possible, mtDNA types were assigned to each of them.

Separate data sets were also collected randomly from Raceform (2003) records, which detail the annual racing careers of all thoroughbreds running in the UK. From this the pedigrees of batches of 1,000 horses shown to have been racing at two years old (2yo), three years old (3yo) and at older ages (3yo+) respectively in 2003 were identified. Where possible, mtDNA haplotypes were assigned to each.

This has allowed subsequent comparisons and correlations of racing data with genetic types to be made taking into account the true frequency distribution of each type in races contested by horses at different age groups and in different countries. It also gives an indication of whether selection may have taken place for racing at different ages, over time or in the different national regions.

### EXAMPLE 2. POSITIVE AND NEGATIVE SELECTION OF GENETIC TYPES OVER TIME AND GEOGRAPHICAL REGION - POPULATION DESCRIPTIONS.

Table 2 shows the percentage distribution of types over time in the UK and USA and at different ages in the UK. Correlations describing these trends are shown at the bottom of the table.

THERE ARE SIGNIFICANT DIFFERENCES IN THE DISTRIBUTION OF TYPES OVER TIME IN THE USA AND UK. In both countries this is the case and the differences in percentage distribution of the types are more pronounced during the period between 1903 and 1953. This indicates that there is a subconscious, yet real selection of certain types over the years. The latter fifty year period, between 1953 and 2003, shows a non-significant change in distribution which makes it viable to compare the effectiveness of different types in their racing abilities with standard model populations based on the same frequency distribution of types for this period in both countries.

THERE ARE SIGNIFICANT DIFFERENCES IN THE CURRENT DISTRIBUTION OF TYPES BETWEEN THE USA AND UK. These differences are real and indicate that a different base population is required for comparing racing success of the types in these countries and other geographical areas. To draw conclusions about the relative racing success of each type their success must be viewed in parallel with the percentage occurrence of each type in the general population. It is clear from this result that this relative success of each type should be based on a different population distribution in each case.

THERE ARE SIGNIFICANT DIFFERENCES IN THE DISTRIBUTION OF TYPES AT DIFFERENT AGES IN THE UK. There are clear differences in the distribution of the different types at different ages in the UK. In assessing the relative racing merits of each type it must be borne in mind that their distribution varies in the general population according to age. In effect, there are three subpopulations based on age, which need to be adjusted for when considering the racing success of the types at different ages. This provides justification for separate population delineation of types at different ages.

The apparent co-incidental selection of favoured types over time and type frequency variation exhibited for groups of horses running at different ages provides initial evidence for a possible role of mtDNA haplotype in contribution to racing performance.

### EXAMPLE 3. ASSOCIATION OF HAPLOTYPE AND GENE VARIIANTS WITH ABSOLUTE RACING ABILITY AND STAMINA POTENTIAL.

To test the above example further, mtDNA type data were assigned for the winners of major UK horse races between 1954 and 2003. All of these races have a relatively high prize value and are described by racing's governing bodies as Group races that are categorised as 1,2 and 3 in order of importance. In such higher value races, where entry fees are correspondingly high and the kudos of winning is greater, horses are likely to be racing to their true potential. A consistent test of stamina or speed should, therefore, be applied. The races were chosen on the basis that they have been run consistently over the same distance and age group during the years covered by the study. In total, 74 races were scored incorporating 21 races for 3yo, 19 for 2yo and 34 for older horses. At least 40 years of data was collected for each race.

The mtDNA type of horses winning each race for up to 50 years was estimated and from this the percentage winning success of the types in all of the races was calculated. By dividing the percentage of wins by the percentage occurrence of each type in the general population for any specific age group it was then possible to assign a success index (Race Index - RI). This was assigned for each race and for the total across all races within each age group.

Furthermore, Race Indices were also calculated for each variant of all of the genes studied and found in the winners of these races. Within each age group, the percentage of wins by each type was also calculated for races grouped into the same distance, e.g. all of the 8f 3yo races were grouped together. By dividing each percentage by the occurrence of each type in the general population at each age group another index was derived and was termed Distance Index (DI). This index can be used to assess the relative success of each type over distances at each age rather than in individual races.

From Raceform data collected over the past 10 years, the pedigrees of horses entered for each of the primary races during this period were examined. mtDNA types were assigned to these horses and were the percentages of each type entered into each race were calculated. By dividing the winning percentages of each type of races grouped according to distance (as with the DI) it was possible to assign an Entries Index (EI) to each type. This gives an indication of whether types are providing a greater return of wins than expected from the number of times each is entered into races.

### EXAMPLE 3A. ABSOLUTE MERIT OR PERFORMANCE

THERE ARE SIGNIFICANT DIFFERENCES IN THE ABSOLUTE OVERALL RACING EFFECTIVENESS OF HORSES OF DIFFERING GENETIC TYPE WITHIN EACH AGE GROUP. Probabilities and Least Significant Differences (LSDs) for variation in racecourse effectiveness of the different genetic types at varying ages are provided in Table 3. It is clear from this that there is a hierarchical order of merit of racecourse effectiveness, which is significant. This order is also dependent upon the age group and changes at different ages of racing. An ANOVA analysis gave highly significant differences between the groups (p<0.01). The resulting summary of LSD analysis can be seen in Table 3.

THERE ARE SIGNIFICANT DIFFERENCES IN THE ABSOLUTE OVERALL EFFECTIVENESS OF HORSES CARRYING DIFFERING GENE VARIANTS WITHIN EACH AGE GROUP. Similarly, horses carrying specific variants or alleles of each gene examined also vary significantly in their relative racing merit at different ages and there is also an age effect, which changes the order of racing effectiveness of the variants. For example, at 3yo, when the ANOVA and LSD analysis is performed for RI of animals carrying differing gene variants, the variant B of CCO1 carried by type IV is significantly smaller than the other groups. In another instance, in the case of Variant A at ATPase6, there is no adverse affect in terms of a reduction in RI. As a result, specific combinations of alleles carried by certain horses are also likely to affect their absolute racing potential.

Similarly, amongst the 2yo group, LSD and Tukey-Kramer HSD analysis of RI of horses varying at the ATP6 locus showed significantly positive effects of possession of variant D (found in Type XII). Similar analysis in Nd4 showed that variant D is significantly better and variant E is significantly worse.

### EXAMPLE 3B. STAMINA BIAS

THERE ARE VARIABLE STAMINA BIASES FOR GENETIC TYPES WITHIN EACH AGE GROUP. Table 4 shows the Race Indices for each genetic type over a number of races of differing length. The RIs are provided for races grouped into the same distance category and the number of races included in each group is provided in the No. races column. RIs can also be measured for individual races. There are clear differences between the types in their RIs at different distances. Some types tend to become more effective at longer distances and others at shorter. The patterns of these stamina preferences vary according to the age group in question.

Table 5 shows statistical evaluation results of the effect of race distance on RI for each type. A number of strong correlations are observed at all ages. In numerous instances there are also significant linear regressions provided, illustrating a real effect of distance of race on the relative racing success of horses from many of the genetic type groupings. The effects are more pronounced at 3yo where the majority of races are run with horses carrying level weights. Use of Quadratic regressions also indicates that some genetic types have significant performance peaks in the middle distance ranges at all ages.

At 2yo the stamina range of races run is less than at the other ages groups. This reduces the chances of obtaining stronger correlations. There is limited scope to enter horses of extended stamina potential into appropriate races, as races of longer distance (8 furlongs plus) do not exist for 2yo horses in the UK. Thus, the perception that horses of middle distance capability (8-10f) at 3yo should run over middle distances (6-7f) at 2yo is misleading.

At the older age group the effect of genetic type on race distance potential may also be affected by the allocation of different racing weight penalties to horses of varying age. In Weight For Age races (WFA), older horses are penalised. Therefore, the enhanced racing ability of genetic types, which are more effective at older ages, may be obscured. This may be true both in terms of absolute racing effectiveness and in stamina bias.

In certain instances it is viable to group types carrying the same or similar functional gene variants but with different D loop sequences, as there is likelihood that these will have similar stamina aptitudes controlled by functional mitochondrial genes of the respiratory complexes. These are referred to as COM 1, 2 and 3 and represent combinations of VII/VIII, VI/XV/XVII and VI/XV/XVI/XVII respectively.

THERE ARE STAMINA BIASES FOR DIFFERENT VARIANTS WITHIN EACH AGE GROUP. Table 6 shows the RIs for each variant at each of the gene loci examined. This is shown for races grouped according to distance and over each age group but the same effect is seen when races are considered individually. Table 7 provides statistical evaluation and again shows that the distance potential of horses carrying specific gene variants may vary. For example, the negative correlation in RI vs. race distance occurring in horses carrying the C variant at the ATPase6 gene is highly significant. This may, in part explain the negative correlation also seen for the genetic haplotypes carrying this variant (e.g. III and XV). Combinations of specific alleles enhance the strength of the correlations and linear and quadratic regressions relating genetic type to stamina potential.

Table 8 shows how mean Race Index of each genetic type changes with age and the relevant correlations describing this are also presented. It is clear that racing effectiveness is age dependent and many of the correlations are significant. Similar effects can be seen for the relationship between specific gene variants and stamina potential over time. Table 9 presents information relating to the change in stamina bias of the genetic types at the different age groups. It is clear that some genetic types have their stamina biases at 2yo reinforced at older ages, some level off and others are reversed.

### EXAMPLE 4. APPLICATION OF HAPLOTYPE, VARIANT AND RACE INDEX DATA TO BREEDING PROGRAMMES.

As these genes are involved in energy release, this has enormous implications for Thoroughbred performance and selection. From comparisons of pedigree and mtDNA data, according to the present invention it has been possible to observe performance trends within the pedigrees of successful horses. Some mtDNA types appear to be more strongly associated with certain stamina attributes. This is important because horses are frequently bred with specific stamina objectives in mind.

Another striking observation is that certain stallions produce better progeny when bred with mares of specific mtDNA type. In horses it is likely that complementation exists between respiratory complex genes carried on the chromosomes and those carried on the mtDNA. Positive complementation results in superior energy release characteristics, which affect performance.

On this basis, by using a test based on variation at all of these variable loci (not just d loop) or pedigree to determine the DNA variant carried by a horse, whether it is correct and correlation with our databases for pedigree and performance data, it is possible to help to form the basis of breeding recommendations. An example of this is provided in Table 10. In this instance, stallions have been recommended for a mare based on the success of these stallions when crossed with mares of the same genetic type in the past. Similarly, stallions of specific RI and DI (genetic stamina profile) may be more suitably mated with mares of similar scores in order to co-ordinate matings between animals of similar racing aptitude. A score is calculated for the stallions suitability on a percentage basis.

Tests can be suitably modified for inclusion into a micro-array or chip based system based on identified SNP variation within these mitochondrial genes.

The mtDNA variation in related non-Thoroughbred breeds indicates which mtDNA types may have been selected for performance. This will to help identify the respiratory complex genes with the greatest influence and provide a genetic test capable of distinguishing the most efficient chromosomal complementing genes for the mtDNA variant carried by a specific horse. A microarray based system can be used to rapidly identify important variants of mtDNA complex genes carried by a horse.

### EXAMPLE 5. APPLICATION OF HAPLOTYPE, VARIANT AND RACE INDEX DATA TO GAMBLING (BETTING, LAYING AND ODDS SETTING).

It is clear that some of the genetic/performance data patterns have many applications to the betting industry.

In this example, it is shown that it is viable to use Race Indices, haplotype variation and variant variation as complementary tools for the assessment of horses' racing ability and stamina attributes. Table 11 illustrates the results of such application for a number of races (for which specific RIs have been calculated for each type). Bets are shown as representing the potential to lay horses (bet against them winning or achieving a place), bet on them to win and bet on them to place. In 11 out of 13 cases a successful outcome (positive P/L) resulted. The RIs for each of the horses for that particular race, based on historical data, is indicated. All of the horses bet on to win had good RIs and that which was laid carried a poor score for that particular race.

The information from this type of analysis would have a major impact in the setting of odds for prominent races and in the large ante-post race market. The betting turnover for the Epsom Derby, alone, amounts to £50 m. If the supplementary genetic data presented by this invention can be used to improve the odds on a favourite by as little as half a point in favour of a bookmaker then a significant increase in profit may be achieved.

Database products according to the invention would be particularly useful to the betting industry prior to the start of a racing season or during ante-post or 'big race' odds composition stages.

### EXAMPLE 6. ASSESSMENT OF HAPLOTYPE RACING ATTRIBUTES FROM EXAMINATION OF PEDIGREES, 3YO HORSE RACING DATA AND HAPLOTYPE ASSIGNMENT.

As time progresses and more DNA samples are collected it is possible to assign haplotypes to previously unassigned pedigree lines. As a result, this section incorporates updated data where 28 incorrect family sub-branches were identified by and were spread over 19 of the 33 lines. Additionally, as a result of laboratory recording idiosyncrasies, the classification of types I, II, III and IV has been reversed for this example. There is, therefore a shift in the RI values and correlations/regressions of genetic types in relation to previous sections.

The mitochondrial DNA (mtDNA) molecule, carrying genes encoding for respiratory chain enzymes, is a candidate for demonstrating associations between genotype and athletic performance in mammalian species. In humans, variation at 7 mitochondrial loci has been implicated in influencing fitness and performance characteristics (Dionne et al., 1993; Perusse et al., 2003). Although thoroughbred horses are selected for racing ability, there have not been any previous reported associations between genotypes and racecourse performance. The multi-factorial nature of the inheritance of racing ability is an obvious complicating factor. However, mitochondrial gene variation may represent a measurable component contributing to performance variability. Previous population studies restricted to examinations of relatively small samples of thoroughbreds (Ishida et al., 1994; Marklund et al., 1995; Hill et al., 2002) have shown the existence of D-Ioop variation. Importantly, we have observed that there is also independent and extensive functional mitochondrial gene variation in the current thoroughbred racehorse population and that significant associations exist between mtDNA haplotype, defined by functional genes, and aspects of racing performance.

Although scientifically unproven, a traditional belief of many thoroughbred breeders is that the mother (dam), rather than the father (sire), contributes more strongly to a horse's racing stamina (Leicester, 1983). Varying degrees of importance have also been attached to differing perceived endurance and performance abilities of certain female breeding lines. Historically, efforts have been made to categorise maternal lines in order of importance defined by the racing quality of horses descended from them (Lowe, 1898; Bobinshi, 1953), though this information is now considered aged and incomplete.

Naturally, dated pedigree-based comparisons describing family origins and performance characteristics do not take into account shared maternal genetic origins prior to formal studbook recordings (Weatherby and Sons, 1791) and overlook potential common genotype-mediated performance characteristics. Additionally, they do not provide any indication of functional gene similarities and associated performance trends in different female lines. However, these observations support the postulation that variation between horses in the mitochondrial protein-encoding genes may affect relative performance characteristics.

DNA samples from 1000 thoroughbred horses were selected. Primers were designed for polymerase chain reaction (PCR) of each of their protein-encoding genes together with the 16S and 12S RNA genes and the D-loop region (Table 12).

Single Strand Conformational Polymorphism (SSCP) analysis was used to genetically characterise the PCR amplicons and representatives of all SSCP variants were selected for DNA sequencing. DNA from representatives of all known thoroughbred female lines represented by our sample was also sequenced. Allelic variation was found in 10 functional genes, including the 16S and 12S RNA genes (Table 13). Horses were assigned as one of 17 genetic 'haplotypes' based on the combined variation at each of these genes and in the D-loop.

The genes coding for NADH hydrogenase 1, 3 and 6, ATP synthase 8 and cytochrome oxidase 3 showed no allelic variation. It was possible that, at these loci, there had been continuing co-incidental selection of specific alleles for enhanced racing ability. We were able to test this by haplotyping non-thoroughbred horses, not selected for racing. There was also an absence of variation within these breeds, indicating the unlikelihood of selection of specific variants at these loci.

Although we identified a total of 17 haplotypes, there were only 14 D-loop variants found. Previously (Marklund et al., 1995; Hill et al., 2002), SSCP and sequencing analysis of this D-loop region has been used as a definitive means of assessing thoroughbred mtDNA variability. However, protein encoding gene variation is not fully described by this, supposedly more hypervariable, region. This limits its use as an isolated basis for assessing potential mtDNA/racing performance correlations or clarification of studbook family origins. Possession of the same D-loop variant by horses from differing female origins did not guarantee that they did not vary at other loci. In particular, the Types II, III and XV, carrying the same D-loop variant 'B', could be distinguished through variation at the MTATP6 and MTND5 loci. Similarly, horses carrying the same functional gene variants sometimes possessed different D-loop versions. This included haplotypes VII and VIII and haplotypes XV. and XVII. Therefore, classification of mtDNA solely by D-loop could lead to misinterpretation of functional gene variation in up to 34.8% of the racing population.

From analysis of the pedigrees of the horses in our sample we determined that 33 different female lines were categorised. Importantly, we were able to ascertain, through use of the greater discerning power of combined loci typing, that past inaccuracies in the recording of thoroughbred studbook registration are far more widespread than indicated through use of D-loop analysis alone (Hill et al., 2002). In the lines analysed, 28 incorrect sub-branches were identified by virtue of anomalous mtDNA inheritance and these were spread over 19 of the 33 lines.

The extent of the sample size provided near saturation coverage of all correct and incorrect female branches exhibited by the current population of horses racing in the UK (98.9%). It has given the ability to extrapolate from pedigree data the mtDNA haplotype of all horses belonging to these lines and to make corrections when necessary. This provides a mechanism whereby it is possible to investigate relative performance merits of haplotypes and to study effectiveness at different racing distances.

The abundance of genetic types was contributed to by the occurrence of between 2 and 6 variants at each variable, functional gene locus. The thoroughbred has been bred for over 200 years for racecourse performance and these observations might suggest a reduced likelihood of coincidental selection of any specific mtDNA haplotype or gene variant. However, different horses perform to varying degrees of competence in races run over a variety of distances and age groups that may require specific physiological attributes. Variability at mtDNA loci may contribute to differential stamina potential.

Using pedigree information, it was possible to assign mtDNA haplotype data for all winners of major UK three years old (3yo) horse races run between 1954 and 2003. From this we calculated the percentage winning success of each haplotype for each race during this period. Dividing the percentage of wins by the percentage occurrence of each haplotype in the general 3yo population provided a success index (Race Index - RI) for each race. Race Indices were also calculated for each gene variant carried by the winners of these races. 3yo races provide the most informative and measurable data compared with other age group races. They represent a range of racing distance, they are mainly single sex and are also run generally with each animal carrying equal weight.

Correlation coefficients, relating RI and race distance for haplotypes occurring at more than 2% of the 3yo population (Table 14), showed that for 5 of them (accounting for 51.6% of the total 3yo population) there was a significant relationship (Figure 1). Haplotypes II, XV and XVI showed negative correlations, significant at P<0.05. Two haplotypes were positively correlated. Type XI exhibited a correlation coefficient significant at P<0.05, whereas Type IV was highly significant (P<0.001).

For each of these haplotypes, ANOVAs were applied using their average RI grouped according to race distance. At two extremes of distance scored (1400-1600m and 2400m, the one race at 2800m was omitted) there were significant differences between haplotypes in average RI. Haplotypes for which RI negatively correlated with distance scored significantly higher at the shorter distances (XVI - 1.42; II-1.27; XV - 1.15; IV - 0.84; XI - 0.24; P<0.001). However, at the longer distance, this was reversed and the positively correlated haplotypes scored higher (IV - 1.34; XI - 1.25; XVI - 1.15; II - 0.85; XV - 0.54; P=0.002). This indicates that there is a true order of racing merit amongst these haplotypes, which changes depending on the distance of race under consideration (Figure 1). It is not possible to state whether these effects are due to direct gene influence or to differential responses to training.

Similarly, without extended physiological studies, it is difficult to draw firm conclusions about the relative contribution of the different loci. However, there are some important points to note regarding candidates for further investigation. The haplotypes with negative RI/stamina correlations are similar in terms of the component variants at the different loci. Their functional gene variants are the same, except Type II, which differs only at the MTND5 locus. Two other types (VI and XVII) also share the same functional gene variants. Notably, they differ from the other haplotypes through common possession of the 'C' variant at the MTATP6 locus. Combination of data from haplotypes carrying this variant gives rise to a strong negative correlation (r = -0.7070) (Table 15) with a significance of P<0.001. Type III, which has a moderate positive RI/distance correlation (r = 0.2891), also shares the same variants as the bulk of this combined group but differs in carrying a unique variant, 'B' at the MTATP6 locus. For this study, the unsuitability of restricted D-loop haplotyping is further emphasised as the significant stamina differences existing between haplotypes II and XV (negative correlation) and Haplotype III that have the same D-loop variant, would be overlooked.

The two haploptypes exhibiting positive RI/distance correlation differ from each other at most loci and have no positions at which they commonly differ from less correlated types. Type I has unique variants at the MTND2 and MTND4 locus. At all other loci it is the same as Type IX, which has a slight negative correlation of RI vs. distance. Type XI has a unique allele at the MTND5 locus but is otherwise similar to haplotypes showing a range of negative and positive, non-significant, correlations and regressions. These observations, however, may be of importance as, in humans, variants of the genes coding for MTND2, MTND4 and MTND5 have been shown to differentially affect VO2 max, initial fitness and responses to training (Dionne et al., 1993; Perusse et al., 2003; Andreu, et al., 1999) and a mutation in MTND4 has also affected exercise intolerance.

Many thoroughbred breeders base breeding strategies on pedigree assessments, attempting to co-ordinate the perceived stamina capabilities of parents and ancestors in the pedigree to achieve specific stamina objectives in the progeny. Over 50% of the 3yo thoroughbred population belong to haplotypes that exhibit a significant leaning towards success at particular stamina extremes. These observations lend support to there being an important female component contributing to stamina optima which could be taken into account when planning thoroughbred breeding strategies.

### Methods.

### DNA Samples.

DNA samples, which had been extracted from white blood cells using a range of non-phenol based techniques, were selected from our collection. The DNA had been stored in 10mM Tris HCL/1mM EDTA buffer at -20°C.

One thousand thoroughbred samples were analysed, representing 33 thoroughbred globally occurring female families. Reference to the thoroughbred General Stud Book showed that the majority of currently existing European female lines, traceable to original stud book members, were represented within this selection, thereby heightening the chances of achieving coverage of all available mtDNA variation within the breed.

Non-thoroughbreds could be categorised into two groups: old breeds that existed prior to the formal instigation of the thoroughbred which consisted of 21 Polish Tarpans, 23 Mongolian riding horses and 22 Peruvian Paso horses; a relatively new breed arising as a result of cross breeding with the thoroughbred consisting of 56 Irish Draught horses.

Primers and PCR amplification conditions.

Primer design (Table 12) was based on a previously reported sequence (Xu & Arnason, 1994). Primer positions were chosen to provide as much coverage as possible at each locus without diminishing the effectiveness of SSCP analysis. 17 loci were covered, including those of 13 protein-encoding genes, the D-Loop and 12S rRNA. Two primer pairs were used to provide amplification of the large 16S rRNA locus in order to describe variation. For haplotyping purposes, variation at these two positions was combined

PCR reactions were carried out in 0.2 ml reaction tubes in a volume of 20*µ*l containing reaction buffer (10mM Tris-HCL, pH 8.0, 50mM KCL, 3.75 mM MgCl), 0.2 mM dNTPs, 0.5 pmol *µ*l-1 of each primer, 0.025 U *µ*l-1 Taq polymerase and 5 ng *µ*l-1 DNA template.

Thirty amplification cycles were carried out for each primer pair. These varied in annealment and extension conditions (Table 12) but commonly started with 1 cycle of denaturation at 94°C for 1 minute and constant 30 s denaturation phases at 94°C.

### SSCP analysis.

SSCP analysis was carried out by denaturing PCR products by heating at 95°C for 10 min, followed by immediate placement on ice. Products were assessed for polymorphisms by running the denatured DNA on 10 % polyacrylamide, 5 % glycerol, 0.5xTBE gels as described by Kukita et al (1997). The gels were run at 0.6 V h-1 × 100bp and stained by silver staining (Caetano-Anolles et al., 1991). Clear SSCP patterns were produced for all detectable variants. Subject to confirmation by sequencing, newly detected SSCP variants were named alphabetically in order of detection.

### DNA Sequencing.

Sequencing was performed using an Applied Biosystems ABI PRISM 377 sequencer/genotyper using the recommended protocol and labelling. PCR amplification products were cleaned for sequencing using Charges witch PCR clean-up kits (DNA Research Instruments). 2ul of PCR product was added to 18ul of water and sequencing of the regions was carried out using the forward and reverse primers in both directions. Sequences were analysed using BioEdit (Hall, 1999). Partial CDS sequences for these loci have been submitted to GenBank.

### Pedigree, haplotype and racing data.

The pedigree records kept for the thoroughbred are unparalleled in detail by any other breed or domestic species. The population is genetically finite and a restricted studbook has been employed since 1791.

Using the General Stud Book we identified the pedigrees of randomly selected batches of 1,000 thoroughbred horses known to have been racing or breeding in the years 1953 and 2003 in the UK. Where possible, we assigned mtDNA types to each of them. There was no discernible shift in haplotype distribution in the UK during this period.

Separate data sets were also collected randomly from Raceform records which detail the annual racing careers of all thoroughbreds running in the UK. From this we identified the pedigrees of 1,000 horses shown to have been racing at three years old (3yo) in 2003. We were able to assign mtDNA haplotypes to 99.8% of the horses and to determine the distribution of identifiable haplotypes in the current population at 3yo.

European racing governing bodies describe the selected races as 'Group' races. In these high value races, where the entry fees and the kudos of winning are correspondingly higher, horses are likely to be racing to their true potential. A consistent test of stamina or speed should, therefore, be applied. The races were chosen on the basis that they have been run consistently over the same distance and age group during the years covered by the study. In total, 21 different races for 3yo horses were scored over at least 44 years (n = 1035). These races were the: Fred Darling, Greenham, Nell Gwyn and Jersey Stakes, all 1400m; 1,000 Guineas, 2,000 Guineas, St. James's Palace, Coronation and Craven Stakes, all 1600m; Sandown Classic, Musidora and Dante Stakes, all 2000m; Lingfield Derby Trial, Derby, Oaks, Ribblesdale, King Edward VII, Great Voltigeur, Chester Vase and Gordon Stakes, all 2400m; St.Leger Stakes, 2800m.

### References

Andrade, M.A. Ed. (2003) Bioinformatics and Genomes: Current Perspectives. Horizon Scientific Press.
Andreu, A.L. et al. (1999). Exercise intolerance due to a nonsense mutation in the mtDNA ND4 gene. Ann. Neurol. 45, 820-823
Bobinski, K. (1953). Family Tables of Racehorses. Zamoyski, London.
Bowling, A. T., Del Valle, A. & Bowling, M. (2000). A pedigree-based study of mitochondrial D-loop DNA sequence variation among Arabian horses. Anim. Genet. 31, 1-7
Caetano-Anolles, G. & Gresshoff, P.M. (1994) Staining nucleic acids with silver. An alternative to radioisotopic and fluorescent labeling. Promega Notes 45, 13pp.
Dionne, F. T., Turcotte, L., Thibault, M. C., Boulay, M. R., Skinner, J. S. & Bouchard, C. (1993). Mitochondrial DNA sequence polymorphism, VO2max, and response to endurance training. Med. Sci. Sports. Exerc. 25(7):766-774
Dunham, I. Ed. (2003). Genome Mapping and Sequencing. Horizon Scientific Press.
Foster, P. (2003). To err is human. Annals of Human Genetics 67, 2-4
Hall, T.A. (1999). BioEdit: a user-friendly biological sequence alignment editor and analysis program for Windows 95/98/NT. Nucl. Acids. Symp. Ser. 41:95-98
Hickman. J. (2003). New tests on the market to aid breeders with mating plans. Racing Post. November 18th, 2003.
Hill, E. W., Bradley, D. G, Al-Barody, M, Ertugrul, O., Splan, R. K., Zakharov, I. & Cunningham, E. P. (2002) History and integrity of thoroughbred dam lines revealed in equine mtDNA variation. Animal Genetics 33, 287-294.
Hodgson, D. R., Rose, R. J., Dimauro, J & Allen, J. R. (1986). Effects of training on muscle composition in horses. Am. J. Vet. Res. 47, 12-15
Ingman, M., Kaessmann, H., Paabo, S. & Gyllensten, U. (2000). Mitochondrial genome variation and the origin of modern humans. Nature. 408, 708-713
Ishida, N., Hasegawa, T., Takeda, K., Sakagami, M., Onishi, A., Inumaru, S., Komatsu M. & Mukoyama H. (1994). Polymorphic sequence in the D-loop region of equine mitochondrial DNA. Anim Genet. 25(4):215-221
Jansen, T., Foster, P., Levine, M. A., Oelke, H., Hurles, M., Renfrew, C., Weber, J. & Olek, K. (2002). Mitochondrial DNA and the origins of the domestic horse. PNAS, vol. 99, no. 16,10905-10910
Kavar, T., Habe, F., Brem, G. & Dovc, P. (1999). Mitochondrial D-loop sequence variation among the 16 maternal lines of the Lipizzan horse breed. Anim. Genet. 30(6), 423-430
Kukita, Y., Tahira, T., Sommer, S. S. & Hayashi, K. (1997). SSCP analysis of long DNA fragments in low pH Gel. Human mutation 10:400-407.
Leicester, C. Bloodstock Breeding (2nd Edition). Revised by H.Wright. J.A.Allen and Co. Ltd. London (1983).
Lowe, C.B. (1898). Breeding racehorses by the figure system. Edited by W. Allison. William R. Jenkins, Veterinary Publisher, New York,
Marklund, S., Chaudhary, R., Marklund, L., Sandberg, K. & Andersson, (1995) L. Extensive mtDNA diversity in horses revealed by PCR-SSCP analysis. Anim. Genet. 26(3), 193-196.
Parsons, T. J. & Coble, M. D. (2001) Increasing the forensic discrimination of mitochondrial DNA testing through analysis of the entire mitochondrial DNA genome. Croat. Med. J. 42(3), 304-309. Perusse, L., Gagnon, J., Province, M. A., Rao, D. C., Wilmore, J. H., Leon, A. S., Bouchard, C. & Skinner, J. S. (2001). Familial aggregation of submaximal aerobic performance in the Heritage Family study. Med. Sci. Sports Exerc. 33(4):597-604
Perusse, L. et al. (2003). The human gene map for physical performance and health related fitness phenotypes: the 2002 update. Med. Sci. Sports Exerc. 35, 1248-1264.
Raceform (2003). Flat annual for 2003. Raceform Ltd. Newbury, Berkshire, UK.
Sambrook, J., Fritsch, E.F. & Maniatis, T. (1989) Molecular cloning: A laboratory manual. Cold Spring Harbor Laboratory Press. New York.
Schaefer, B.C. Ed (1997) Gene Cloning and Analysis: Current Innovations. Horizon Scientific Press.
Vila C., Leonard, J. A., Gotherstrom, A., Marklund, S., Sandberg, K., Liden, K., Wayne, R. K & Ellegren, H. (2001). Widespread origins of domestic horse lineages. Science 291, 474-477**.**
Weatherby and Sons (1791). An introduction to the general stud book. Weatherby and Sons (London).
Wood. G. (2002). The man to see about a horse. The Guardian. February 7th, 2002.
Xu X. & Arnason V. (1994). The complete mitochondrial DNA sequence of the horse, Equus caballus: extensive heteroplasmy of the control region. Gene 148: 357-362
Yang, Y. H., Kim, K. I., Cothran, E. G. & Flannery, A. R. (2002). Genetic diversity of Cheju horses (Equus caballus) determined by using mitochondrial DNA D-loop polymorphism. Biochem. Genet. 40(5-6): 175-86

**Table 1. Haplotypes and gene variants**

| **LOCI** | | | | | | | | | | | |
|---|---|---|---|---|---|---|---|---|---|---|---|
| | **D loop** | **Cyto B** | **ATP 6** | **ND2** | **ND4** | **ND4L** | **ND5** | **CcO1** | **CcO2** | **16S** | **12S** |
| **TYPE** | | | | | | | | | | | |
| **I** | A | A | A | C | A | A | F | D | A | A | A |
| **II** | B | B | B | A | B | A | A | A | B | A | A |
| **III** | B | B | C | A | B | A | D | A | B | A | A |
| **IV** | D | A | A | A | C | B | B | B | D | A | A |
| **V** | C | A | A | A | C | B | C | C | D | A | A |
| **VI** | F | B | C | A | B | A | A | A | B | A | A |
| **VII** | K | A | A | A | C | B | C | C | C | A | B |
| **VIII** | E | A | A | A | C | B | C | C | C | A | B |
| **IX** | L | A | A | A | D | A | F | D | A | A | A |
| **X** | M | A | E | A | C | B | A | A | D | A | C |
| **XI** | I | A | A | A | C | B | E | C | D | A | A |
| **XII** | H | C | D | B | C | B | A | D | D | C | A |
| **XIII** | C | A | A | A | E | B | C | C | D | A | A |
| **XIV** | J | A | A | A | C | B | A | A | D | A | C |
| **XV** | B | B | C | A | B | A | A | A | B | A | A |
| **XVI** | G | B | C | A | B | A | A | A | B | B | A |
| **XVII** | N | B | C | A | B | A | A | A | B | A | A. |
| **mplicon** | 440 | 1110 | 671 | 993 | 1001 | 277 | 1000 | 1000 | 668 | * | 931 |
| **length** | | | | | | | | | *16S-1 825bp+16S-2 811bp | | |
| **No. ariants.** | 14 | 3 | 5 | 3 | 5 | 2 | 6 | 4 | 4 | 3 | 3 |

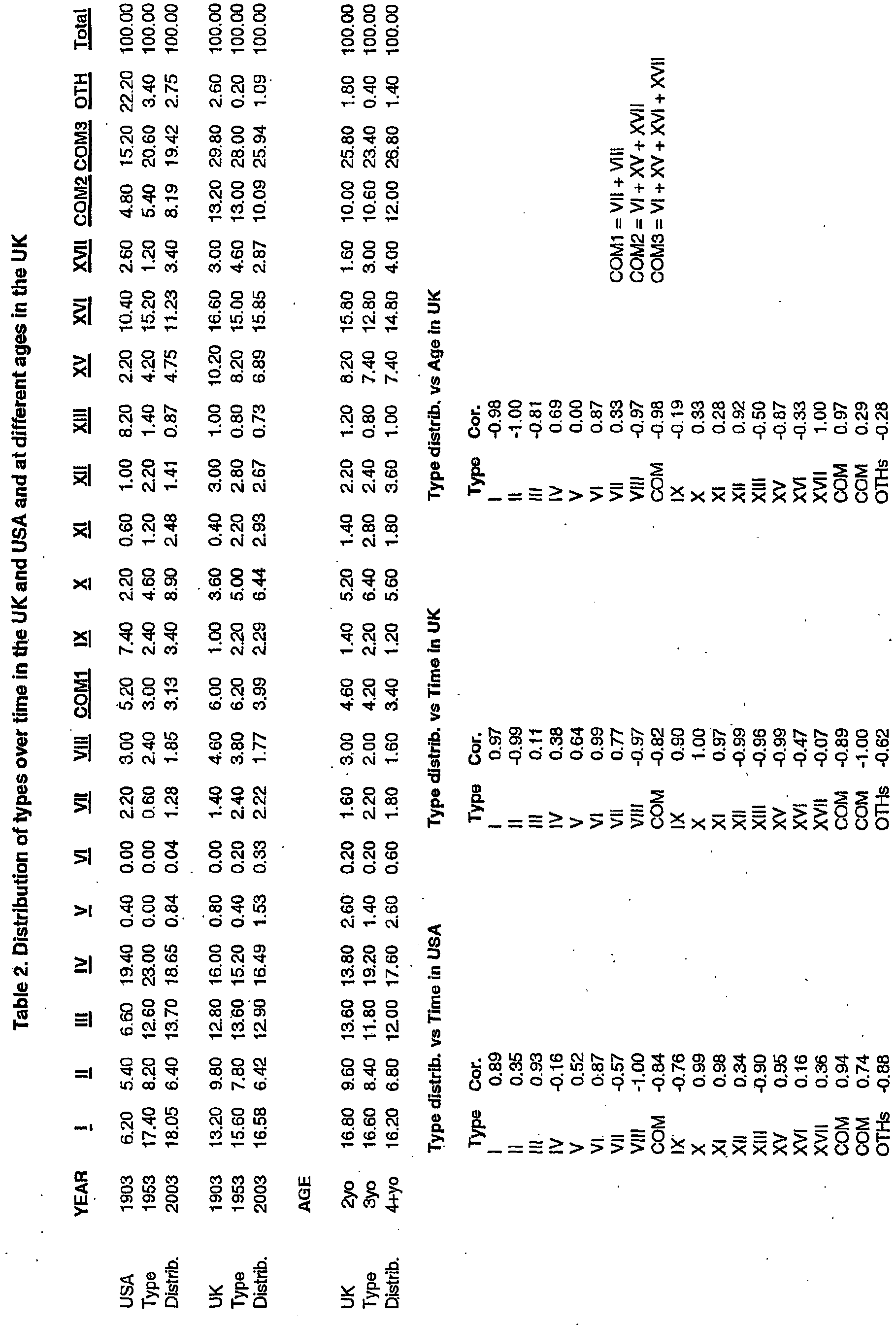

**Table 3. LSDs for RI for each haplotype at various ages**

| **Mitochondrial Type** | **2 years old races** | **3 years old races** | **3+ years old races** |
|---|---|---|---|
| I | 1.0808 cdef | 1.1970 abc | 1.0805 cd |
| II | 0.6067 def | 1.0195 abc | 1.3158 c |
| III | 0.9787 cdef | 1.0762 abc | 1.0848 cd |
| IV | 0.9461 cdef | 0.7388 bcd | 0.9130 cd |
| V | 0.4453 ef | 1.4949 a | 0.5625 d |
| VI | 1.5789 abed | 0.7797 bc | 1.0353 cd |
| VII | 1.3486 abcde | 1.2452 ab | 0.9396 cd |
| VIII | 1.0570 cdef | 1.1947 abc | 2.1921 b |
| IX | 2.3308 a | 0.9725 abc | 2.7607 a |
| X | 0.8375 cdef | 0.5855 cd | 0.7027 d |
| XI | 0.5639 ef | 0.7507 bcd | 0.7023 d |
| XII | 1.7982 abc | 1.2970 ab | 0.8078 cd |
| XIII | 0.0877 f | 0.1315 d | 1.0035 cd |
| XV | 1.1473 bcde | 0.8796 abc | 1.0539 cd |
| XVI | 1.0794 cdef | 1.1672 abc | 0.9687 cd |
| XVII | 2.1244 ab | 1.0710 abc | 0.8522 cd |
| ANOVAp_value | >0.001 | 0.0109 | >0.001 |

| | | | |
|---|---|---|---|
| Mitochondrial types not connected with the same letter are significantly different. | | | |

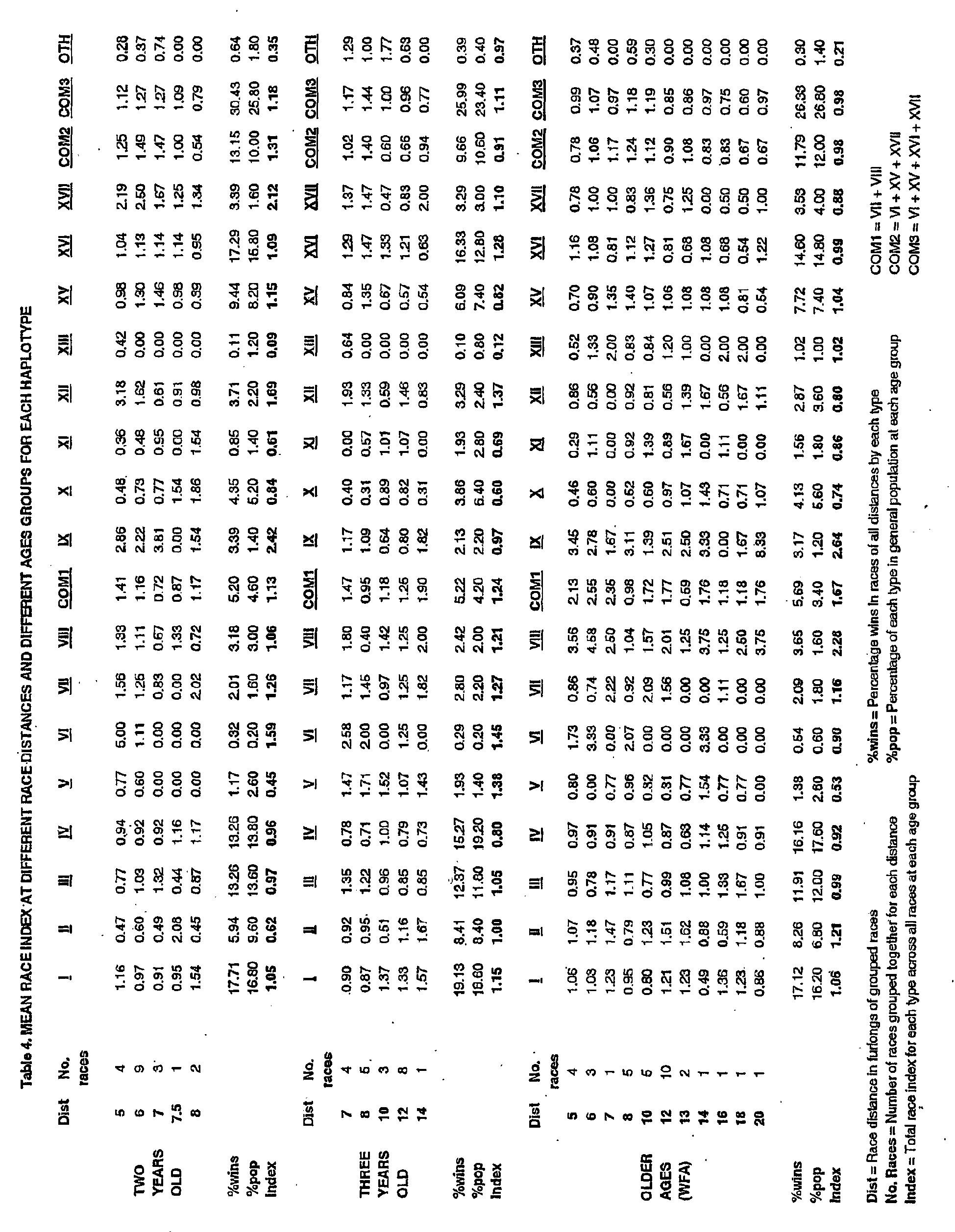

**Table 5. Correlations and Regressions of Race Index vs distance for different types**

| **2YO** | | | | **3YO** | | | | **OLDER** | | | |
|---|---|---|---|---|---|---|---|---|---|---|---|
| **TYPE** | **COR.** | **REG.** | **SIG.** | **TYPE** | **COR.** | **REG.** | **SIG.** | **TYPE** | **COR.** | **REG.** | **SIG.** |
| I | 0.1677 | | | I | 0.6684 | 0.0009 | p<0.001 | I | 0.0926 | | |
| II | 0.2017 | | | II | 0.2891 | | | II | 0.0708 | | |
| III | 0.0814 | | | III | -0.4467 | 0.0424 | p<0.05 | III | 0.2059 | | |
| IV | 0.2653 | | | IV | 0.0747 | | | IV | -0.0132 | | |
| V | -0.3615 | | | V | -0.1329 | | | V | -0.0563 | | |
| VI | -0.3167 | | | VI | -0.1830 | | | VI | -0.3823 | 0.0257 | p<0.05 |
| VII | -0.0562 | | | VII | 0.0234 | | | VII | -0.0983 | | |
| VIII | -0.2557 | | | VIII | 0.0870 | | | VIII | -0.1973 | | |
| COM1 | -0.2558 | | | COM1 | 0.0787 | | | COM1 | -0.2415 | | |
| IX | -0.1812 | | | IX | -0.0809 | | | IX | 0.0432 | | |
| X | 0.6014 | 0.0064 | p<0.01 | X | 0.3278 | | | X | 0.3637 | 0.0345 | p<0.05 |
| XI | 0.2667 | | | XI | 0.3724 | 0.0965 | p<0.10 | XI | -0.0487 | | |
| XII | -0.4961 | 0.0307 | p<0.05 | XII | -0.1136 | | | XII | 0.1876 | | |
| XIII | -0.3159 | | | XIII | -0.2943 | | | XIII | 0.0674 | | |
| XV | -0.1464 | | | XV | -0.4355 | 0.0485 | p<0.05 | XV | -0.0254 | | |
| XVI | 0.0024 | | | XVl | -0.4339 | 0.0494 | p<0.05 | XVI | -0.2857 | | |
| XVII | -0.1798 | | | XVII | -0.2113 | | | XVII | -0.0912 | | |
| COM2 | -0.2759 | | | COM2 | -0.5396 | 0.0116 | p<0.05 | COM2 | -0.4073 | 0.0168 | p<0.05 |
| COM3 | -0.1994 | | | COM3 | -0.6574 | 0.0012 | p<0.01 | COM3 | -0.3241 | 0.0615 | p<0.10 |
| OTHERS | -0.1960 | | | OTHERS | -0.1647 | | | OTHERS | -0.3275 | 0.0587 | p<0.10 |

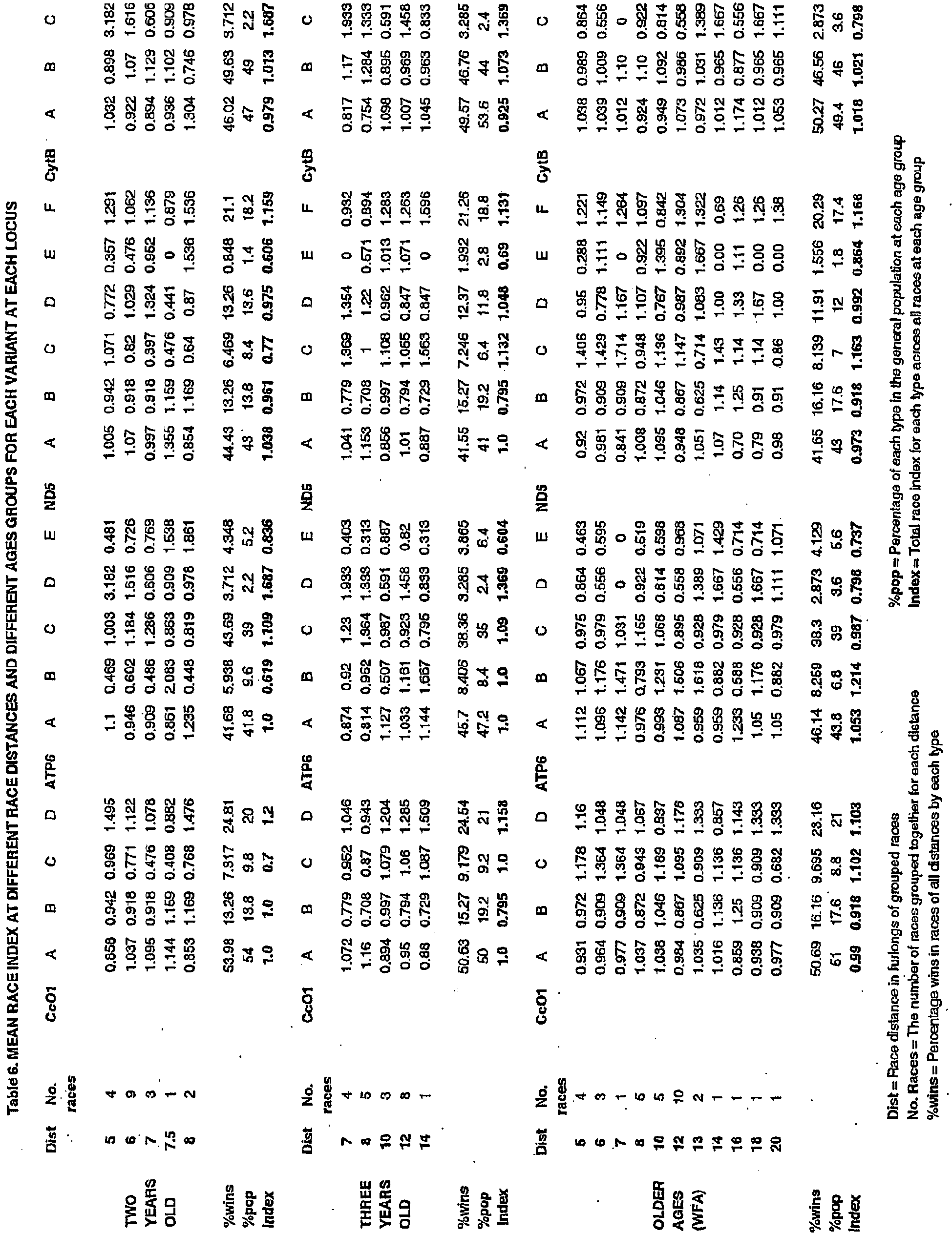

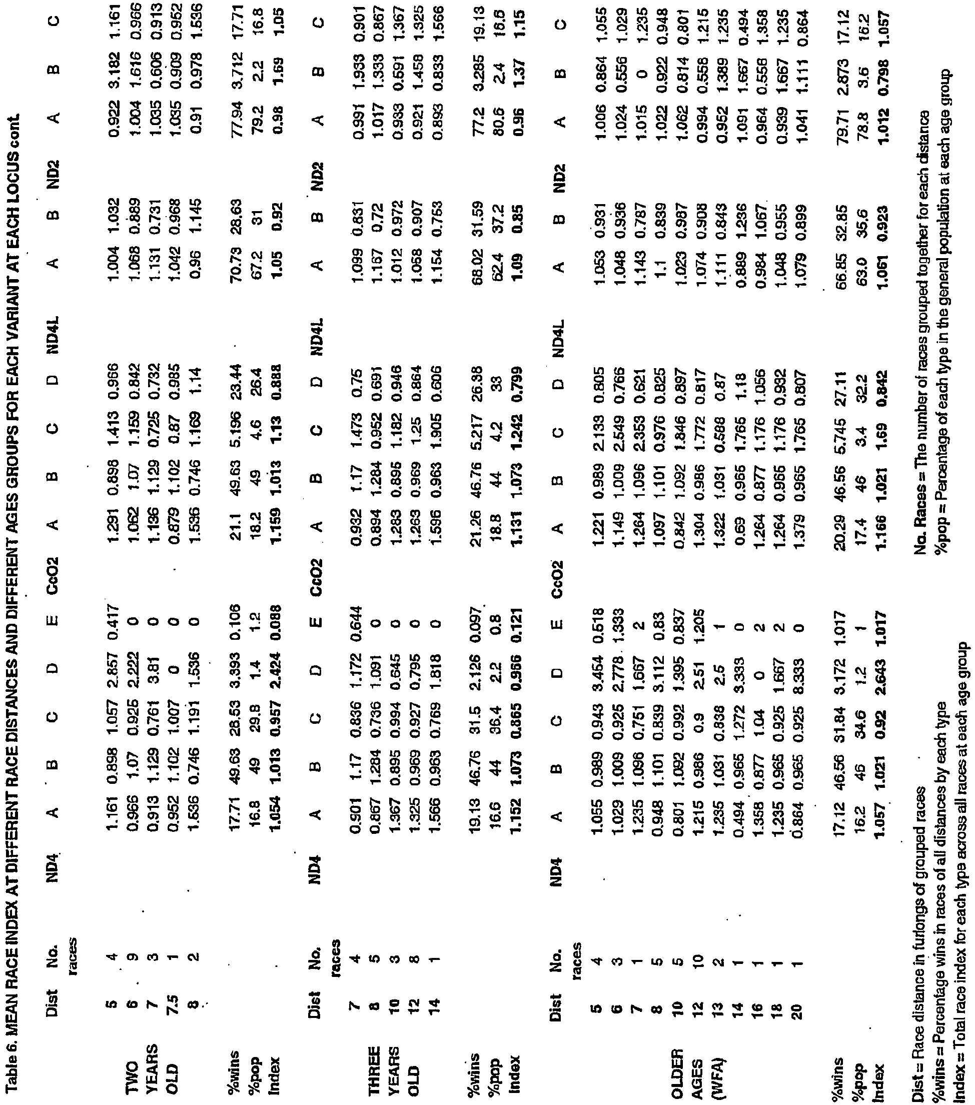

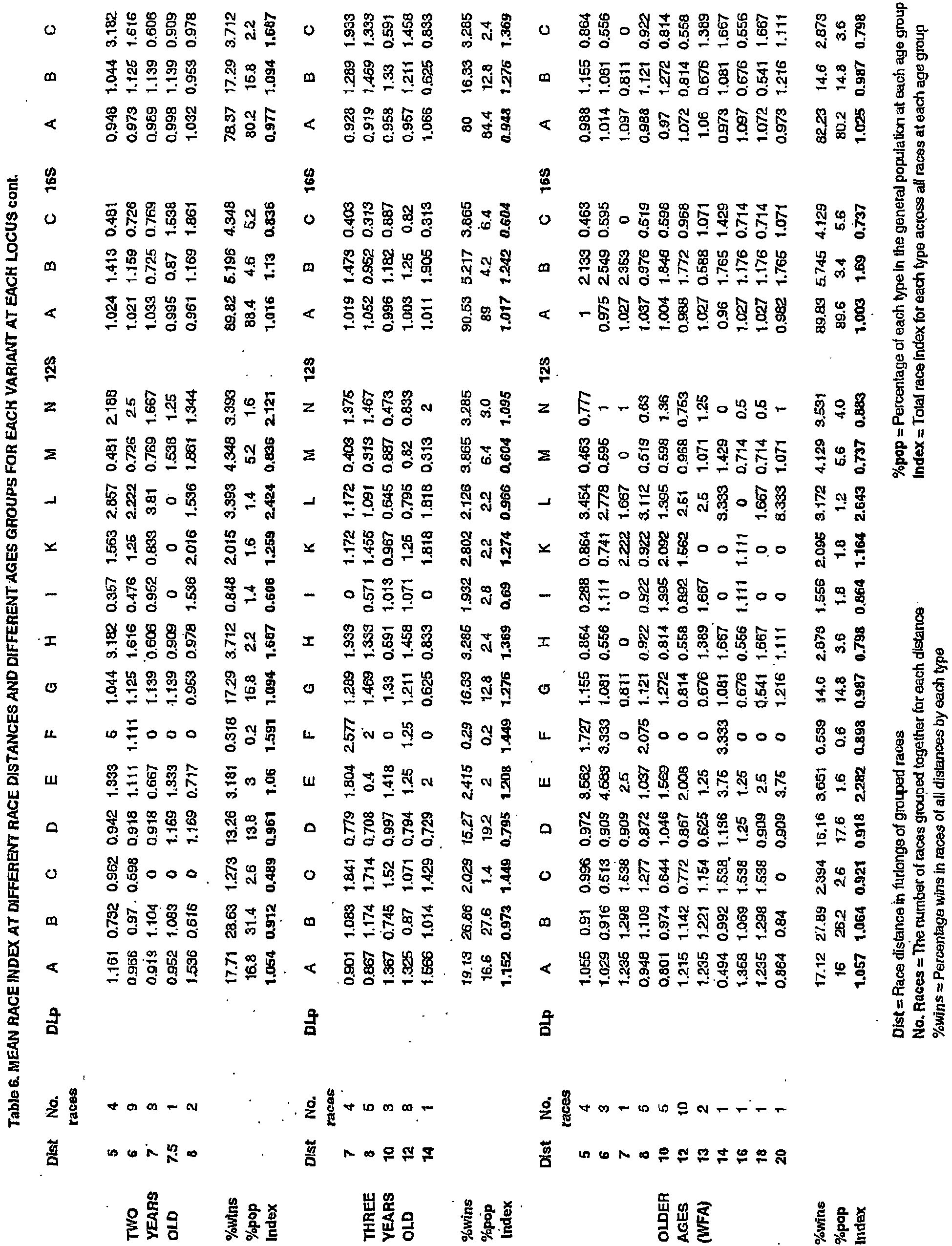

**Table 7. Correlations and Regressions of Race Index vs distance for different gene variants**

| **2YO** | | | | | **3YO** | | | | | **OLDER** | | | | |
|---|---|---|---|---|---|---|---|---|---|---|---|---|---|---|
| **LOCUS** | **VAR.** | **COR.** | **REG.** | **SIG.** | **LOCUS** | **VAR.** | **COR.** | **REG.** | **SIG.** | **LOCUS** | **VAR.** | **COR.** | **REG.** | **SIG.** |
| CCO1 | | | | | CCO1 | A | -0.4425 | 0.0446 | p<0.05 | CCO1 | | | | |
| | | | | | | D | 0.4904 | 0.0240 | p<0.05 | | | | | |
| ATP6 | D | -0.4961 | 0.0307 | p<0.05 | ATP6 | A | 0.5139 | 0.0172 | p<0.05 | ATP6 | E | 0.3637 | 0.0345 | p<0.05 |
| | E | 0.6014 | 0.0065 | p<0.01 | | | | | | | | | | |
| | | | | | | C | -0.6850 | 0.0006 | p<0.001 | | | | | |
| ND5 | C | -0.4363 | 0.0618 | p<0.1 | ND5 | D | -0.4467 | 0.0424 | p<0.05 | ND5 | | | | |
| | | | | | | E | 0.3724 | 0.0965 | p<0.10 | | | | | |
| | | | | | | F | 0.6247 | 0.0025 | p<0.01 | | | | | |
| CYT B | C | -0.4961 | 0.0307 | p<0.05 | CYT B | A | 0.5976 | 0.0042 | p<0.01 | CYT B | | | | |
| | | | | | | B | -0.5271 | 0.0141 | p<0.05 | | | | | |
| ND4 | | | | | ND4 | A | 0.6684 | 0.0009 | p<0.001 | ND4 | | | | |
| | | | | | | B | -0.5271 | 0.0141 | p<0.05 | | | | | |
| CCO2 | | | | | CCO2 | A | 0.6247 | 0.0025 | p<0.01 | CCO2 | | | | |
| | | | | | | B | -0.5271 | 0.0141 | p<0.05 | | | | | |
| ND4L | | | | | ND4L | | | | | ND4L | | | | |
| ND2 | B | -0.4961 | 0.03074 | p<0.05 | ND2 | A | -0.5187 | 0.01599 | p<0.05 | ND2 | | | | |
| | | | | | | C | 0.6684 | 0.00093 | p<0.001 | | | | | |
| 12S | C | 0.6014 | 0.00645 | p<0.01 | 12S | | | | | 12S | C | 0.3637 | 0.03446 | p<0.05 |
| 16S | C | -0.4961 | 0.03074 | p<0.05 | 16S | A | 0.4060 | 0.06779 | p<0.10 | 16S | | | | |
| | | | | | | B | -0.4339 | 0.04941 | p<0.05 | | | | | |

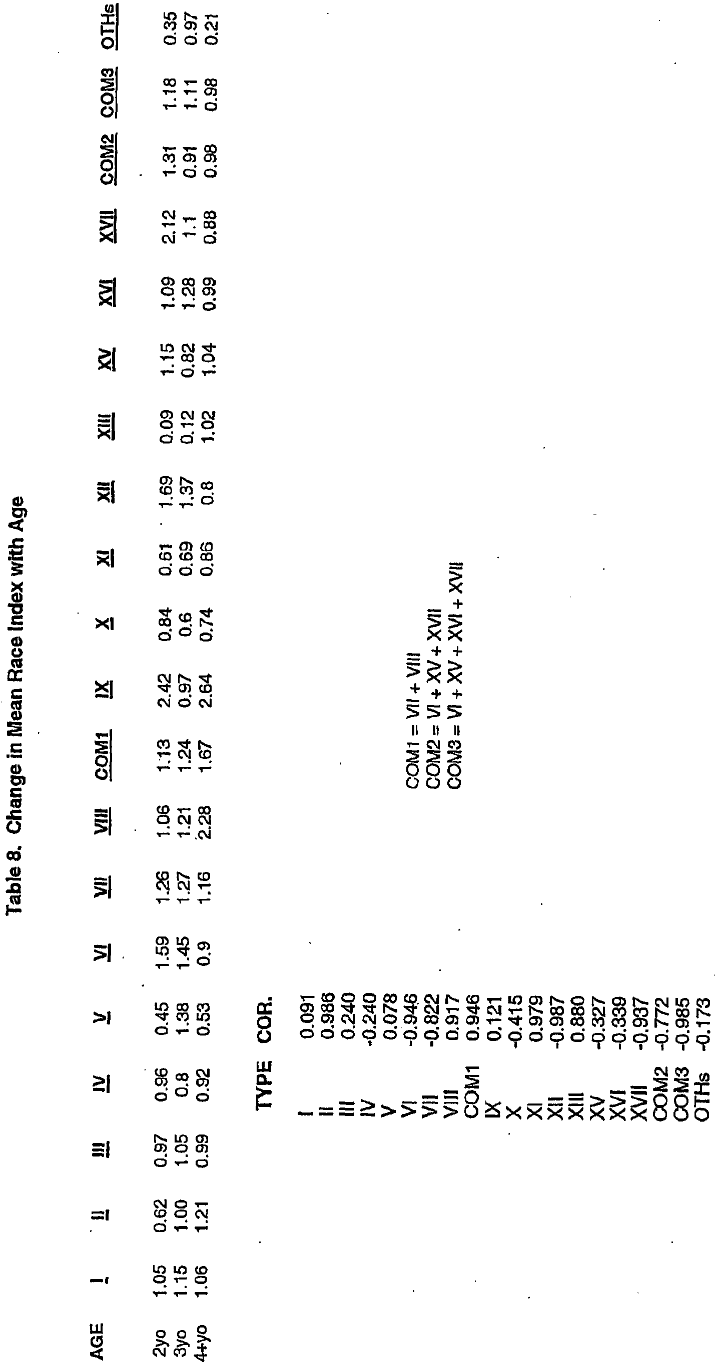

**Table 9. Change in stamina bias at different age groups.**

| | **2yo** | **3yo** | **4yo** | | **Correl.** |
|---|---|---|---|---|---|
| I | 0.1677 | 0.6684 | 0.0926 | I | -0.1200 |
| II | 0.2017 | 0.2891 | 0.0708 | II | -0.5957 |
| III | 0.0814 | -0.4467 | 0.2059 | III | 0.1796 |
| IV | 0.2653 | 0.0747 | -0.0132 | IV | -0.9781 |
| V | -0.3615 | -0.1329 | -0.0563 | V | 0.9611 |
| VI | -0.3167 | -0.1830 | -0.3823 | VI | -0.3229 |
| VII | -0.0562 | 0.0234 | -0.0983 | VII | -0.3403 |
| VIII | -0.2557 | 0.0870 | -0.1973 | VIII | 0.1594 |
| COM1 | -0.2558 | 0.0787 | -0.2415 | COM1 | 0.0377 |
| IX | -0.1812 | -0.0809 | 0.0432 | IX | 0.9981 |
| X | 0.6014 | 0.3278 | 0.3637 | X | -0.7994 |
| XI | 0.2667 | 0.3724 | -0.0487 | XI | -0.7199 |
| XII | -0.4961 | -0.1136 | 0.1876 | XII | 0.9977 |
| XIII | -0.3159 | -0.2943 | 0.0674 | XIII | 0.8900 |
| XV | -0.1464 | -0.4355 | -0.0254 | XV | 0.2872 |
| XVI | 0.0024 | -0.4339 | -0.2857 | XVI | -0.6493 |
| XVII | -0.1798 | -0.2113 | -0.0912 | XVII | 0.7112 |
| COM2 | -0.2759 | -0.5396 | -0.4073 | COM2 | -0.4985 |
| COM3 | -0.1994 | -0.6574 | -0.3241 | COM3 | -0.2632 |
| OTHERS | -0.1960 | -0.1647 | -0.3275 | OTHERS | -0.7611 |

| | | | | | |
|---|---|---|---|---|---|
| COM1 = VII + VIII COM2 = VI + XV + XVII COM3 = VI + XV + XVI + XVII | | | | | |

**Table 10. Example of stallion/mare mating scheme incorporating a score based on mtDNA haplotype commonality**

**Table 10**

| | **Mare** | **TYPE** | **DI at 8f** | **RI at 8f** | **RI correl. at 3yo** | |
|---|---|---|---|---|---|---|
| | Orby's Girl | VIII | 1.25 | 1.36 | | |
| **Pref. order** | **STALLION SELECTION** | **TYPE** | | | | **SCORE** |
| **1** | Cape Cross | VIII | 1.36 | 1.23 | -0.44 | 75% |
| **2** | Definite Article | VIII | 1.72 | 1.34 | -0.44 | 70% |
| **2** | Barathea | X | 1.23 | 1.34 | -0.23 | 70% |
| **2** | Noverre | VIII | 1.38 | 1.34 | -0.44 | 70% |
| **2** | Diktat | VIII | 1.23 | 1.76 | -0.44 | 70% |
| **6** | Danehill Dancer | V | 1.27 | 1.23 | -0.26 | 67% |
| **6** | Fasliyev | V | 1.54 | 1.23 | -0.26 | 67% |
| **6** | Namid | VI | 1.26 | 0.99 | -0.13 | 67% |
| **6** | Indian Ridge | XIII | 1.26 | 1.22 | -0.23 | 59% |
| **9** | Desert Style | X | 1.23 | 1.09 | -0.12 | 59% |
| **9** | Domedriver | XIII | 1.78 | 1.09 | -0.23 | 59% |

**Table 11. Trial betting results for Royal Ascot and The Epsom Derby 2004**

| **Event Name** | **Horse*** | **Open Date** | **P&L** |
|---|---|---|---|
| UK / R Ascot 19th Jun:TO BE PLACED | Cape Of Good Hope (1.11) | 2004-06-19 15.45 | 31.16 |
| UK / R Ascot 19th Jun:1m4f Grp2 | Doyen (1.25) | 2004-06-19 15.05 | 9.88 |
| UK / R Ascot 18th Jun:1m Grp 1 | Attraction (10.00) | 2004-06-18 15.45 | 13.01 |
| UK / R Ascot 17th Jun:TO BE PLACED | Papineau (2.3) | 2004-06-1715.45 | 11.17 |
| UK / R Ascot 17th Jun:TO BE PLACED | Sahool (1.25) | 2004-06-17 15.05 | 15.33 |
| UK / R Ascot 17th Jun:5f Grp3 | Blue Dakota (1.35) | 2004-06-17 14.30 | 9.21 |
| UK / R Ascot 16th Jun:TO BE PLACED | Soar (2.7) | 2004-06-1616.55 | 7.03 |
| UK / R Ascot 16th Jun:1m2f Grp1 | Rakti (2.4) | 2004-06-16 15.45 | 25.65 |
| UK / R Ascot 16th Jun:TO BE PLACED | Favourable Terms (3.1) | 2004-06-1615.05 | 15.01 |
| UK / R Ascot 16th Jun:TO BE PLACED | Fokine (4.3) | 2004-06-16 14.30 | 14.44 |
| UK / R Ascot 15th Jun:6f Grp2 | Iceman (1.25) | 2004-06-1514.30 | 22.8 |
| UK / Epsom 5th Jun:LAID NOT TO BE PLACED | Snow Ridge (0.00) | 2004-06-05 16.20 | 57.95 |
| | | | |
| | | TOTAL | **232.64** |

| | | | |
|---|---|---|---|
| * RI in brackets | | | |

**Table 12. Loci and primers.**

| **Gene** | **Locus** | **Forward Primer 5' to 3'** | **Reverse Primer 5' to 3'** | **mtDNA position (bp)** | **length (bp)** | **Annealment 30 secs** | **Extension 72°C** |
|---|---|---|---|---|---|---|---|
| NADH dehydrogenase 1 | *MTND1* | TGTTCATAATTAACGTCCTC | CTATGTTTGTGGTGGGATG | 2772 - 3727 | 955 | 47.5°C | 3 min |
| NADH dehydrogenase 2 | *MTND2* | CCCTTATCTTCACAACTATTC | GGGAGGATATAACAATTAACG | 3944 - 4936 | 992 | 48.5°C | 3 min |
| NADH dehydrogenase 3 | *MTND3* | ATAAACCTCATACTGACACTCC | TTTGGGTTCATTCGTAGG | 9498 - 9822 | 324 | 50.5°C | 1.5 min |
| NADH dehydrogenase 4 | *MTND4* | CAATAGCCTAAACTTCTCAC | GAATAGCTCTCCAATTAGG | 10345 - 11344 | 999 | 46.5°C | 3 min |
| NADH dehydrogenase 4L | *MTND4L* | ATATCTTCCTAGCATTCACAG | TAGCATTGGAGGAGGTTAAG | 9934 - 10210 | 276 | 49°C | 1.5 min |
| NADH dehydrogenase 5 | *MTND5* | TTTCCAACTGTTCATCGG | GTTGGAGATGAAGAATCCG | 12193 -13192 | 999 | 51°C | 3 min |
| NADH dehydrogenase 6 | *MTND6* | AAACCTTCACCTATTTATGG | TTAATCTCCACGAGTAACC | 13587 - 14048 | 461 | 47°C | 2 min |
| Cytochrome c oxidase 1 | *MTCO1* | ACATCGGCACTCTGTACC | AAGAAGATGAAGCCTAGAGC | 5402 - 6401 | 999 | 50°C | 3 min |
| Cytochrome c oxidase 2 | *MTCO2* | CCCTTCCAACTAGGATTC | ATTGATGCAGATCATTCTTC | 7057 - 7724 | 667 | 48.5°C | 2.5 min |
| Cytochrome c oxidase 3 | *MTCO3* | CACCAAACCCACGCTTAC | TCCTCATCAATAAATAGAGACG | 8651 - 9424 | 773 | 52°C | 2.5 min |
| ATP synthase 6 | *MTATP6* | AAATCTATTCGCCTCTTTC | AGGTGTTGTCGTGTAAGTAAAG | 7973 - 8643 | 671 | 49°C | 2.5 min |
| ATP synthase 8 | *MTATP8* | ATGCCACAGTTGGATACATC | GTAGCGAAAGAGGCGAATAG | 7804 - 7996 | 192 | 49°C | 1.5 min |
| Cytochrome b | *MTCYB* | CATCCGGAAATCTCACCC | TTCGATGGTGCTTGCGAG | 14196 -15306 | 1110 | 55°C | 3 min |
| 12S RNA | *MTRNR1* | AGAATTACACATGCAAGTATCC | CAAGTACACCTTCCGGTATAC | 108 - 1039 | 931 | 52°C | 3 min |
| 16S RNA | *MTRNR2* | CTAAAGCTAGCCCAAACAATAC | GTTTGTGTTTGCCGAGTTC | 1114 - 1939 | 825 | 50.5°C | 3 min |
| 16S RNA | *MTRNR2* | TGTTAACCCAACACAGGC | GGCGGTAGAAGTTATAAATTAG | 1868 - 2679 | 811 | 52°C | 3 min |
| D-loop | *DLOOP* | GCTCCACCATCAACACCCAAAG | TGAAGAAAGAACCAGATGCCAG | 15420 - 15860 | 440 | 52°C | 2 min |

**Table 13. Haplotypes and their constituent variants.**

| | Loci | | | | | | | | | | |
|---|---|---|---|---|---|---|---|---|---|---|---|
| | D loop | Cyto B | ATP 6 | ND2 | ND4 | ND4L | ND5 | CcO1 | CcO2 | 16S | 12S |
| Haplotype | | | | | | | | | | | |
| I | D | A | A | A | C | B | B | B | D | A | A |
| II | B | B | C | A | B | A | D | A | B | A | A |
| III | B | B | B | A | B | A | A | A | B | A | A |
| IV | A | A | A | C | A | A | F | D | A | A | A |
| V | C | A | A | A | C | B | C | C | D | A | A |
| VI | F | B | C | A | B | A | A | A | B | A | A |
| VII | K | A | A | A | C | B | C | C | C | A | B |
| VIII | E | A | A | A | G | B | C | C | C | A | B |
| IX | L | A | A | A | D | A | F | D | A | A | A |
| X | M | A | E | A | C | B | A | A | D | A | C |
| XI | I | A | A | A | C | B | E | C | D | A | A |
| XII | H | C | D | B | C | B | A | D | D | C | A |
| XIII | C | A | A | A | E | B | C | C | D | A | A |
| XIV | J | A | A | A | C | B | A | A | D | A | C |
| XV | B | B | C | A | B | A | A | A | B | A | A |
| XVI | G | B | C | A | B | A | A | A | B | B | A |
| XVII | N | B | C | A | B | A | A | A | B | A | A |
| Number of variants | 14 | 3 | 5 | 3 | 5 | 2 | 6 | 4 | 4 | 3 | 3 |

**Table 14. Average Race Index (RI) for each haplotype at different racing distance.**

| | | | Haplotype | | | | | | | | | | | | |
|---|---|---|---|---|---|---|---|---|---|---|---|---|---|---|---|
| Race Distance (m) | Races at each distance | Total races scored | I | II | III | IV | VII | VIII | IX | X | XI | XII | XVI | XVI | XVII |
| 1400-1600 | 9 | 444 | 0.74 | 1.28 | 0.93 | 0.84 | 1.33 | 1.08 | 1.15 | 0.36 | 0.21 | 1.65 | 1.12 | 1.42 | 1.42 |
| 2000 | 3 | 141 | 0.98 | 0.96 | 0.50 | 1.41 | 0.95 | 1.36 | 0.69 | 0.90 | 1.06 | 0.58 | 0.70 | 1.31 | 0.44 |
| 2400 | 8 | 400 | 0.81 | 0.85 | 1.16 | 1.34 | 1.25 | 1.25 | 0.80 | 0.82 | 1.25 | 1.46 | 0.54 | 1.15 | 0.83 |
| 2800 | 1 | 50 | 0.73 | 0.85 | 1.67 | 1.57 | 1.82 | 2.00 | 1.82 | 0.31 | 0.00 | 0.83 | 0.54 | 0.62 | 2.00 |
| Percentage in population | | | 19.20 | 11.80 | 8.40 | 16.60 | 2.20 | 2.00 | 2.20 | 6.40 | 2.80 | 2.40 | 7.40 | 13.00 | 3.00 |
| Correlation Coefficient (r) | | | 0.097 | -0.447 | 0.289 | 0.684 | 0.023 | 0.087 | -0.081 | 0.328 | 0.478 | -0.114 | -0.469 | -0.519 | -0.211 |
| Significance (P) | | | | <0.05 | | <0.001 | | | | | <0.05 | | <0.05 | <0.05 | |

**Table 15. Correlations and Regressions (RI vs distance for gene variants)**

| **LOCUS** | **VAR.** | **COR.** | **REG.** | **SIG.** |
|---|---|---|---|---|
| CCO1 | A | -0.4948 | 0.0226 | p<0.05 |
| | D | 0.4999 | 0.0210 | p<0.05 |
| | | | | |
| ATP6 | A | 0.5658 | 0.0075 | p<0.01 |
| | C | -0.7070 | 0.0003 | p<0.001 |
| | | | | |
| ND5 | D | -0.4467 | 0.0424 | p<0.05 |
| | E | 0.4778 | 0.0285 | p<0.05 |
| | F | 0.6356 | 0.0020 | p<0.01 |
| | | | | |
| CYTB | A | 0.6284 | 0.0023 | p<0.01 |
| | B | -0.5670 | 0.0074 | p<0.01 |
| | | | | |
| ND4 | A | 0.6839 | 0.0006 | p<0.001 |
| | B | -0.5670 | 0.0074 | p<0.01 |
| | | | | |
| CCO2 | A | 0.6356 | 0.0020 | p<0.01 |
| | B | -0.5670 | 0.0074 | p<0.01 |
| | | | | |
| ND4L | | NONE | | |
| | | | | |
| ND2 | A | -0.5349 | 0.0125 | p<0.05 |
| | C | 0.6839 | 0.0006 | p<0.001 |
| | | | | |
| 12S | | NONE | | |
| | | | | |
| 16S | A | 0.4823 | 0.0268 | p<0.10 |
| | B | -0.5186 | 0.0160 | p<0.05 |

## Claims

1. A process for determining or predicting sporting performance aptitude of horses,
comprising analysis of the presence, distribution or expression of one or more nucleotide sequence variants, or a set of nucleotide sequence variants which represent a haplotype,
having an association with sporting performance aptitude, said variants comprising mitochondrial nucleotide sequence variants and/or chromosomal nucleotide sequence variants which are able to complement said mitochondrial nucleotide sequence variants, said variants being selected from the group consisting of NADH dehydrogenase 2, NADH dehydrogenase 4, NADH dehydrogenase 4L, NADH dehydrogenase 5, Cytochrome oxidase I, Cytochrome oxidase II, ATPase 6, Cytochrome B, rRNA 16S-1, rRNA 16S-2, rRNA 12S and dloop variants,
said variants contributing to or representing haplotypes selected from the group consisting of types I - XVII listed in Table 1.

2. A process according to claim 1, wherein the one or more nucleotide sequence variants includes sequences located between nucleic acid primer pairs, said nucleic acid primers being selected from the group consisting of SEQ ID NOs: 1-36.

3. A process according to claim 1, wherein the one or more nucleotide sequence variants are selected from one or more of the group consisting of SEQ ID NOs: 37-75.

4. A process according to any preceding claim, wherein the sport is racing.

5. A process according to any preceding claim, wherein the performance aptitude is for speed, stamina, optimum racing distance or precocity of age for racing.

6. A process according to any preceding claim, wherein the horses are selected from thoroughbred, standardbred and trotting horses.

7. A process according to any preceding claim, wherein the nucleotide sequence variants are determined by an analysis step comprising amplification of the nucleotide sequence variants by PCR using one or more of the nucleic acid primers selected from the group consisting of SEQ ID NOs: 1-36.

8. Use of a haplotype selected from any of types I - XVII listed in Table 1 for the determination of sporting aptitude of horses.

9. Use of a haplotype selected from any of types I - XVII listed in Table 1 for training.

10. A kit for use in the process of any claims 1 to 7 comprising one or more nucleic acid primers selected from the group consisting of SEQ ID NOs: 1-36, for the determination of the haplotype of horses, wherein the haplotype is selected from the group consisting of types I - XVII listed in Table 1.

11. A kit according to Claim 10, wherein the one or more nucleic acid primers are capable of amplifying one or more nucleotide sequence variants selected from the group consisting of SEQ ID NOs: 37-75.

12. A kit according to claims 10 or 11, which comprises a microarray.

13. Use of a process according to any of claims 1 to 7 in the organisation or reorganisation of stud books or pedigree databases or in the verification of pedigree.

14. Use of a process according to any of claims 1 to 7 in a typing system for equine maternally-inherited extra-chromosomal DNA.

15. Use of a process according to any of claims 1 to 7 in the study of horse physiology.

16. Use of a process according to any of claims 1 to 7 in the study of complementation between respiratory complex genes carried on the chromosomes and those carried on mtDNA and other genetic interactions so as to identify positive outcomes resulting in or contributing to, superior energy release or other characteristics which affect sporting performance aptitude.

17. Use of a process according to any of claims 1 to 7 for ranking the genetic profile of a horse, a group of horses, or predicting the relative outcome of racing involving several horses, comprising
measuring or estimating the haplotype and optionally measuring or estimating the presence, distribution or differential expression of one or more nucleotide sequence variants, and applying the resulting analysis so as to rank the subjects according to preferred racing category or distance.

18. Use according to claim 17, wherein an index is calculated using the percentage of the relevant type related to the frequency of that type in the relevant population.

## Patentansprüche

1. Prozess zum Ermitteln oder Voraussagen einer sportlichen Leistungsfähigkeit von Pferden, umfassend die Analyse des Vorliegens, der Verteilung oder der Expression von einer oder mehreren Nukleotidsequenzvarianten oder einem Satz von Nukleotidsequenzvarianten, die einen Haplotyp repräsentieren, der mit sportlicher Leistungsfähigkeit assoziiert ist, wobei die Varianten mitochondriale Nukleotidsequenzvarianten und/oder chromosomale Nukleotidsequenzvarianten umfassen, die in der Lage sind, die mitochondrialen Nukleotidsequenzvarianten zu ergänzen, wobei die Varianten aus der Gruppe ausgewählt sind, die aus NADH-Dehydrogenase 2, NADH-Dehydrogenase 4, NADH-Dehydrogenase 4L, NADH-Dehydrogenase 5, Zytochromoxidase I, Zytochromoxidase II, ATPase 6, Zytochrom B, rRNA 16S-1, rRNA 16S-2, rRNA 12S und D-Loop-Varianten besteht, wobei die Varianten zu Haplotypen beitragen oder Haplotypen repräsentieren, die aus der Gruppe ausgewählt sind, die aus den Typen I - XVII besteht, die in Tabelle 1 aufgeführt sind.

2. Prozess nach Anspruch 1, wobei die eine oder mehreren Nukleotidsequenzvarianten Sequenzen umfassen, die zwischen Nukleinsäureprimerpaaren liegen, wobei die Nukleinsäureprimer aus der Gruppe ausgewählt sind, die aus SEQ-ID-NUMMERN 1-36 besteht.

3. Prozess nach Anspruch 1, wobei die eine oder mehreren Nukleotidsequenzvarianten aus einer oder mehreren der Gruppe ausgewählt sind, die aus SEQ-ID-NUMMERN 37-75 besteht.

4. Prozess nach einem der vorhergehenden Ansprüche, wobei der Sport Rennsport ist.

5. Prozess nach einem der vorhergehenden Ansprüche, wobei die Leistungsfähigkeit sich auf Geschwindigkeit, Ausdauer, optimale Rennstrecke oder Frühreife hinsichtlich des Rennalters bezieht.

6. Prozess nach einem der vorhergehenden Ansprüche, wobei die Pferde aus Vollblütern, Standardbred und Trabern ausgewählt sind.

7. Prozess nach einem der vorhergehenden Ansprüche, wobei die Nukleotidsequenzvarianten durch einen Analyseschritt ermittelt werden, umfassend die Amplifikation der Nukleotidsequenzvarianten mittels PCR unter Verwendung von einem oder mehreren der Nukleinsäureprimer, die aus der Gruppe ausgewählt sind, die aus SEQ-ID-NUMMERN 1-36 besteht.

8. Verwendung eines Haplotyps, der aus den Typen I - XVII ausgewählt ist, die in Tabelle 1 aufgeführt sind, zur Ermittlung der sportlichen Fähigkeit von Pferden.

9. Verwendung eines Haplotyps, der aus einem der Typen I - XVII ausgewählt ist, die in Tabelle 1 aufgeführt sind, zum Training.

10. Kit zur Verwendung In dem Prozess nach einem der Ansprüche 1 bis 7, umfassend einen oder mehrere Nukleinsäureprimer, die aus der Gruppe ausgewählt sind, die aus SEQ-ID-NUMMERN 1-36 besteht, zur Ermittlung des Haplotyps von Pferden, wobei der Haplotyp aus der Gruppe ausgewählt ist, die aus den Typen I - XVII besteht, die in Tabelle 1 aufgeführt sind.

11. Kit nach Anspruch 10, wobei der eine oder die mehreren Nukleinsäureprimer in der Lage sind, eine oder mehrere Nukleotidsequenzvarianten zu amplifizieren, die aus der Gruppe ausgewählt sind, die aus SEQ-ID-NUMMERN 37-75 besteht.

12. Kit nach Ansprüchen 10 oder 11, das ein Microarray umfasst.

13. Verwendung eines Prozesses nach einem der Ansprüche 1 bis 7 bei der Organisation oder Reorganisation von Zuchtbüchern oder Abstammungsdatenbanken oder bei der Verifizierung der Abstammung.

14. Verwendung eines Prozesses nach einem der Ansprüche 1 bis 7 bei einem Typisierungssystem für von der Mutter vererbte, extrachromosomale DNA beim Pferd.

15. Verwendung eines Prozesses nach einem der Ansprüche 1 bis 7 bei der Untersuchung der Pferdephysiologie.

16. Verwendung eines Prozesses nach einem der Ansprüche 1 bis 7 bei der Untersuchung der Komplementierung zwischen Atmungskomplexgenen, die auf den Chromosomen getragen werden, und denen, die auf mtDNA getragen werden, und anderen genetischen Wechselwirkungen, um positive Ergebnisse zu erkennen, die zu überlegener Energiefreisetzung oder anderen Merkmalen führen oder beitragen, die sich auf sportliche Leistungsfähigkeit auswirken.

17. Verwendung eines Prozesses nach einem der Ansprüche 1 bis 7 zum Rangordnen des genetischen Profils eines Pferd, einer Gruppe von Pferden, oder zum Voraussagen des relativen Ergebnisses von Rennen unter Beteiligung mehrerer Pferden, umfassend das Messen oder Schätzen des Haplotyps und optional das Messen oder Schätzen des Vorliegens, der Verteilung oder differenziellen Expression von einer oder mehreren Nukleotidsequenzvarianten, und das Anwenden der sich ergebenden Analyse zum Rangordnen der Versuchstiere entsprechend der bevorzugten Rennklasse oder Rennstrecke.

18. Verwendung nach Anspruch 17, wobei ein Index anhand des Prozentsatzes des relevanten Typs berechnet wird, der in Verbindung mit der Häufigkeit dieses Typs in der relevanten Population steht.

## Revendications

1. Procédé permettant de déterminer ou de prédire l'aptitude aux performances sportives de chevaux, comprenant l'analyse de la présence, la distribution ou l'expression d'une ou de plusieurs variantes de séquences nucléotidiques ou d'une série de variantes de séquences nucléotidiques qui représentent un haplotype ayant une association avec l'aptitude aux performances sportives, lesdites variantes comprenant des variantes de séquences nucléotidiques mitochondriales et/ou des variantes de séquences nucléotidiques chromosomiques capables de s'apparier aux dites variantes de séquences nucléotidiques mitochondriales et étant sélectionnées dans le groupe consistant en des variantes de la NADH-déshydrogénase 2, de la NADH-déshydrogénase 4, de la NADH-déshydrogénase 4L, de la NADH-déshydrogénase 5, de la cytochrome-oxydase I, de la cytochrome-oxydase II, de l'ATPase 6, du cytochrome B, de l'ARNr 16S-1, de l'ARNr 16S-2, de l'ARNr 12S et de structures en épingle à cheveux, lesdites variantes contribuant ou correspondant à des haplotypes sélectionnés dans le groupe consistant en les types I - XVII répertoriés au Tableau 1.

2. Procédé selon la revendication 1, dans laquelle la ou les variantes de séquences nucléotidiques inclu(en)t des séquences situées entre des paires d'amorces d'acide nucléique, lesdites amorces d'acide nucléique étant sélectionnées dans le groupe consistant en les SÉQ ID N° : 1-36.

3. Procédé selon la revendication 1, dans laquelle la ou les variantes de séquences nucléotidiques est(sont) sélectionnée(s) dans le groupe consistant en les SÉQ ID N° : 37-75.

4. Procédé selon l'une quelconque des revendications précédentes, dans lesquelles le sport concerné est la course hippique.

5. Procédé selon l'une quelconque des revendications précédentes, dans lesquelles l'aptitude aux performances concerne la vitesse, l'endurance, la distance optimale de course et la précocité de l'âge auquel un cheval peut courir.

6. Procédé selon l'une quelconque des revendications précédentes, dans lesquelles les chevaux sont sélectionnés parmi des pur-sang, des trotteurs américains et des chevaux de trot.

7. Procédé selon l'une quelconque des revendications précédentes, dans lesquelles les variantes de séquences nucléotidiques sont déterminées par une étape d'analyse comprenant l'amplification par PCR des variantes de séquences nucléotidiques en utilisant une ou plusieurs des amorces d'acide nucléique sélectionnées dans le groupe consistant en les SÉQ ID N° : 1-36.

8. Utilisation d'un haplotype sélectionné parmi l'un quelconque des types I - XVII répertoriés au Tableau 1 pour la détermination de l'aptitude sportive de chevaux.

9. Utilisation d'un haplotype sélectionné parmi l'un quelconque des types I - XVII répertoriés au Tableau 1 pour l'entraînement.

10. Kit à utiliser dans le procédé de l'une quelconque des revendications 1 à 7, comprenant une ou plusieurs amorces d'acide nucléique sélectionnées dans le groupe consistant en les SÉQ ID N° : 1-36 pour la détermination de l'haplotype de chevaux, l'haplotype étant sélectionné parmi l'un quelconque des types I - XVII répertoriés au Tableau 1.

11. Kit selon la revendication 10, dans laquelle l'amorce ou les amorces d'acide nucléique est(sont) capable(s) d'amplifier une ou plusieurs variantes de séquences nucléotidiques sélectionnées dans le groupe consistant en les SÉQ ID N° : 37-75.

12. Kit selon les revendications 10 ou 11, qui comprend un microréseau d'ADN.

13. Utilisation d'un procédé selon l'une quelconque des revendications 1 à 7 pour l'organisation ou la réorganisation des livres ou des banques de données généalogiques ou pour la vérification du pedigree.

14. Utilisation d'un procédé selon l'une quelconque des revendications 1 à 7 dans un système de typage de l'ADN extra-chromosomique d'origine maternelle chez le cheval.

15. Utilisation d'un procédé selon l'une quelconque des revendications 1 à 7 dans l'étude de la physiologie équine.

16. Utilisation d'un procédé selon l'une quelconque des revendications 1 à 7 dans l'étude de l'appariement entre des gènes du complexe respiratoire portés sur les chromosomes et ceux portés sur l'ADN mitochondrial et d'autres interactions génétiques dans le but d'identifier des impacts positifs conduisant ou contribuant à une libération accrue d'énergie ou à d'autres caractéristiques qui affectent l'aptitude aux performances sportives.

17. Utilisation d'un procédé selon l'une quelconque des revendications 1 à 7 pour le classement du profil génétique d'un cheval ou groupe des chevaux ou pour la prédiction du résultat relatif d'une course à laquelle participent plusieurs chevaux, comprenant une mesure ou estimation de l'haplotype et, en option, une mesure ou estimation de la présence, la distribution ou l'expression différentielle d'une ou de plusieurs variantes de séquences nucléotidiques et l'exploitation de l'analyse résultante pour le classement des sujets en fonction de leur catégorie ou distance de course préférée.

18. Utilisation selon la revendication 17, dans laquelle un indice est calculé en employant le pourcentage du type pertinent par rapport à la fréquence de ce type dans la population pertinente.
